# EUROPEAN PATENT SPECIFICATION

(11) **EP 1 321 477 B1**
(45) Date of publication and mention of the grant of the patent: **13.10.2004**
(21) Application number: 01130805.3
(22) Date of filing: 22.12.2001
(51) Int. Cl.: C07K 16/00, C12N 5/20, C12N 5/10, C12N 5/06, A61K 39/00

(54) **Method for the generation of genetically modified vertebrate precursor lymphocytes and use thereof for the production of heterologous binding proteins**
Verfahren zur Herstellung von genetisch veränderten Lymphozyten Vorläuferzellen von Wirbeltieren und deren Gebrauch zur Produktion von heterologen Bindeproteinen
Procédure pour la production de cellules précurseur lymphocytaires, génétiquement modifiées et leur utilisation pour la production de protéines de liaison hétérologues

(43) Date of publication of application: 25.06.2003
(73) Proprietor: 4-Antibody AG, 4056 Basel (CH)
(72) Inventor: Grawunder, Ulf, 4052 Basel (CH); Melchers, Georg Friedrich, 79639 Grenzach-Wyhlen (DE)
(74) Representative: Stürken, Joachim

(56) References cited:
- WO-A-01/87058
- WO-A-92/03918
- AN DONG SUNG ET AL: "Lentivirus vector-mediated hematopoietic stem cell gene transfer of common gamma-chain cytokine receptor in rhesus macaques." JOURNAL OF VIROLOGY, vol. 75, no. 8, April 2001 (2001-04), pages 3547-3555, XP002201133 ISSN: 0022-538X
- FLUCKIGER A-C ET AL: "IN VITRO RECONSTITUTION OF HUMAN B-CELL ONTOGENY: FROM CD34+ MULTIPOTENT PROGENITORS TO IG-SECRETING CELLS" BLOOD, W.B. SAUNDERS, PHILADELPHIA, VA, US, vol. 92, no. 12, 15 December 1998 (1998-12-15), pages 4509-4520, XP002944960 ISSN: 0006-4971
- GHIA PAOLO ET AL: "Ordering of human bone marrow B lymphocyte precursors by single-cell polymerase chain reaction analyses of the rearrangement status of the immunoglobulin H and L chain gene loci." JOURNAL OF EXPERIMENTAL MEDICINE, vol. 184, no. 6, 1996, pages 2217-2229, XP002201134 ISSN: 0022-1007
- DUENAS M ET AL: "In vitro immunization of naive human B cells yields high affinity immunoglobulin G antibodies as illustrated by phage display" IMMUNOLOGY, BLACKWELL SCIENTIFIC PUBLICATIONS, GB, vol. 89, 1 September 1996 (1996-09-01), pages 1-7, XP002093905 ISSN: 0019-2805

## Description

### Field of the invention

The present invention generally relates to the fields of genetic engineering and antibody production. In particular, it relates to the generation of genetically modified vertebrate precursor lymphocytes that have the potential to differentiate into more mature lymphoid lineage cells, and to the use thereof for the production of any heterologous antibody or binding protein.

### Background of the invention

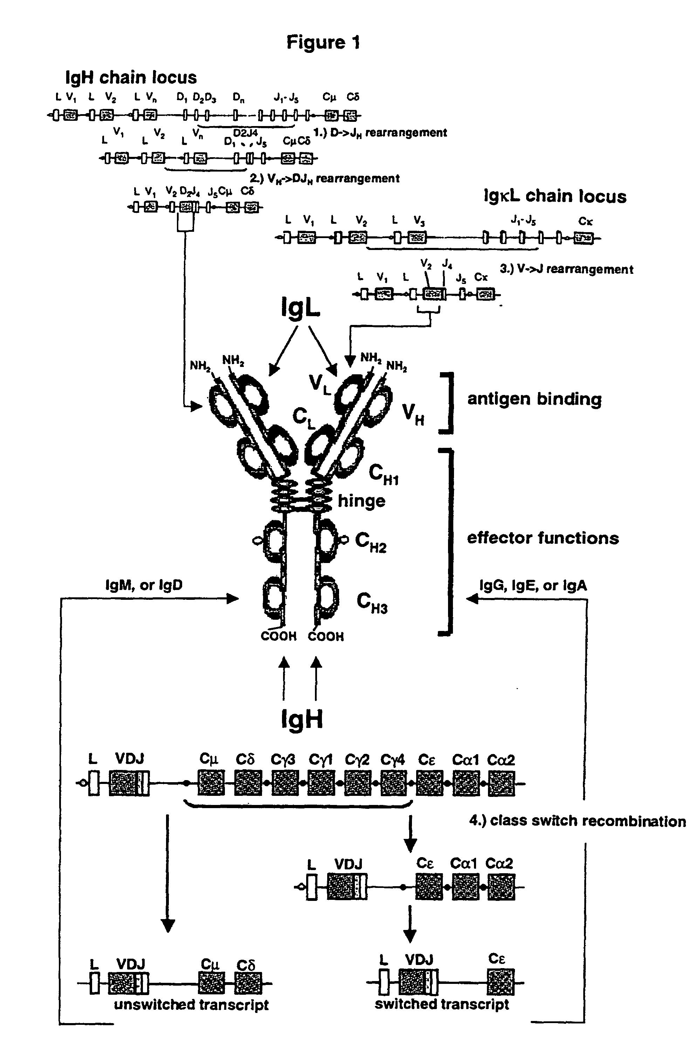

Monoclonal antibodies have been proven to be effective reagents for both diagnosis, prevention and therapy of disease (Glennie and Johnson, *Immunol. Today*, 21, p. 403-410, 2000). This is due to their unique capacity to bind very specifically to particular epitopes on target molecules (antigens) via their variable domains and, at the same time, to mediate effector functions via their constant region domains (Frazer and Capra, *Fundamental Immunology,* W.E. Paul (Ed.), Fourth Edition, p. 37-74, 1999) (Fig. 1). This enables monoclonal antibodies to specifically identify unique antigens in complex mixtures of macromolecules and to direct effector functions to these targets.

Antibodies (or immunoglobulins) consist of two identical heavy (H) chain and light (L) chain glycoproteins that are linked via disulphide bonds (Fig. 1). Each H and L chain comprises an N-terminal variable domain that varies between different antibodies and a C-terminal constant region, that is identical in different antibodies belonging to the same immunoglobulin isotype (Fig. 1). The combination of H and L chain variable domains generates the antigen binding pocket of the antibody and determines its specificity (or idiotype), whereas the constant regions determine its isotype (Frazer and Capra, s.a.). The variability of immunoglobulins results from the fact that V_{H} and V_{L} domains are encoded by a multitude of gene segments, that are designated V (variable), D (diversity; only present in the H chain locus), and J (joining) gene segments (Tonegawa, *Nature,* 302, p. 575-581, 1983) (Fig. 1). During the differentiation of B lymphocytes one V, D and J gene segment is randomly selected in each cell for the site-specific recombination process of V(D)J recombination, that assembles the gene segments, such that new coding regions for V_{H} or V_{L} domains are generated (Grawunder et al., *Curr*. *Opin. Immunol*., 10, p. 172-180, 1998). Due to the multitude of V, D, and J gene segments, and imprecision in gene segment joining, an enormous repertoire of different V region specificities can be generated by the millions of B lymphocytes produced by the immune system every day (Melchers et al., *Curr. Opin*. *Immunol*., 7, p. 214-227, 1995).

During evolution, immunoglobulin genes have slightly diverged between different species, such that the constant regions of antibodies differ between species. As a consequence, immunoglobulins from one species are most often immunogenic, if introduced into the vascular system of another species.

The immunogenicity of xenogeneic monoclonal antibodies therefore limits their use in the therapy of human disease, because exposure of patients to xenogeneic antibodies may result in adverse effects, even acute toxicity, or might simply lead to the neutralization and clearance of the applied antibody, thereby reducing its pharmacological efficacy (Clark, *Immunol Today,* 21, p. 397-402, 2000) In contrast, administration of fully human antibodies to patients usually does not lead to any of the aforementioned complications.

Human antisera or human polyclonal antibodies have occasionally been isolated from the blood of individual patients for the treatment or prevention of rare and usually highly lethal diseases, like Ebola virus infections, or e.g. for the treatment of individuals after exposure to snake venoms. However, this approach, for many reasons, is impractical for the treatment of diseases affecting larger populations. Furthermore, for ethical reasons, it is impossible to immunize humans with a given antigen for the purpose of monoclonal antibody production, because the B-lineage cells in humans producing the desired antibodies develop and reside in secondary lymphoid organs and cannot easily be obtained. Furthermore, many potential target antigens for therapeutic antibodies, in particular for cancer therapy, are human proteins (Glennie and Johnson, s.a.). Under normal circumstances, humans will not develop antibodies against these targets. Therefore, substantial efforts have been made in the recent past to develop procedures for the development of therapeutic human or humanized antibodies without the need of the human immune system.

The simplest approach uses standard genetic engineering techniques for cloning of cDNAs encoding the variable domains of H and L chains (V_{H} and V_{L} domains) from a hybridoma secreting a xenogeneic antibody of desired specificity. The cloned variable region cDNAs are then cloned into appropriate *E.coli*, yeast, insect or mammalian cell expression vectors containing human constant region genes. This will allow the production of monoclonal antibodies with the xenogeneic V_{H} and V_{L} domains fused to the human C_{H} and C_{L} constant region domains, thereby resulting in a humanized antibody. However, this approach has the disadvantage that the fusion of xenogeneic V_{H} and V_{L} domains to human C_{H} and C_{L} constant regions may result in either decreased affinity or altered specificity. In addition, the heterologous V_{H} and V_{L} of the humanized antibody are still immunogenic in humans, because framework regions of V domains vary between different species. This problem can in some cases be circumvented by grafting individual complementarity determining regions (CDRs), which mediate the direct contact to antigens, onto human framework regions of H and L chain variable domains (Fiorentini et al., *Immunotechnology*, 3, p. 45-59, 1997). However, for unknown reasons, CDR grafting still often results in the generation of immunogenetic antibodies and/or the decrease/loss of affinity and specificity.

Therefore, two different and more effective approaches have been developed in recent years for the generation of completely human immunoglobulins.

The first method is based on the expression (display) of single chain variable regions (scFv) consisting of one V_{H} and V_{L} domain on the surface of filamentous bacteriophages of *E.coli* (Hoogenboom and Chames, *Immunol*. *Today,* 21, p. 371-3818, 2000) (cf. also EP 0 585 287 B1, EP 0 605 522 B1). Phage display libraries containing a diverse repertoire of V_{H} and V_{L} chain can be constructed, and individual scFv specificities can be isolated from such libraries by binding of recombinant phages to immobilized antigen (McCafferty et al., *Nature,* 348, p. 552-554, 1990).

Phage display libraries for scFv binding proteins derived from a natural human repertoire have the drawback of being restricted to specificities contained in that repertoire, and specificities for many human antigens are therefore most often not represented in these libraries. Although combinatorial or synthetic phage display libraries may be used to circumvent this problem, a general drawback of this technology is that high affinity binding proteins for a given antigen can often not be isolated. Therefore, substantial efforts have been invested in constructing very large primary libraries by either brute force cloning or site specific recombination procedures.

The best combinatorial or synthetic libraries are estimated to contain 10⁹-10¹¹ potentially different binding sites and can yield binding specificities in the 1-200 nM range (Hoogenboom and Chames, s.a.). However, higher affinities in the picomolar range (10⁻¹² M), that can be reached during affinity maturation of immunoglobulins in germinal center B cells *in vivo,* can only be generated employing additional tedious genetic engineering procedures, including V gene shuffling, error-prone PCR, the use of *E.coli* mutator strains etc.

Whatever the outcome of a phage display library selection process is, eventually the genes encoding the scFv binding site need to be recloned into suitable expression vectors for human immunoglobulins, and these need to be stably transferred into a cellular expression system allowing the large-scale production of human antibodies, which is another time-consuming and expensive procedure.

The second approach for the production of fully human monoclonal antibodies is based on the use of transgenic mice harbouring constructs for the human immunoglobulin H and L chain gene loci (Jakobovits, *Curr*. *Opin. Biotechnol*., 6, p. 561-566, 1995). The human immunoglobulin transgenes are eventually bred onto a genetic background that does no longer allow assembly of the endogenous murine antigen receptor genes. In these mice, development of B-lineage cells depends on the expression of immunoglobulin H and L chains from the human transgenic constructs, and B lineage cells of these mice are only capable of producing human antibodies. There are three variations of this technique. In one, human immunoglobulin miniloci are used as transgenes, from which only a limited repertoire of human antibodies can be generated (Fishwild et al., *Nat. Biotechnol.,* 14, p. 845-851, 1996) (also cf. to, EP 0 546 073 B1, US 5,874,299, WO 92/03918). In a second variation, large regions of the human IgH and IgκL chain gene loci encompassing the majority of the variable region gene segments cloned into yeast artificial chromosomes are used. In a third variation, pieces of human chromosomes containing the entire human immunoglobulin H and L chain gene loci are integrated into the germline of mice generating so-called trans-chromosomic animals. Mice with such complex transgenes develop a practically normal human immunoglobulin repertoire.

Immunization of these transgenic or trans-chromosomic mice results in a humoral immune response resulting in the generation of fully human antibodies. This approach of generating human antibodies has one important advantage over the phage display library technology: High affinity antibodies for a given antigen can be generated, because the human immunoglobulins can undergo affinity maturation in germinal center B cells, which is a normal process in the course of an immune reaction.

However, there is one important drawback of this technology: First, the generation of transgenic or trans-chromosomic mice is very tedious, difficult, time-consuming, and therefore relatively expensive. For example, WO 92/03918 discloses B cells of a transgenic mouse expressing human monoclonal antibodies. In order to achieve this transgenic mouse, fertilized eggs are transfected with exogenous elements by introducing the heterologous IgH and IgL chain transgenes by pronuclear injection. The entire procedure requires the generation of at least four different mouse strains: two strains with targeted disruptions within the endogenous IgH and IgL chain gene loci, as well as two strains carrying transgenes or trans-chromosomes for part or all of the human IgH and IgL chain gene loci. In addition, all these four mouse strains have to be bred together, resulting in mice with a genotype carrying homozygous disruptions of both the endogenous IgH and IgL chain gene loci and at the same time carrying the human transgenes encoding both the heterologous IgH and IgL chains. Generation of the different knock-out and transgenic mouse strains, their screening and eventual crossbreeding is a lengthy procedure that will require at least two years, even if each step is completely optimized. The extensive time-frames required for the development of a xenomouse strain therefore leaves little flexibility for designing different mouse strains containing modified transgenic constructs. Therefore, this technology is not suitable for the modification and improvement of existing antibodies (e.g. of their affinity), because this would require the lengthy procedure of generating a novel transgenic mouse strain for this one antibody. This technology is therefore basically restricted to the *de novo* generation of human antibodies.

Based on the aforementioned facts, there is clearly the need for a technology allowing the production of fully human antibodies that would combine the advantages of both the phage display system (i.e. speed and flexibility in generating human antibodies, and the ability to modify and improve the properties of existing antibodies), and of the human immunoglobulin transgenic mouse technology (i.e. the ability to obtain high affinity antibodies due to affinity maturation occurring in the immune system, and production of antibodies with physiologic and natural structural features).

### Summary of the invention

The present invention provides means and methods to generate vertebrate precursor lymphocytes that can be used for the production of any binding protein or functional fragment(s) thereof with the ability to selectively bind to an antigen or ligand, including any heterologous antibody, antigen receptor composed of variable domains and constant regions comprising T cell receptors and membrane bound immunoglobulins, any artificial binding protein displaying either wild-type immune effector functions or modified or artificial effector functions not derivable from germline encoded heterologous immunoglobulins or antigen receptors, and any functional fragment(s) thereof. In particular, the invention provides means and methods to genetically modify vertebrate precursor lymphocytes and to effect their differentiation into more mature lymphoid lineage cells either *in vitro* or *in vivo,* thereby generating lymphocytes capable of producing said binding protein or functional fragment(s) thereof, as well as a method for the production of said binding protein or functional fragment(s) thereof. Furthermore, the present invention provides genetically modified vertebrate precursor lymphocytes and mature lymphoid lineage cells as well as immortalized cells derived therefrom suitable for carrying out the methods according to the invention.

Thus, the methods of the present invention allow a great deal of flexibility for the generation of lymphoid lineage cells with the potential to express simply any type of antibody or binding protein, within short periods of time. At the same time, the possibility to transplant these cells into compatible vertebrate hosts, where the cells participate in specific immune functions such as affinity maturation, allows the exploitation of the selectivity of the immune system for generating antibodies and immunoglobulin-like or even artificial binding proteins of high binding affinities for any given antigenic compound or composition.

### Description of the drawings

Figure 1 is a schematic representation of immunoglobulin structure and genetics. Antibodies or immunoglobulins (Ig) are glycoproteins that in their monomeric form are composed of two identical heavy chains (IgH) and two identical light chains (IgL) that are covalently linked by disulfide bonds. Each IgH chain comprises one N-terminal variable domain (V_{H}) and 3-4 constant region domains (C_{H}1-3, or 4), depending on the antibody isotype. In addition, antibodies contain a hinge region between the first and the second C_{H} domain, lending flexibility to the two antigen binding arms of the heterotetrameric glycoprotein. Light chains consist of only one N-terminal variable (V_{L}) and one C-terminal constant domain (C_{L}). The arrangement of the two IgH and IgL chains results in a Y shaped monomeric antibody with two highly variable binding sites for antigen, which are formed by the association of the IgH and IgL chain variable domains. Variable domains differ to a great extent in so-called hypervariable regions, if V-regions are compared between different antibodies. This variability results from the fact that V regions are not encoded by single genes, but, in the case of V_{H} regions, by a number of different V, D and J gene segments, from which one each is randomly selected and then recombined to form the variable coding region of IgH chains (see upper left part of the drawing). This process is called V(D)J recombination and occurs during early B cell development. The IgL chain gene loci contain only a number of different V and J gene segments, from which coding regions for variable domains are generated by a random V to J rearrangement (see upper right part of the drawing). The constant region domains of antibodies from the same subclass (isotype) determine the effector functions of the antibody, and do not differ between different antibodies. However, in the course of an immune response, individual B lymphocytes can be induced to change the isotype of the antibody that is expressed. In this case, a somatic DNA recombination event occurs between switch regions (indicated by a dot in the graphic representation of the constant region genes in the lower part of the drawing) located 5' of the different constant region genes. For instance, recombination between the µ- and ε-switch regions can result in the deletion of all constant region genes from Cµ to Cγ4, as indicated in the drawing. As a result from this, a VDJ variable coding region is brought into proximity to a Cε constant region, leading to the expression of an antibody of IgE isotype.

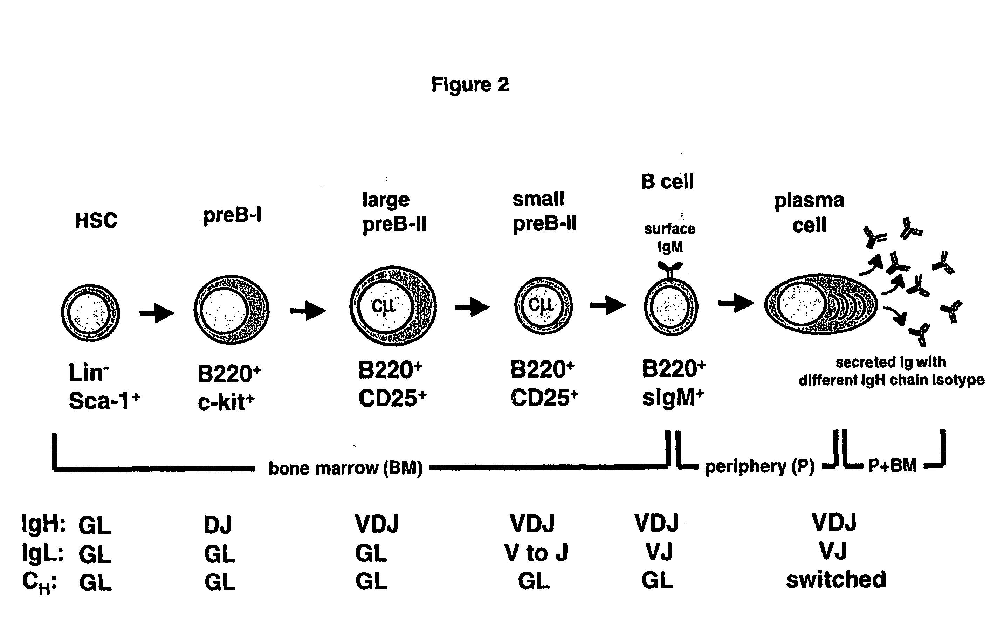

Figure 2 illustrates B cell development in the mouse. The differentiation of B lymphocytes in the mouse can be subdivided into distinct stages that can phenotypically and genotypically be subdivided. The earliest committed precursor B (preB) cells that can be found in the bone marrow of wildtype mice are derived from a pluripotent hematopoietic stem cell (HSC, left), that does not express lineage specific markers (which is designated as Lin⁻), but is characterized by the surface expression of stem cell antigen 1 (Sca-1). From this, preB cells are generated that usually carry DJ_{H} rearrangements on both IgH chain alleles and that are characterized by surface expression of B220 (a pan-B cell marker) and c-kit. At this stage all other Ig gene loci are usually still in germline (GL) configuration. DJ_{H} rearranged preB cells are designated preB-I cells and can be expanded in tissue culture under specific conditions. The next rearrangement event in these cells involves one of the V_{H} gene segments that rearranges to a pre-existing DJ_{H} element. If this leads to a productive rearrangement on any allele such that a µH chain can be expressed, these cells receive a proliferative signal, expand and differentiate into preB-II cells. These cells loose expression of the c-kit surface marker, but gain expression of CD25. A further productive rearrangement on any of the IgL chain alleles results in the differentiation to immature B cells expressing membrane bound IgM. These immature, naïve B cells can leave the bone marrow, migrate into peripheral lymphoid organs, where they might eventually encounter antigen. In the course of an immune response, these B cells can then differentiate into antibody secreting plasma cells, during which another DNA recombination process (class switch recombination) may occur in the IgH constant region locus resulting in the change of antibody isotype that is secreted by the plasma cells.

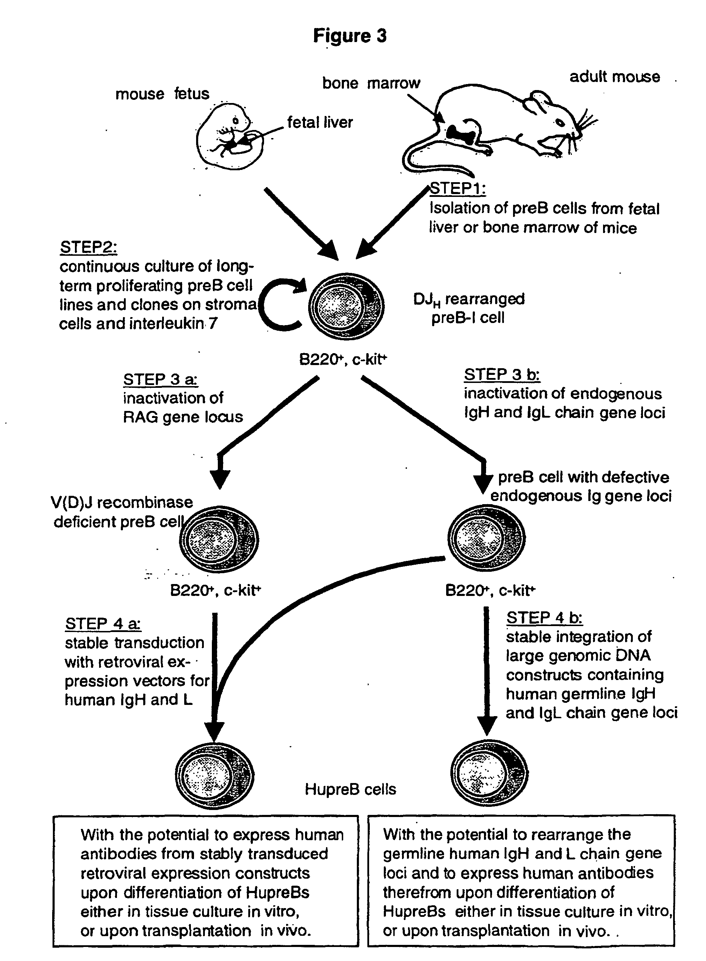

Figure 3 is a schematic overview of the method according to the invention for the generation of humanized precursor B lymphocytes (HupreB's). Murine DJ_{H} rearranged preB-I cells can be isolated from fetal liver of mice 14-19 days post coitum, or from the bone marrow of adult mice (Step 1). The long-term culture of these cells requires special tissue culture conditions, including stromal cell feeder layers and/or the presence of certain growth factors, such as e.g. IL-7 (interleukin 7). Under these conditions, preB-I cells do not differentiate further (Step 2). In order to use these cells for the production of heterologous antibodies, it must be prevented that further productive endogenous Ig gene rearrangements can be performed in these cells. This can e.g. be accomplished by introducing *cis*-acting mutation(s) on both alleles of the IgH and IgL chain gene loci (Step 3b), or, alternatively, by introducing *trans*-acting mutation(s) that ablate further V(D)J recombination of Ig genes, e.g. by deleting one of the two recombination activating genes, RAG-1 or RAG-2 (Step 3a). The latter cells can then be stably transduced with recombinant retroviruses capable of mediating expression of xenogeneic, in this case human, IgH and IgL chains (Step 4a). These retroviral vectors can also be used for the stable transduction of the former cells carrying *cis*-acting mutations in endogeneous Ig gene loci. In addition, preB cells carrying *cis*-acting mutations are still competent for Ig gene rearrangements. It is therefore possible to stably transfect these cells with heretologous germline Ig gene loci, which may undergo V(D)J rearrangements, and from which novel xenogeneic antibodies can be produced (Step 4b). If the heterologous or xenogeneic IgH and IgL chain genes are from human origin, the preB-I cells will only have the potential for the expression of fully human antibodies, and are therefore referred to as HupreB cells.

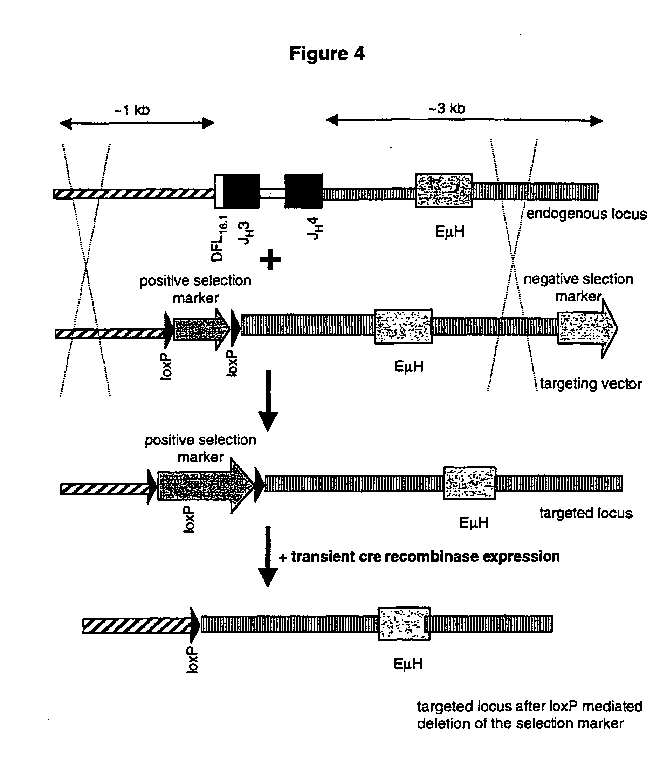

Figure 4 is a schematic representation illustrating the targeting of endogenous gene loci using positive-negative gene targeting vectors. For the targeting of endogenous gene loci, a genomic region of approximately 1 kb upstream (short arm) of that locus and a region of approximately 3 kb (long arm) downstream of that gene is cloned into an empty positive-negative targeting vector. As an example, the targeting strategy for a DJ_{H}3 rearranged IgH chain locus is depicted. This construct is transfected into preB-I cells and integration of the targeting vector by homologous recombination is selected and eventually screened for. The positive selection marker allows selection for the stable integration of the construct into the genome of the transfected cell. The expression of the negative selection marker would be toxic for the cell, and cells will only survive, if the negative selection marker is lost upon stable integration, which occurs by two homologous recombination events at the correct locus. Two possible sites for homologous recombination between the endogenous gene locus and the targeting construct are indicated by hatched lines. If integration of the construct by such homologous recombination events occurs, the positive drug selection marker replaces the endogenous region located in between the short and long arms of homology. If the positive selection marker is flanked by two loxP recombination signals, as indicated here, transient expression of cre recombinase can mediate the deletion of the selection marker, such that the same targeting construct can be used for targeting of the second allele of the same gene locus.

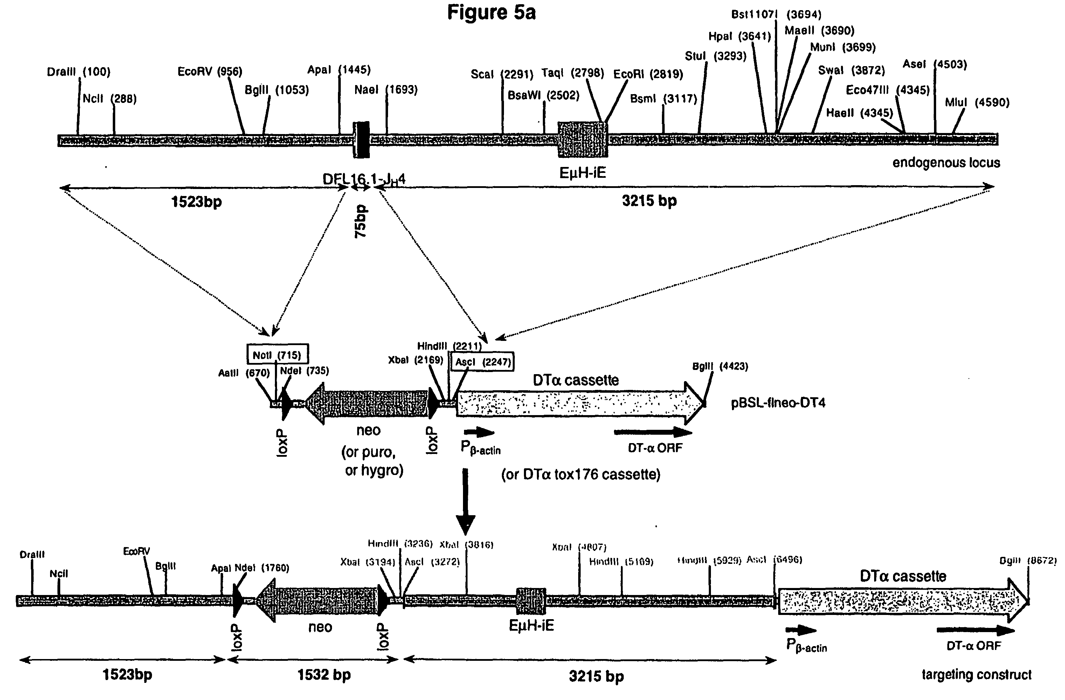

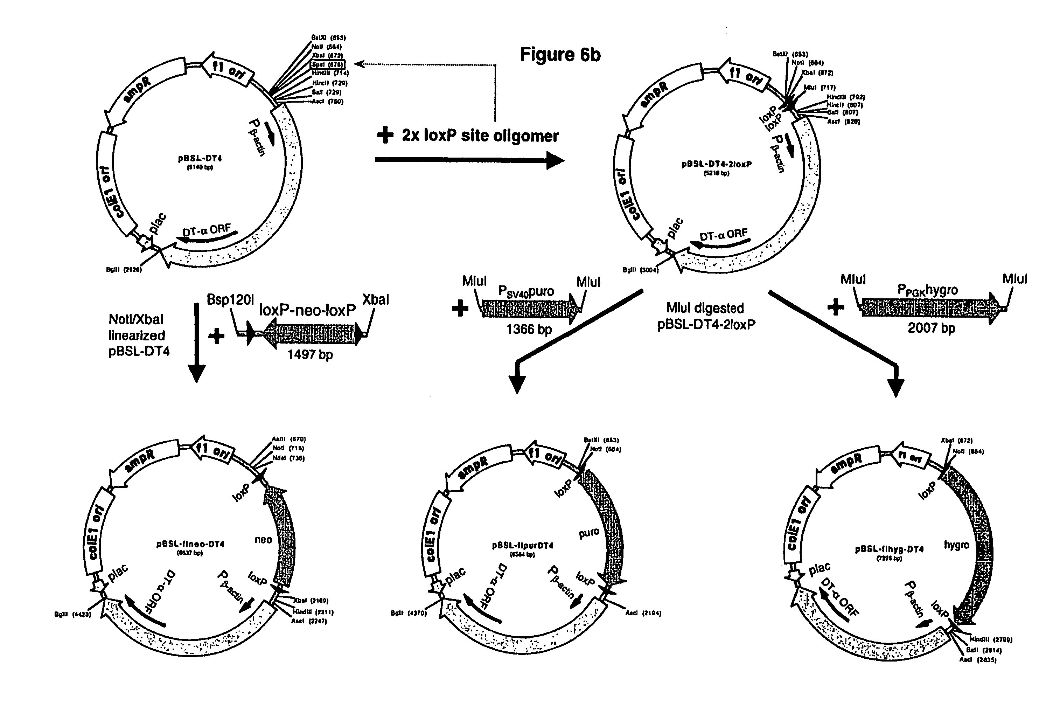

Figure 5a illustrates the cloning strategy for the construction of an IgH chain gene locus targeting vector. The detailed cloning strategy for the construction of one possible targeting construct for DJ_{H} rearranged murine IgH chain gene loci is depicted here at the example of a D_{FL16.1}-J_{H}4 rearranged gene locus. Other DJ_{H} rearranged IgH chain gene loci can be targeted with the same targeting vector. Unique restriction sites are indicated in the endogenous gene locus (top construct) and the regions of 1523 and 3215 bp that are PCR cloned from genomic DNA into the empty positive-negative targeting vector pBSL-flneo-DT4 are indicated (see Fig. 6b, pBSL-flneo-DT4 was chosen as one example of various different empty targeting vectors). The PCR primers for the short and the long arms are designed to contain Not I and Asc I restriction sites for cloning of the fragments into the unique and compatible restriction sites in pBSL-flneo-DT4. The organisation of the final targeting construct is depicted on the bottom, and positions of selected restriction sites are indicated.

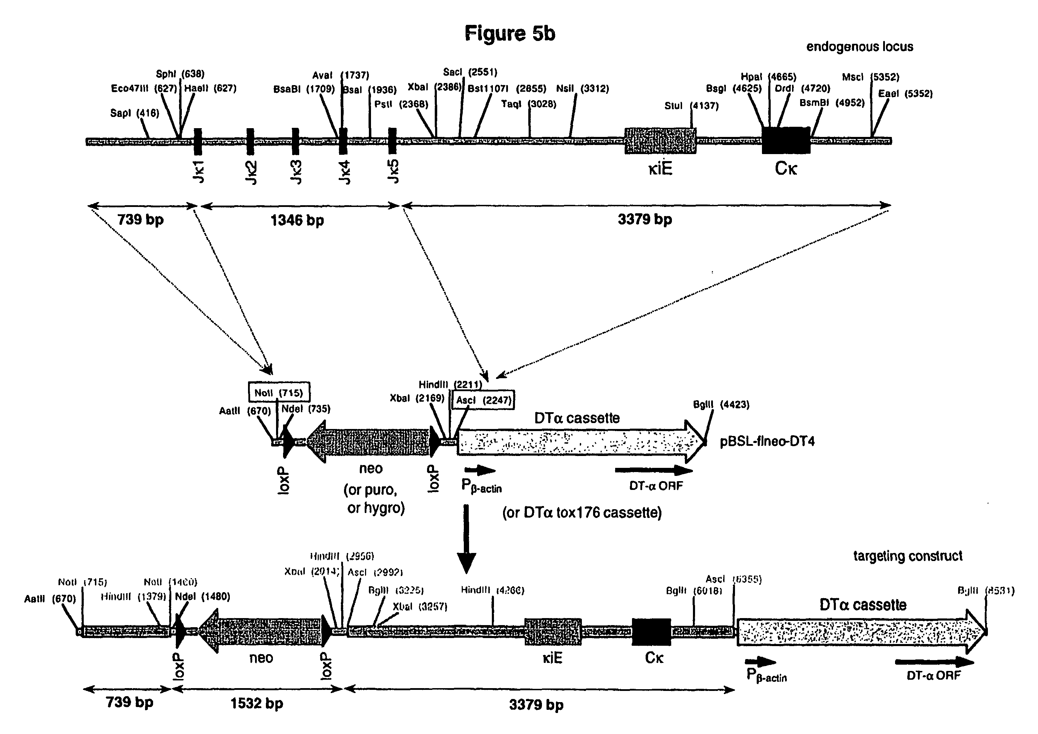

Figure 5b illustrates the cloning strategy for the construction of an IgκL chain gene locus targeting vector. The detailed cloning strategy for the construction of one possible targeting construct for murine IgκL chain gene loci designed for the targeted deletion of all germline Jκ gene segments is depicted here. Unique restriction sites are indicated in the endogenous gene locus (top construct) and the regions of 739 and 3379 bp that are PCR cloned from genomic DNA into the empty positive-negative targeting vector pBSL-flneo-DT4 are indicated (see Fig. 6b, pBSL-flneo-DT4 was chosen as one example of various different empty targeting vectors). As in Fig. 5a, PCR primers for the short and the long arms are designed to contain Not I and Asc I restriction sites for cloning of the short and long arms into compatible restriction sites of pBSL-flneo-DT4. The organisation of the final targeting construct is depicted on the bottom, and positions of selected restriction sites are indicated.

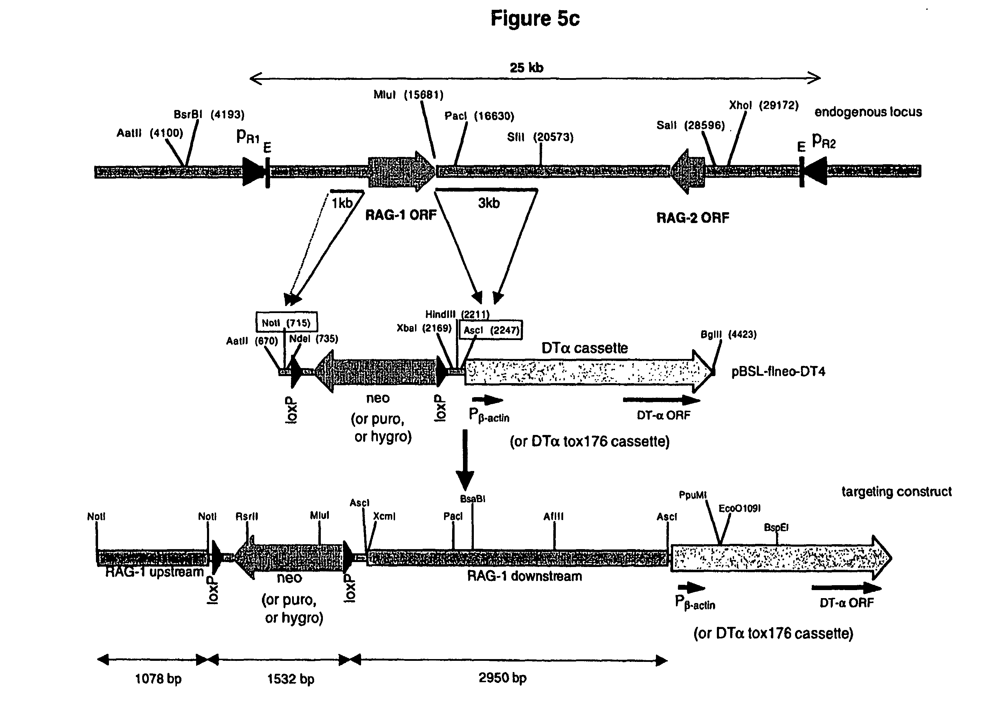

Figure 5c illustrates the cloning strategy for the construction of a RAG gene locus targeting vector. The detailed cloning strategy for the construction of one possible targeting construct for the murine RAG gene locus designed for the targeted deletion of the RAG-1 gene is depicted here. The RAG gene locus in the mouse is located on chromosome 2 and contains the two very closely linked genes, RAG-1 and RAG-2, that are each essential for Ig gene rearrangements. The open reading frames (ORFs) of both genes are encoded by one single exon, but a short untranslated exon downstream of each promoter element (indicated by triangles) is spliced to one large exon containing the entire RAG ORFs. Unique restriction sites are indicated in the endogenous gene locus (top construct). Genomic regions of 1078 and of 2950 bp up and downstream of the RAG-1 ORF, respectively, are PCR cloned from genomic DNA into the empty positive-negative targeting vector pBSL-flneo-DT4, as indicated (see Fig. 6b for pBSL-flneo-DT4, which was chosen as one example of various different empty targeting vectors). As in Fig. 5a, PCR primers for the short and the long arms are designed to contain Not I and Asc I restriction sites for cloning of the short and long arms into compatible restriction sites of pBSL-flneo-DT4. The organisation of the final targeting construct is depicted on the bottom, and positions of selected restriction sites are indicated.

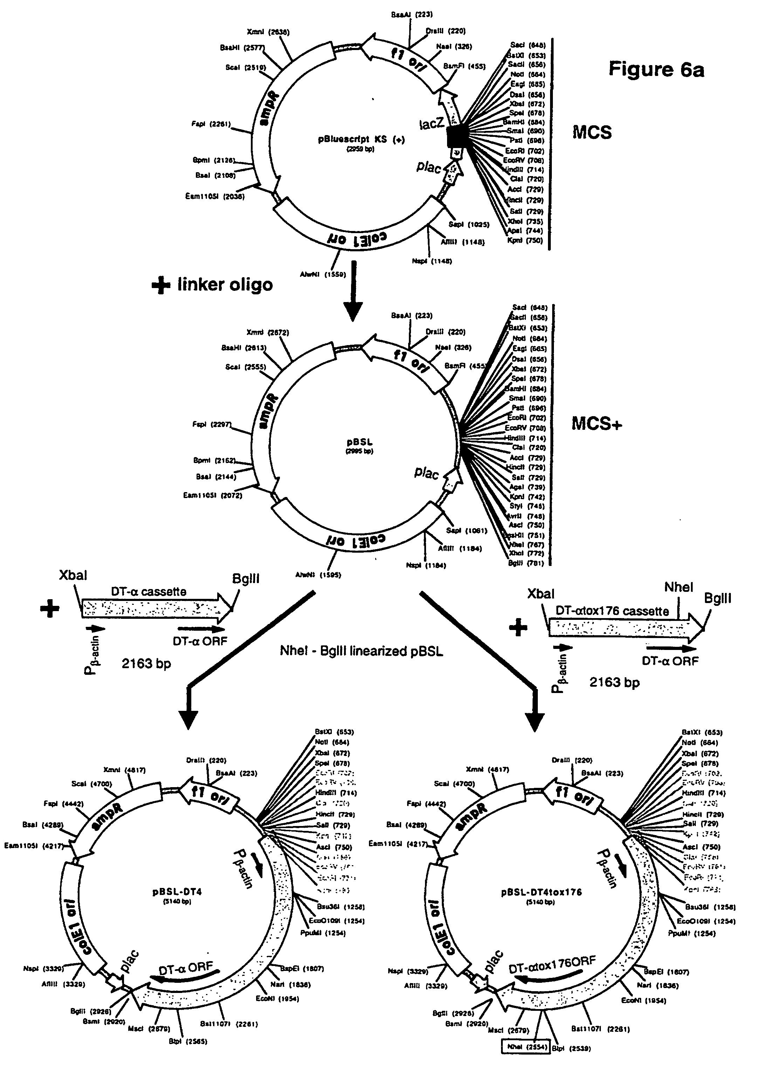

Figure 6a shows the detailed cloning strategy for DT-α and DT-α(tox176) based positive-negative vectors for gene targeting (preparatory steps). A prerequisite for the generation of empty targeting vectors is to extend the multiple cloning site (MCS) of a regular cloning plasmid, like pBluescript, such that additional useful restriction sites are added to the polylinker, which can then be used for the insertion of expression cassettes for positive and negative selection markers, as well as for genomic regions required for the targeting of an endogenous gene locus by homologous recombination. Depicted here is the insertion of an additional oligomer with additional restriction sites into the MCS of pBluescript, thereby generating plasmid pBSL with an extended polylinker. This construct is used to insert a β-actin promoter driven expression cassette for either wild-type diphteria toxin α or an attenuated version thereof, denoted DTα(tox176) (cf. example 5b, step 2). These two constructs with extended multiple cloning site are designated pBSL-DT4 (bottom, left) and pBSL-DT4tox176 (bottom, right), respectively. The diphtheria toxin expression cassettes are cloned into pBSL using Xbal and Bglll restriction sites, as indicated. A diagnostic Nhel restriction site that is unique for the attenuated DTtox176 expression cassette is indicated as well.
Figure 6b illustrates the detailed cloning strategy for DT-α and DT-α(tox176) based positive-negative vectors for gene targeting (final cloning steps). Cloning of empty positive-negative targeting vectors containing either neomycin, hygromycinB, or puromycin as positive selection markers and a wild-type diphtheria toxin α expression cassette are described. The same cloning strategies can be used for vectors containing an expression cassette for DT-α(tox176) instead. Restriction enzymes used for cloning of fragments and linearization of constructs are indicated. All positive selection markers are designed to be flanked by IoxP sites (floxed) which can be recognized by cre recombinase. While a floxed neomycin expression cassette can be recloned from the existing vector pGL2-neo(m)+ (DSM 14705), floxed hygromycinB and floxed puromycin expression cassettes needed to be inserted in two steps. For this, a synthetic DNA oligomer containing two IoxP sites separated by a Mlul site was inserted into pBSL-DT4 in order to generate pBSL-DT4-2loxP, as indicated. In a final step, expression cassettes for hygromycinB and puromycin resistance were PCR cloned into the unique Mlul site of pBSL-DT4-2loxP.

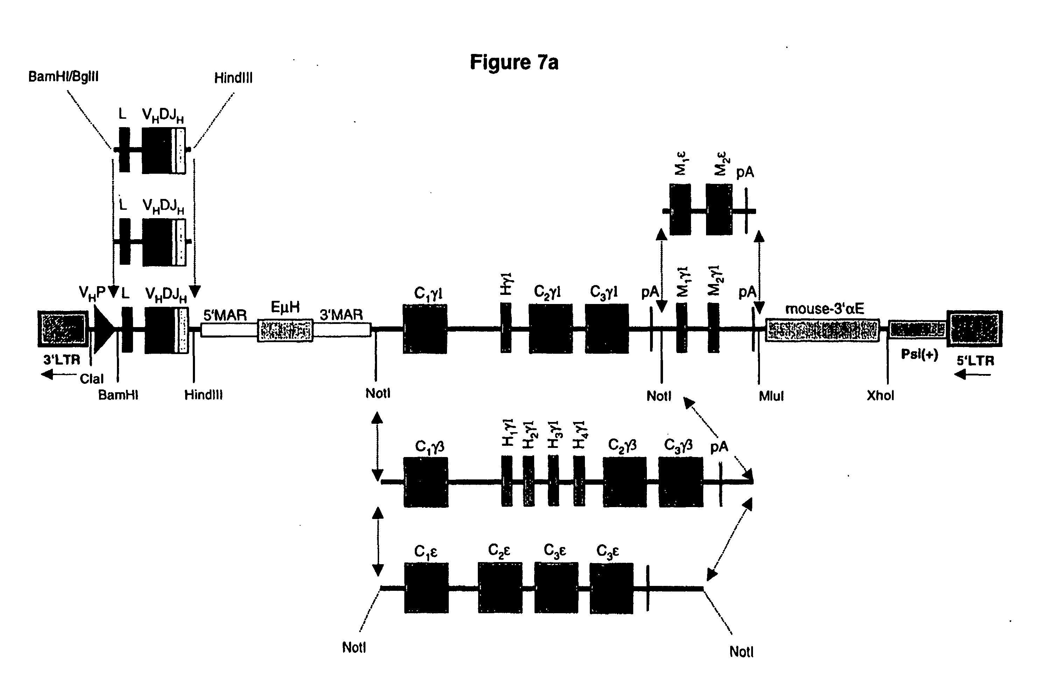

Figure 7a shows the schematic organization of retroviral expression vectors for human IgH chains. Recombinant retroviral vectors allowing the expression of human IgH chain proteins exhibit a modular design containing Ig locus promoter and enhancer elements, as well as coding regions for the variable and constant region domains of IgH chains. The transcriptional orientation of the IgH expression cassette is opposite to the retroviral 5'LTR promoter element in order to confer a B cell specific expression pattern based on the Ig specific promoter and enhancer elements. The expression cassette contains a murine V_{H} promoter a leader (L) sequence with VDJ exons encoding IgH variable domains, the murine intron heavy chain enhancer (EµH), exons encoding the constant regions of heterologous IgH chains, exons for the membrane spanning regions of immunoglobulins, and elements from the murine IgH 3'α enhancer. Different L-VDJ regions, and even libraries of L-VDJ regions may be cloned into the unique BamHI and HindIII restriction sites of the IgH expression vector using restriction enzymes generating compatible overhangs. Due to the modular design, constant region exons or enhancer elements may be replaced using simple molecular biology techniques (as indicated).

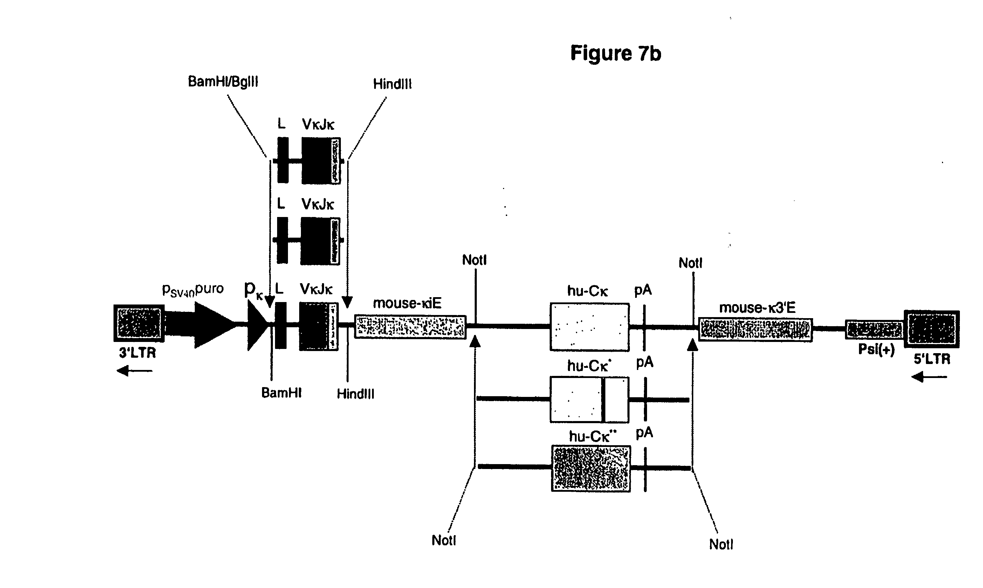

Figure 7b shows the schematic organization of retroviral expression vectors for human IgκL chains. Like the vectors depicted in Fig. 7a, recombinant retroviral vectors allowing the expression of human IgκL chain proteins exhibit a similar modular design containing IgL chain specific locus promoter and enhancer elements, as well as coding regions for the variable and constant region domains of IgκL chains. The transcriptional orientation of the IgκL expression cassette is opposite to the retroviral 5'LTR promoter element, in order to confer a B cell specific expression pattern based on the Ig specific promoter and enhancer elements. The expression cassette contains a murine Vκ promoter, a leader (L) sequence with VJ exons encoding IgκL variable domains, the murine κ intron enhancer (κiE), exons encoding the constant region of heterologous IgL chains, and elements from the murine Ig κ 3' enhancer (κ3'E), as indicated. In addition, retroviral IgκL chain expression vectors contain a complete puromycin expression cassette, allowing the selection of stably transduced preB cells. Different L-VJ regions, and even libraries of L-VJ regions may be cloned into the unique BamHI and HindIII restriction sites of the IgH expression vector using restriction enzymes generating compatible overhangs. Due to the modular design, modified constant region exons or enhancer elements may be replaced using simple molecular biology techniques (as indicated).

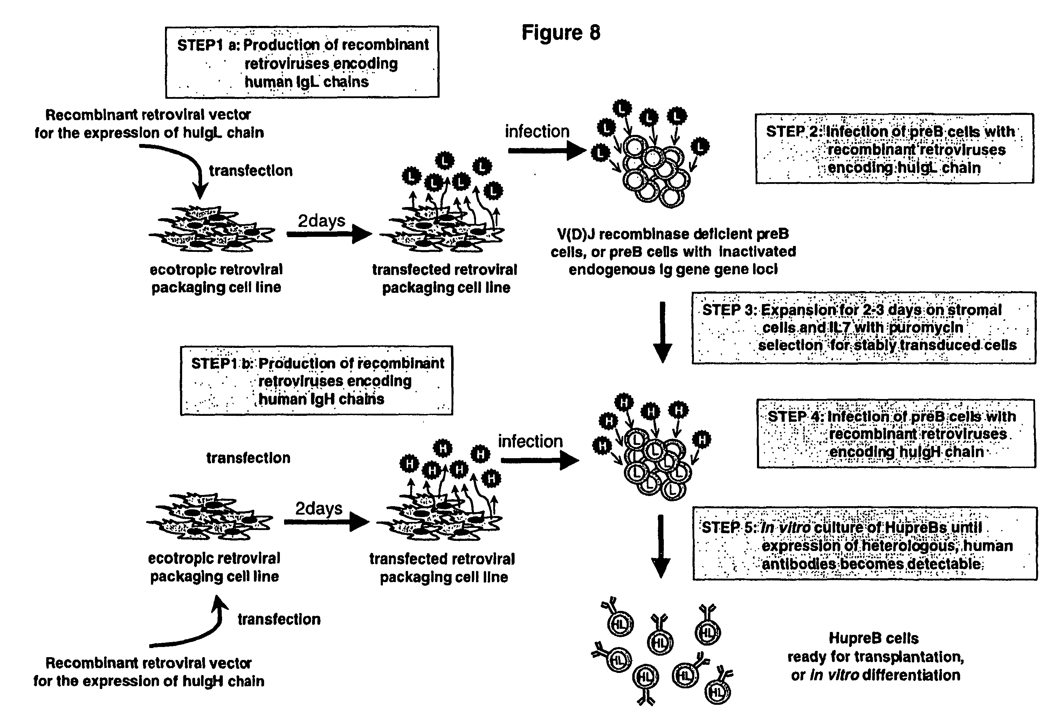

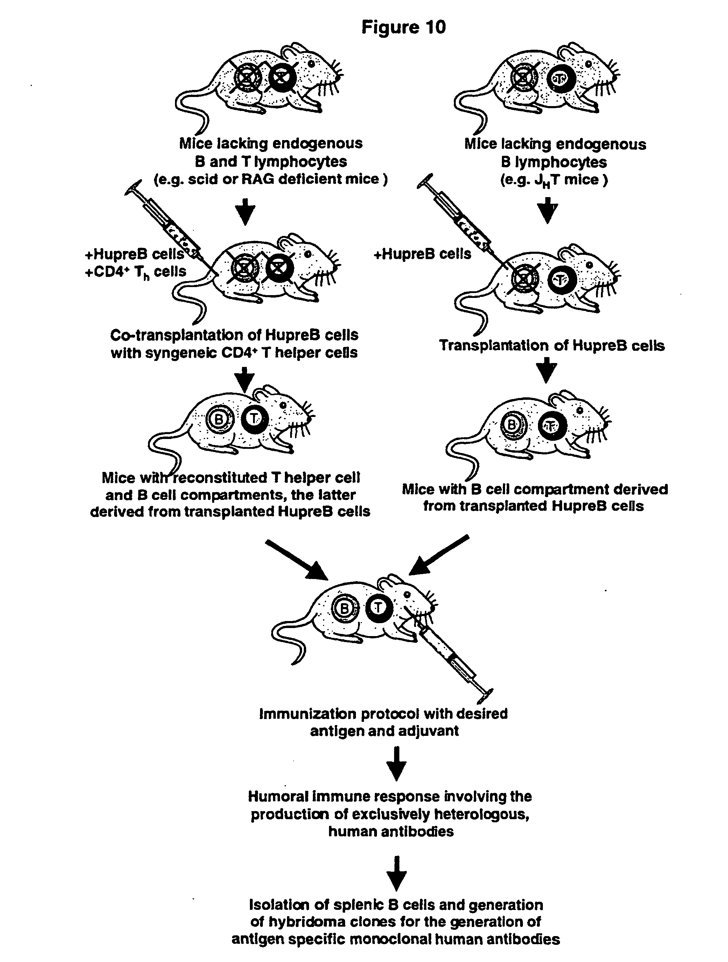

Figure 8 illustrates the procedure for the retroviral transduction of murine stromal cell/IL7 dependent preB cells with retroviral vectors encoding heterologous IgH and IgL chains. Long-term proliferating stromal cell/IL7 dependent preB cell lines with mutations interfering with endogenous Ig gene rearrangements (cf. Fig. 3) can be sequentially transduced with retroviral expression vectors encoding IgL chains and IgH chains, as indicated. After successive transduction with first heterologous IgL and then IgH chain expressing retroviral constructs, the cells have the potential to express heterologous antibodies upon differentiation of the cells either *in vitro,* or *in vivo* (see Fig. 10).

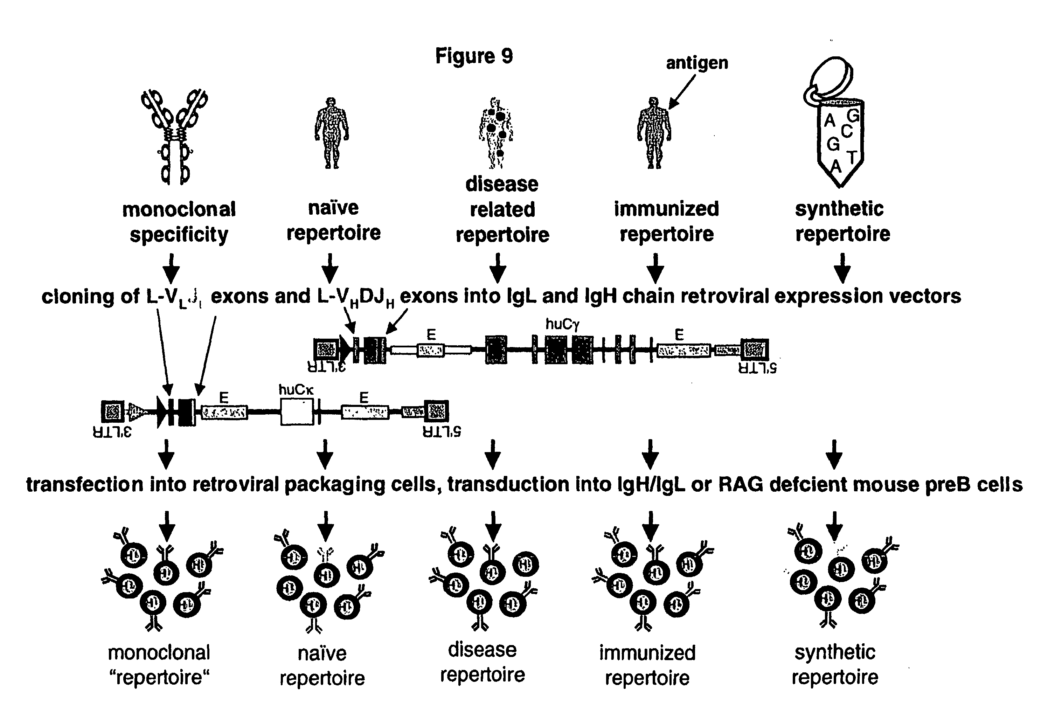

Figure 9 is a schematic overview of sources, from which variable domain coding regions can be isolated. Variable domain coding regions can be isolated from many different sources, as indicated in this figure. The modular design of the retroviral vectors allows the cloning of retroviral vectors encoding e.g. only a single specificity, for instance from an existing useful monoclonal antibody. Upon transduction of these vectors and transplantation of transduced cells into mice *in vivo* (see Fig. 10), single specificities may then be subjected to affinity maturation. Alternatively, complete VDJ and VJ libraries may be isolated from peripheral blood lymphocytes (PBLs) from either healthy, sick or immunized patients and then cloned into IgH and IgL chain retroviral expression vectors. In fact, it is even possible to generate completely synthetic V_{H} and V_{L} region repertoires, that may be cloned into retroviral Ig expression vectors, which can subsequently be stably transduced and functionally selected *in vivo* (see Fig. 10).
Figure 10 illustrates transplantation of HupreBs into immunodeficient mice for the production of fully human monoclonal antibodies. HupreB cells, like normal murine preB cells, can be transplanted into murine hosts, where they are able to participate in immune responses of the host. If HupreB cells are transplanted into immunodeficient hosts lacking endogenous B cells, the only antibodies that can then be produced are heterologous (human) antibodies from the transplanted HupreB cells. There are many different host strains that may be used for the transplantation of HupreB cells. Strains that are devoid of both B and T lymphocytes are e.g. the RAG-1- and RAG-2-deficient and *scid* mouse strains. In order to be able to elicit a T cell dependent immune response involving B lymphocytes derived from transplanted HupreB cells, T helper lymphocyte populations can be isolated and co-transplanted into these mice (left side). Alternatively, mouse strains lacking only B lymphocytes, like e.g. J_{H} deficient mice, can be reconstituted by transplantation with HupreB cells alone (right side). Once the peripheral B and T lymphocyte compartments are reconstituted after transplantation, these mice can be immunized using convenient immunization protocols against any desired antigen. Plasma cells derived from these mice can then be immortalized, e.g. by fusion with myeloma cells, in order to produce (hybridoma) cells capable of permanently secreting heterologous (human) monoclonal antibodies specific for the injected immunogenic compound or composition.

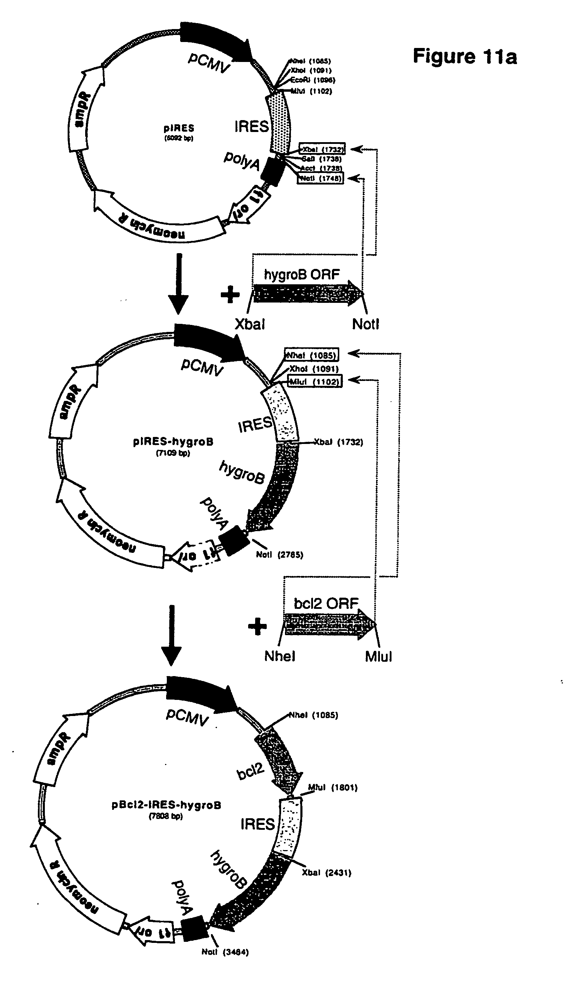

Figure 11a illustrates the construction of a retroviral expression vector for constitutive expression of the anti-apoptotic gene bcl-2 (preparatory steps). Removal of IL-7 from continuously proliferating preB cell cultures *in vitro* results in the differentiation of preB cells, but at the same time also leads to the induction of apoptosis. This apoptosis can be prevented by constitutive expression of an anti-apoptotic gene, like e.g. bcl-2. For the generation of a selectable retroviral expression vector for bcl-2, the murine bcl-2 cDNA is sequentially cloned with an hygromycin B open reading frame into a dual gene expression vector pIRES. This vector contains an internal ribosomal entry sequences flanked by two multiple cloning sites, into which two different genes can be cloned. This allows the simultaneous expression of two genes in mammalian cells from one single promoter. The strategy for the insertion of the bcl-2 ORF and the hygromycin B selection marker gene into pIRES for the generation of a pBcl2-IRES-hygroB vector is indicated.

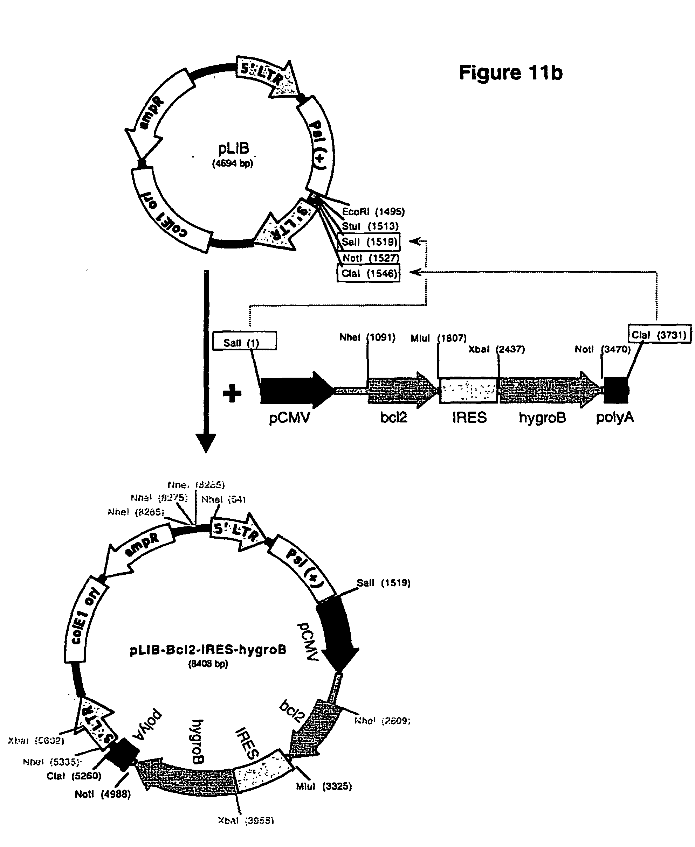

Figure 11 b illustrates the construction of a retroviral expression vector for constitutive expression of the anti-apoptotic gene bcl-2 (final cloning step). After assembly of the CMV promoter driven bcl-2-IRES-hygromycinB cassette, the entire dual expression cassette from the pBcl2-IRES-hygroB vector is recloned as a Sall-Clal fragment into the retroviral transfer vector pLIB, as depicted. This generates the recombinant retroviral construct pLIB-Bcl2-IRES-hygroB that can be used to stably transduce preB cells and to confer simultaneous expression of bcl-2 and resistance to hygromycinB.

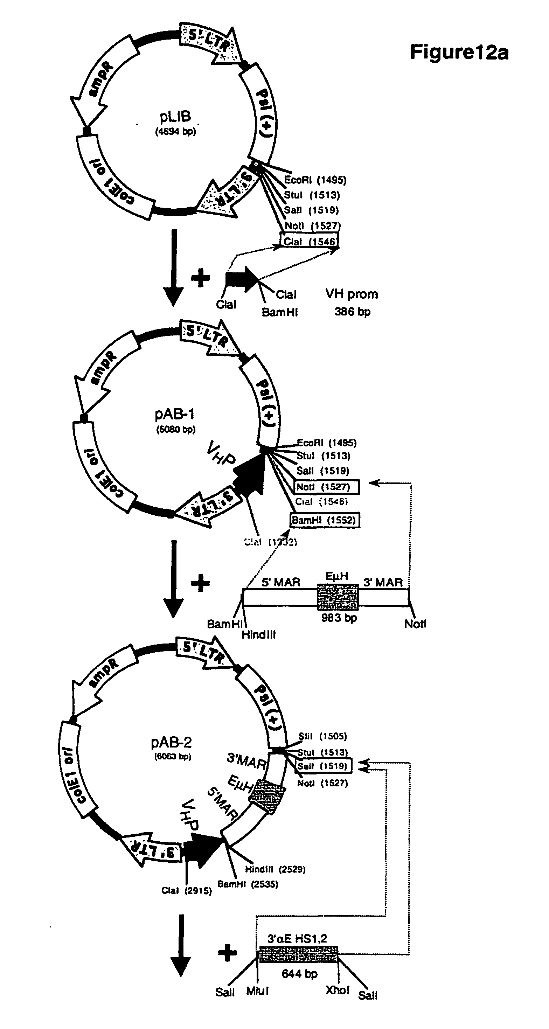

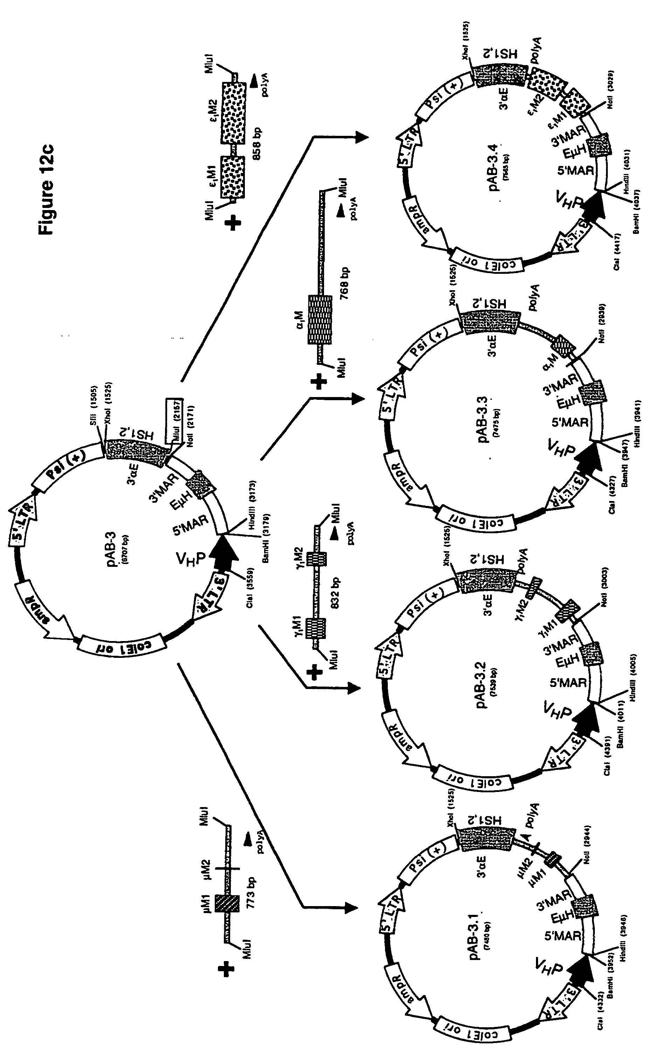

Figure 12a examplifies the construction of retroviral expression vectors for the expression of human IgH chains (preparatory step I). The various IgH promoter and enhancer elements, as well as the coding regions for the different variable and constant region domains, are sequentially cloned into a retroviral transfer vector, like e.g. plasmid pLIB. This plasmid contains all elements required for retroviral packaging and provirus integration, i.e. a 5' LTR, a packaging signal, and a 3' LTR, cloned into a bacterial, ampicillin selectable plasmid backbone with a ColE1 origin of replication (as indicated). Depicted here are the first cloning steps for inserting a V_{H}promoter, the murine heavy chain intron enhancer (EµH), including flanking matrix attachmant regions (MAR) and the IgH chain 3'α enhancer (3'αE). Restriction enzymes incorporated at the termini of PCR amplified fragments, as well as unique compatible restriction enzyme sites in the plasmid backbone, which can be used in the various cloning steps, are highlighted.

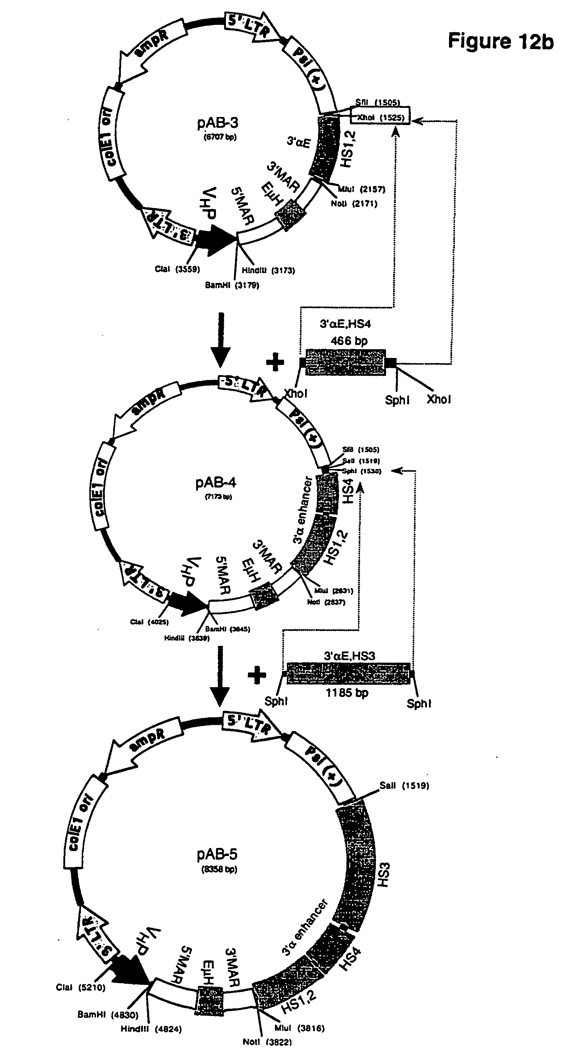

Figure 12b examplifies the construction of retroviral expression vectors for the expression of human IgH chains (preparatory step II). The IgH 3'α enhancer contains four functional regions, which upon activation of the enhancer during terminal B cell differentiation become sensitive to DNasel digestion and are therefore referred to as hypersensitive regions HS 1, 2, 3, and 4. Each of these HS regions contributes to the enhancer activity of the 3'αE, but most of the enhancer activity is conferred by the HS1,2 regions. Although the HS1,2 region of the 3'αE alone is able to drive reporter gene expression to almost the same levels as the entire 3'αE region, it may be desirable for some retroviral IgH expression constructs to include the other functional regions of the 3'αE. Therefore, the cloning of the HS3 and HS4 regions of the 3'αE is depicted here. Restriction enzymes incorporated at the termini of genomic PCR fragments, as well as compatible restriction enzyme sites in the plasmid backbone, which can be used in the various cloning steps, are highlighted.
Figure 12c shows the construction of retroviral expression vectors for the expression of human IgH chains (preparatory step III). Following the cloning of IgH promoter and enhancer elements into a retroviral transfer vector, coding regions for IgH chains need to be inserted. In order to be able to express membrane bound, as well as secreted forms of immunoglobulins, exons for the membrane spanning regions of IgH chains need to be included in the retroviral expression vectors. The membrane spanning regions differ significantly between different isotypes in length, primary sequence and exon/intron structure. Therefore, different membrane spanning regions for IgM, IgG, IgA and IgE heavy chains need to be cloned. As an example, the PCR cloning and insertion of the four respective different membrane spanning regions into plasmid pAB-3 is depicted. The same cloning procedures can be performed with vectors pAB-4 and pAB5, containing extended versions of the IgH 3'αE. Restriction enzymes incorporated at the termini of genomic PCR fragments, as well as compatible restriction enzyme sites in the plasmid backbone, which can be used in the various cloning steps, are highlighted.

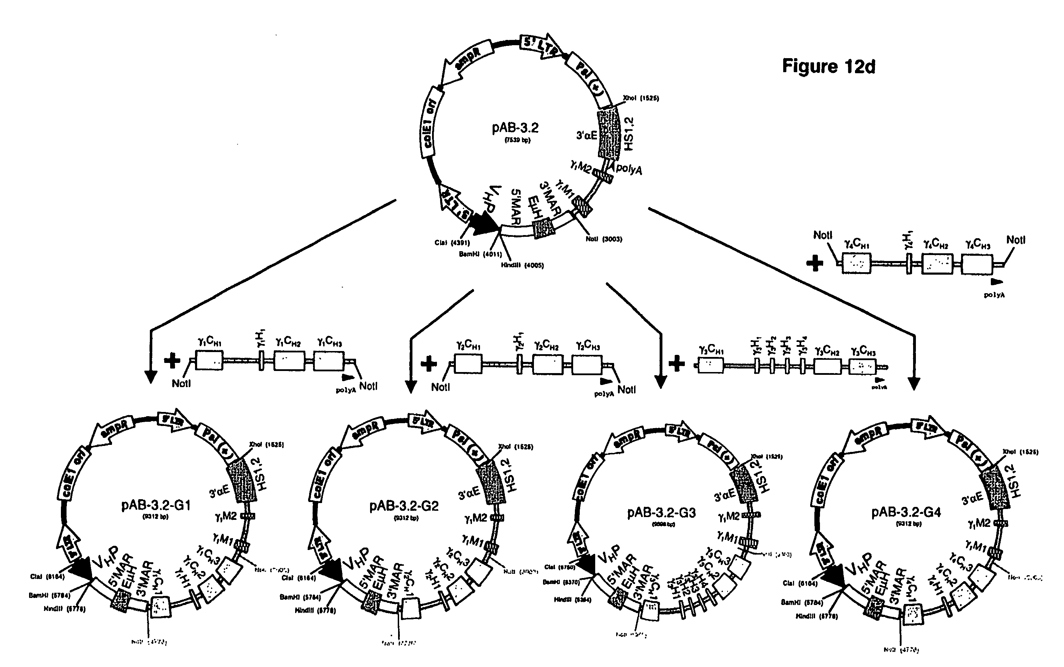

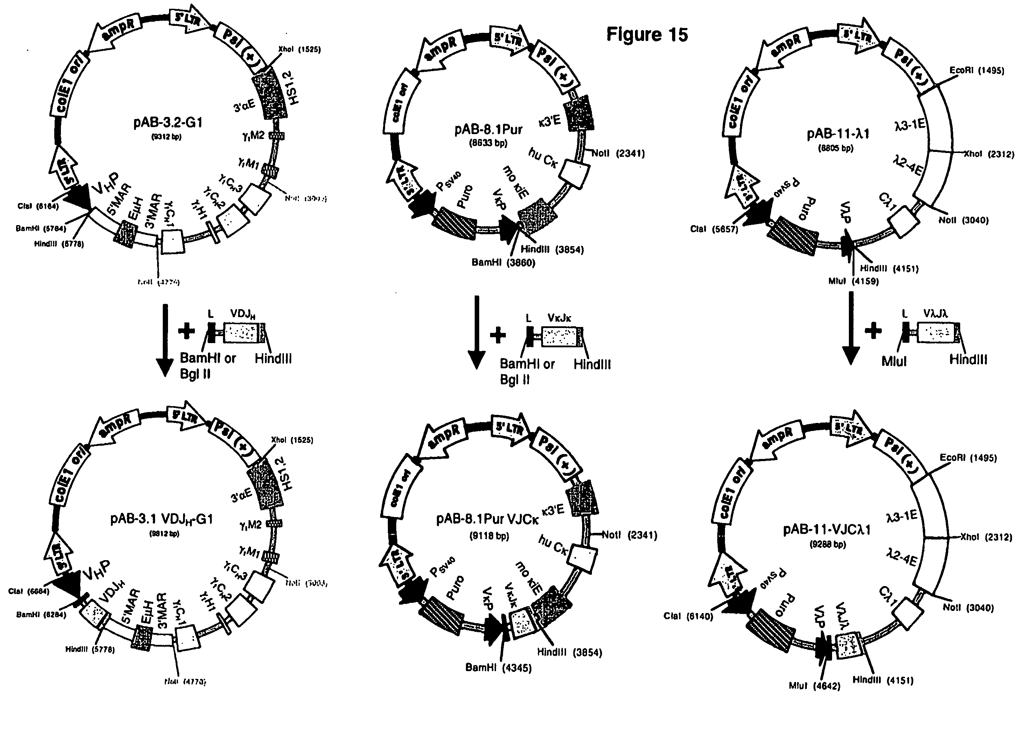

Figure 12d illustrates the construction of retroviral expression vectors for the expression of human IgH chains (preparatory step IV). Human B lymphocytes are able to produce four different IgG isotypes, IgG1, IgG2, IgG3, and IgG4, that differ slightly in sequence and that exhibit different physiologic effector functions. The cloning of the constant region genes into an intermediate vector pAB-3.2, already containing IgG membrane spanning exons, is depicted here. The endogenous exon/intron organisation including the exons encoding the hinge (H) regions for the various IgG isotypes is indicated. Restriction enzymes incorporated at the termini of genomic PCR fragments, as well as unique compatible restriction enzyme sites in the plasmid backbone, which can be used in the various cloning steps, are indicated. Upon cloning of a VDJ rearranged variable domain exon, these constructs are capable of expressing complete IgG chains (see Fig. 15).

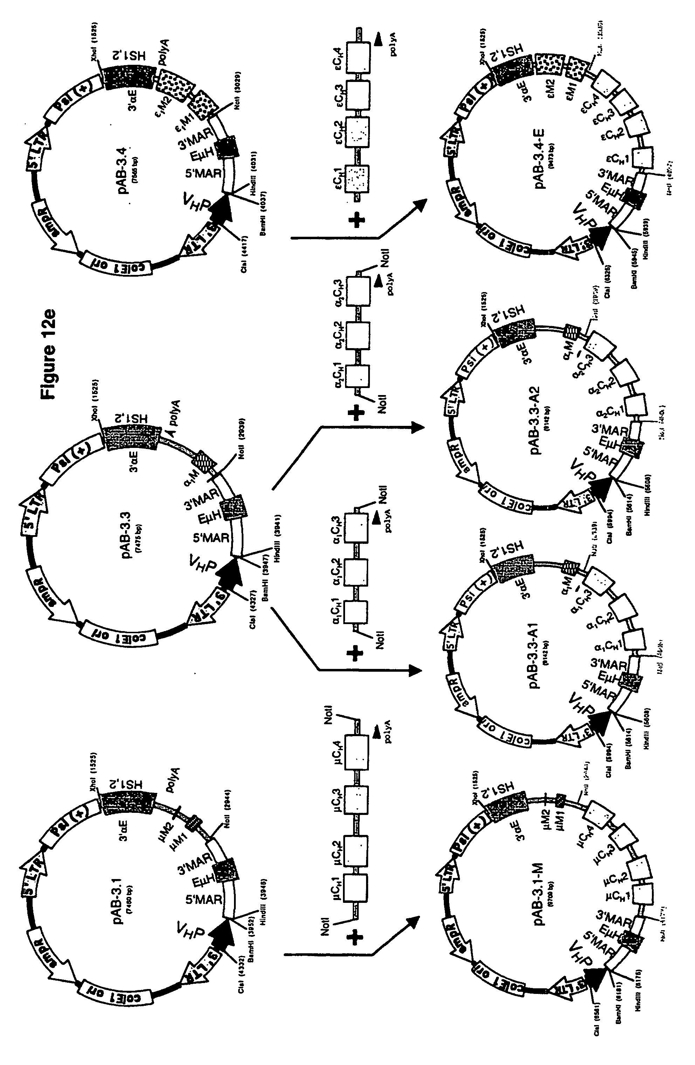

Figure 12e shows the construction of retroviral expression vectors for the expression of human IgH chains (preparatory step V). The cloning of constant region genes for the remaining human Ig isotypes IgM, IgA1, IgA2, and IgE is depicted here. Similar to the cloning procedure for IgG constant region genes, depicted in Fig. 12d, the genomic regions for these isotypes are inserted as Notl digested PCR fragments into unique Notl restriction sites upstream of the respective membrane spanning exons for these isotypes. The endogenous exon/intron organisation including the exons encoding the hinge (H) regions for the various Ig isotypes is indicated. Restriction enzymes incorporated at the termini of genomic PCR fragments, as well as unique compatible restriction enzyme sites in the plasmid backbone, which can be used in the various cloning steps, are indicated. Upon cloning of a VDJ rearranged variable domain exon, these constructs are capable of expressing complete IgH chains of the respective isotype (see Fig. 15).

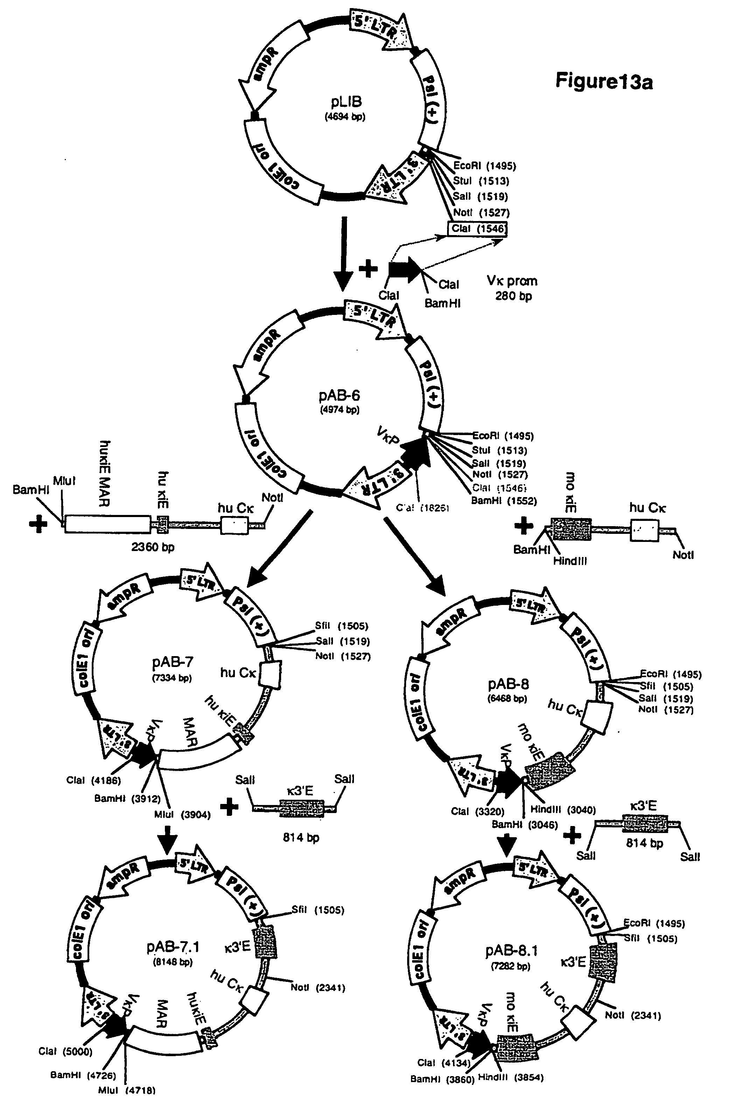

Figure 13a shows the construction of retroviral expression vectors for the expression of human IgκL chains (preparatory step I). Similar to the construction of retroviral expression vectors for IgH chains, expression vectors for IgκL chains require the κL chain locus specific promoter and enhancer elements in addition to the coding regions for the κL chain proteins, which need to be sequentially cloned into a retroviral transfer vector. Starting from the basic retroviral vector pLIB, first, a Vκ promoter is cloned into pLIB, as indicated. This is followed by the insertion of a PCR product containing the human κ intron enhancer (κiE, with its upstream matrix associated region, MAR) and the human κL chain constant region gene (left side of the drawing), or, alternatively, by the insertion of a fusion PCR fragment of the mouse κiE with the human κL chain constant region gene (right side of the drawing), which is generated by a single overlap extension (SOE) PCR. Into both constructs, a PCR product containing the murine κ 3' enhancer (κ3'E) is inserted. Restriction enzymes incorporated at the termini of the various genomic PCR fragments, as well as unique compatible restriction enzyme sites in the plasmid backbone, which can be used in the various cloning steps, are indicated.

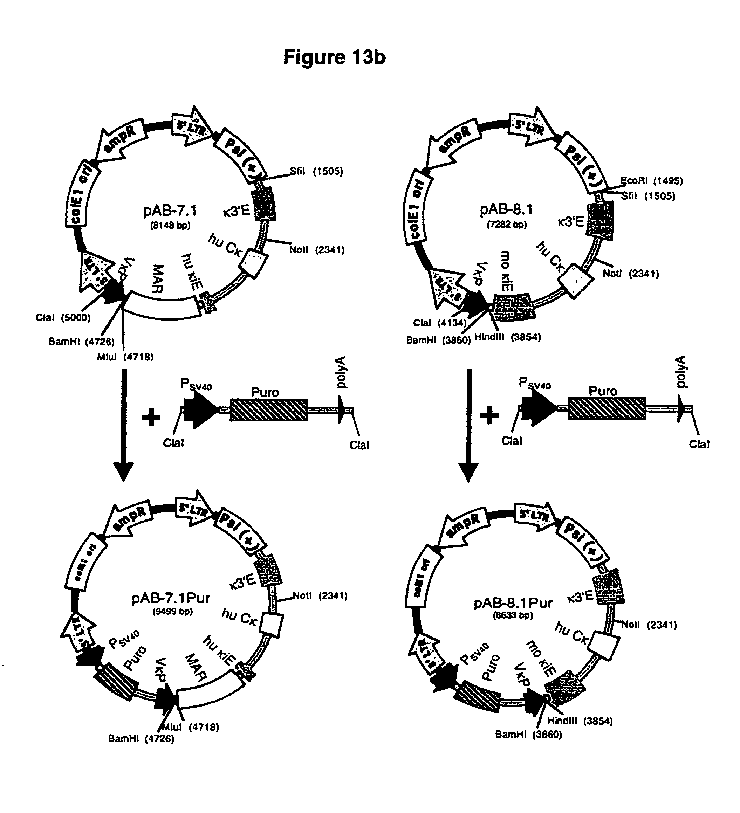

Figure 13b illustrates the construction of retroviral expression vectors for the expression of human IgκL chains (preparatory step II). Retroviral expression constructs for human IgH and IgκL chains will be sequentially transduced into preB cells, and it is therefore desireable to be able to select for stably transduced cells after the first round of transduction with IgκL chain expressing vectors. For this reason a SV40 promoter driven puromycin expression cassette is inserted into the two possible IgκL chain retroviral transfer vector intermediates pAB-7.1 and pAB-8.1, as indicated. Upon cloning of a VκJκ rearranged variable domain exon into these vectors, these retroviral constructs are capable of expressing human IgκL chains (see Fig. 15).

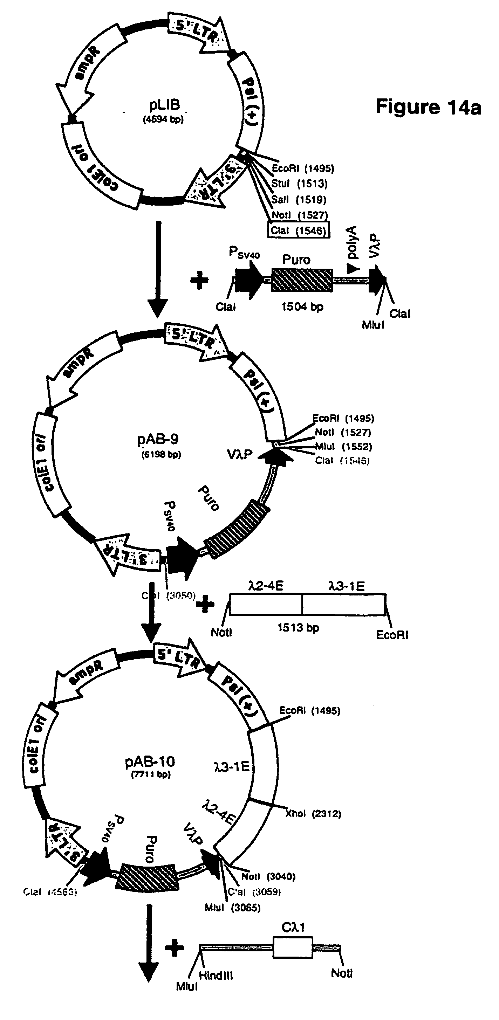

Figure 14a shows the construction of retroviral expression vectors for the expression of human IgλL chains (preparatory step I). Retroviral expression constructs for human IgλL chains are cloned by first inserting a fusion PCR product of a puromycin cassette and a Vλ promoter into pLIB (the PCR fusion is performed by single overlap extension (SOE) PCR as described in the example section). In the next step, a SOE-PCR fusion construct of two murine λL chain enhancer elements, the λ2-4 and the λ3-1 enhancer, are inserted into the construct, as indicated. Finally, the coding region of the human Cλ1 region is inserted by PCR cloning into this retroviral cloning vector using the indicated restriction enzymes. Upon cloning of a VλJλ rearranged variable domain exon into these vectors, these retroviral constructs are capable of expressing human IgλL chains (see Fig. 15).

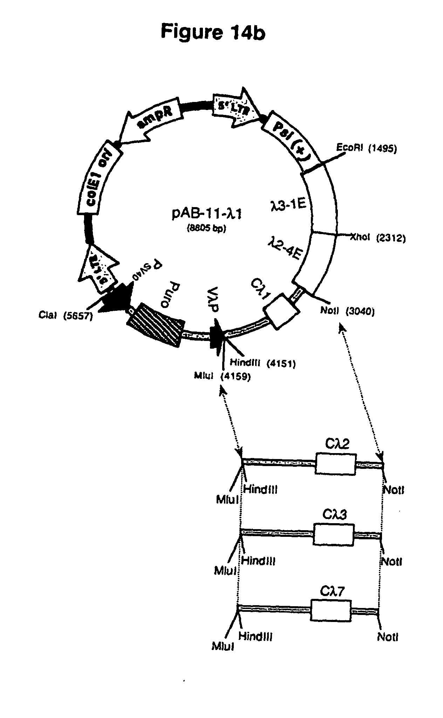

Figure 14b illustrates the construction of retroviral expression vectors for the expression of human IgλL chains (preparatory step II). Other human IgλL chain constant region genes may be used to replace the Cλ1 coding region by simple cloning using the unique Mlul-Notl restriction enzymes, as indicated. Upon cloning of VλJλ rearranged variable domain exons into these vectors, these retroviral constructs are capable of expressing the different human IgλL chain isotypes, as indicated (see Fig. 15).
Figure 15a shows the insertion of V-region genes into retroviral human IgH, IgκL and IgλL chain expression vectors. All retroviral expression constructs for human IgH and L chains that have been described so far did not contain coding regions for the variable domains. These can be isolated by PCR cloning from various sources (see e.g. Fig. 9) and may be inserted into the retroviral vectors as V region exons encoding a single specifity, or as libraries encoding different specificities (which need to be PCR amplified with degenerate primer pairs). V regions are PCR amplified together with their characteristic leader (L) sequences, as indicated. Restriction enzymes that can be used for the cloning of variable region exons are indicated. This drawing depicts some selected final retroviral IgH and IgL chain expression vectors. Due to the modular design of these vectors, different coding regions and promoter and enhancer elements may be used.

### Definition of terms used throughout the specification

**Affinity:** The strength of the binding of an antigen receptor (or of any receptor) for its cognate antigen (or its ligand or binding partner), determined by the ratio of association to dissociation rate of the receptor-ligand interaction.

**Affinity maturation:** A highly regulated immunological process of antigen driven improvement of the binding specificities of antibodies produced by antigen stimulated B lymphocytes in germinal centers. The process is caused by somatic hypermutation within the coding regions for the variable domains of antibodies coupled with the selective expansion and survival of B lymphocytes generating higher affinity antibodies.

**Antibody:** A glycosylated polypeptide produced by B lymphocytes and plasma cells comprising in its monomeric form two identical heavy (H) chains and two identical light chains, each being composed of one variable domain and one (L chains) or several (H chains) constant region domains. The two H and two L chains assemble into a symmetric Y shaped disulphide linked antibody molecule that has two binding domains formed by the combination of the variable regions of H and L chains.

**Antigen:** Any biomolecule or chemical entity that can be bound by the variable domains of immunoglobulins (or antibodies).

**Antigen receptor:** Antigen receptors are composed of variable domains and constant regions, the former having the ability to bind to antigens, and the latter to mediate effector functions or to transduce signals into cells. Antigen receptors comprise T cell receptors and membrane bound immunoglobulins.

**Artificial binding protein:** A binding protein containing polypeptide sequences (e.g. synthetic spacer sequences) not derivable from natural genes or fragments thereof.

**Binding protein:** The most general term for any type of polypeptide with the ability to selectively bind to an antigen or ligand, with or without additional effector functions. Binding proteins include artificial binding proteins, fragments of antibodies, like e.g. a single chain variable fragment (scFv), and also fusion products combining variable and constant region domains of immunoglobulins and T cell receptors, or vice versa. Furthermore, a binding protein can be the fusion product of variable domains with functional parts of other receptor molecules. Conversly, a binding protein can be the fusion of a binding moiety from a non-Ig or TCR related receptor with constant region domains of antigen receptors.

***Cis*-acting:** Having an influence only on genetic elements and gene loci in vicinity or on the same allele.

**Coding region:** A genetic element that has an open reading frame that can be expressed into a polypeptide.

**Complementary determining regions (CDRs):** The regions in the three dimensional structure of a variable antigen receptor domain that directly establish contact to antigens. The CDRs are usually the most diverse parts of antigen receptors.

**Domain:** A structural moiety of a biomolecule that is characterized by a particular three dimensional structure (e.g. variable or constant region domains of immunoglobulins, that are structurally related, such as Ig-like domains that can be found in many molecules of the immune system, which belong to the so-called Ig-superfamily).

**Effector functions:** Functions of constant regions of immunoglobulins in the context of the immune system, e.g. the ability to activate complement, or to activate certain immune cells by binding to specific receptors for constant region (F_{c} receptors).

**Endogenous:** Being self to a certain cell or organism.

**Enhancer:** A genetic element that can stimulate the activity of promoter elements over large distances, independent of orientation or position.

**Epitope:** A three dimensional structural entity of an antigen that is recognized by a variable binding region of an antibody.

**Functional fragment (of an antibody, antigen receptor or binding protein).** A functional fragment derivable from a given polypeptide selected from the group consisting of antibodies, antigen receptors and binding proteins, that shares at least one of the desired properties inherent to said polypeptide with respect to the specific field of application. For example, a functional fragment may be a polypeptide or oligopeptide maintaining the capability of said polypeptide to bind to certain antigens and/or ligands. The same applies to fragments mediating specific effector functions characteristic for said polypeptide. A further example relates to fragments being able to activate or inhibit specific immunogenic and/or physiologic effector functions of antibodies, antigen receptors and regular receptor-ligand systems.

**Genetic elements:** Genes, gene loci, or fragments thereof, like promoters, enhancers, and coding regions, alone or in any functional or non-functional combination.

**Germinal center:** A distinct histological structure in peripheral lymphoid organs (e.g. lymph nodes or spleen) where cognate interactions between antigen presenting cells and different lymphocyte populations occur, resulting in the proliferative expansion of antigen reactive lymphocytes, as well as affinity maturation and class switch recombination of antibodies produced by antigen reactive B lymphocytes.

**Germline configuration:** The native configuration of genes and gene loci, as they are inherited from the parents, and as they will be passed on to further generations through the germline. DNA recombination events occuring in somatic cells, like e.g. V(D)J recombination in lymphocytes, lead to the reshuffling or loss of genetic information on certain gene loci and therefore to a change of the genes from the germline configuration.

**Heterologous:** Being different from endogenous.

**Humanized antibody:** An artificial antibody generated by fusing the variable domains of non-human antibodies to human constant region domains.

**Hybridoma:** An immortal cell line that has been generated by the fusion of an immortal myeloma cell line (unable to secrete antibody) and a mortal plasma cell (that may secrete large amounts of antibodies).

**Idiotype:** The binding characteristic (specificity) of an antigen receptor.

**Isotype:** The structural characteristic conferred to by the constant region of an antigen receptor determining their (effector) functions.

**Immunoglobulin (Ig):** Synonymous for antibody.

**Immunoglobulin minilocus:** An artificial genetic construct comprising few (i.e. single digit) V, D and/or J gene segments, capable of undergoing V(D)J recombination, thereby generating a limited, oligoclonal repertoire of variable coding regions.

**Library:** A collection of different genetic elements.

**Monoclonal antibody:** Antibody with one defined specificity determined by the unique structure of the variable antigen binding region of the antibody.

**Myeloma cell:** An immortal cell derived from a cell at plasma cell differentiation stage without the ability to express endogenous immunoglobulin proteins.

**Polyclonal antibodies:** A mixture of antibodies having different structures of the variable antigen binding regions, and therefore, most likely, different binding specificities.

**PreB lymphocyte:** A precursor B lymphocyte is characterized by the expression of lymphoid specific factors involved in V(D)J recombination (e.g. RAG-1, RAG-2), usually has initiated V(D)J recombination on at least one immunoglobulin heavy chain allele, but still carries the light chain gene loci in germline configuration, and therefore does not express complete antibodies.

**Primary lymphoid organs:** Organs, in which lymphocytes develop from hematopoietic stem cells, in mice and humans e.g. the bone marrow, the thymus, and during fetal life, the liver.

**Repertoire:** A collection of different (binding) specificities.

**Somatic hypermutation:** A process in somatic cells resulting in the introduction of point mutations into specific regions of the genome at a high frequency (>10⁻⁴ mutations per basepair per cell division).

**Stromal cells:** Proliferating adherent cells derived from the bone marrow with the capacity to support proliferation of preB cells in the presence of additional preB cell growth factor(s).

***Trans*-acting:** Having an influence on genetic elements and gene loci located on different chromosomes.

**Transfecting:** The process of introducing nucleic acid sequences into eukaryotic cells, usually associated with using chemical and physical methods.

**Transforming:** The process of immortalizing a cell for the establishment of a continuously proliferating cell line.

**Transgene:** An artificial genetic element introduced into the germline of animals. The transgene is therefore inherited from one generation to the other.

**Transducing:** The process of delivering DNA into mammalian cells via the production of recombinant viruses. For this, a packaging cell line, expressing structural proteins for viral particles is transfected with a recombinant viral DNA construct comprising the regulatory elements for packaging of the viral DNA construct into the viral structural proteins. By this, recombinant viruses are produced that can be used to infect mammalian target cells leading to the introduction of the genetic information cloned into the recombinant viral genome.

**V(D)J recombination:** A DNA recombination process during which one of many V(variable), sometimes D (diversity), and J (joining) gene segments are assembled to a coding region for the variable domains of antigen receptors.

**Vector:** An artificially generated nucleic acid construct which can be used to shuttle nucleic acid elements between different organisms and species, and which can further be used to propagate, amplify and maintain genomic information.

**Xenogeneic:** Being derived from a different species (often used synonymous to heterologous).

All references cited throughout the specification are incorporated herewith.

### Detailed description of the invention

According to a first aspect, the present invention provides a method for the generation of vertebrate lymphocytes that can be used for the production of any binding protein or functional fragment(s) thereof with the ability to selectively bind to an antigen or ligand, including any heterologous antibody, any antigen receptor composed of variabte domains and constant regions comprising T cell receptors and membrane bound immunoglobulins, any artificial binding protein displaying either wild-type immune effector functions or modified or artificial effector functions not derivable from germline encoded heterologous immunoglobulins or antigen receptors, and any functional fragment(s) thereof, comprising the steps of:
(a) genetically modifying vertebrate precursor lymphocytes, which
   (i) are derived from primary lymphoid organs, and
   (ii) have the potential to differentiate into mature lymphoid lineage cells, by introducing at least one exogenous genetic element encoding at least one binding protein or functional fragment thereof; and
(b) effecting differentiation of said genetically modified precursor lymphocytes into mature lymphoid lineage cells either *in vitro* or *in vivo,* thereby generating lymphocytes capable of producing said binding protein or functional fragment(s) thereof, under the proviso that the *in vivo* production in humans is excluded.

In order to achieve large scale production of the above binding proteins or functional fragments thereof, it is preferred that the terminally differentiated lymphocytes producing the polypeptides of interest are immortalized, or, alternatively, that the genetic information coding for the heterologous antibodies or binding proteins are isolated and transferred from these cells into different expression systems, where said genetic information can be maintained, amplified and/or used for the production of the respective immunoglobulins or immunoglobulin-like proteins or functional fragments thereof.

The immortalization of said lymphocytes can preferably be achieved by:
(a) fusing the same to immortal myloma cells for the generation of hybridoma cells;
(b) infecting the same with transforming viruses, like e.g. Abelson murine leukemia virus (A-MuLV); or
(c) transfecting the same with an appropriate vector construct ensuring the expression of at least one transforming oncogene, like e.g. v-abl, or the SV40 large T antigen, that upon overexpression leads to the immortalization of stably transfected cells;
thereby generating vertebrate lymphocytes capable of permanently producing said binding protein or functional fragment(s) thereof.

In a preferred embodiment, the vertebrate precursor lymphocytes are able to express at least one component of the lymphoid V(D)J recombination machinery and originate from jawed vertebrates comprising cartilaginous fish, bony fish, amphibians, reptilia, birds, mammals including pigs, sheep, cattle, horses and rodents including mice, rats, rabbits and guinea pigs, with murine precursor (pre) B lymphocytes from mice being preferred.

Primary precursor B lymphocytes in all jawed vertebrate species from human down to the evolutionarily most primitive cartilaginous fish are not only characterized by the expression of a specific set of cell surface markers (like the combination of B220 and c-kit in mice), but also by their potential to assemble the gene segments encoding the variable domains of antibodies for which they express the lymphoid specific components of the V(D)J recombination machinery, like RAG-1, RAG-2, and TdT (terminal deoxynucleotidyl transferase). Although some species, like birds, have different primary lymphoid organs (e.g. the bursa of Fabricius) where these precursor B lymphocytes are generated, and despite the fact that in addition to V(D)J recombination, other species also use different mechanisms for antibody diversification, like somatic hypermutation (e.g. sheep), or gene conversion (e.g. rabbits), precursor B lymphocytes from all vertebrate species are capable of undergoing V(D)J recombination and have the potential to differentiate into more mature B lineage cells, such that antibodies generated from VDJ rearranged immunoglobulin gene loci can be expressed. Therefore, if such vertebrate precursor B lymphocytes are isolated or generated that are incapable of expressing endogenous antibodies, and if genetic elements encoding all or parts of heterologous immunoglobulin gene loci are stably introduced into these lymphocytes, they will gain the potential to produce heterologous antibodies and are thus encompassed by the present invention.

The method according to the invention makes use of vertebrate precursor B (preB) lymphocytes present in primary lymphoid organs. In the case of mice, these cells can be isolated from fetal liver or adult bone marrow, e.g. by preparative cell sorting using conveniently labeled antibodies, binding for instance to the cell surface markers B220 and c-kit on mouse preB cells; alternatively, they can be selectively outgrown from murine fetal liver or bone marrow cell suspensions due to their strong proliferative response to both bone marrow derived feeder (stromal) cells, and growth factors such as interleukin-7 and interleukin-3.

In terms of expression of lymphoid components of the V(D)J recombination machinery, the potential to differentiate to mature lymphoid lineage cells and the genetic mechanism for the diversification of antigen receptor proteins, T lineage lymphocytes are very similar to B lineage lymphocytes. It is therefore to be understood that the methods described herein for the genetic modification of precursor B lymphocytes for the purpose of producing heterologous antibodies or binding proteins can likewise be applied to precursor T lymphocytes. Therefore, the present methods primarily described with respect to the system of murine preB lymphocytes, can also be applied to all vertebrate precursor lymphocytes from all jawed vertebrates as set forth before. It is to be understood, that the use of any other precursor lymphocyte system for carrying out the principles of the present invention solely depends on the feasability to both prevent expression of endogenous antibodies and to introduce heterologous genetic elements into these cells encoding heterologous antibodies or binding proteins. Since the broad applicability of these principles will be appreciated by those skilled in the art, the general concept underlying the present invention is primarily described with respect to the use of murine precursor B (preB) lymphocytes.

In the mouse, the development from hematopoietic stem cells to antibody secreting plasma cells is a highly regulated process and, *in vivo*, depends on the ordered rearrangement of gene segments in the immunoglobulin receptor gene loci and the regulated expression of immunoglobulin proteins from productively rearranged Ig gene loci (Fig. 2). Usually, rearrangements occur first on both alleles of the IgH chain gene loci, with D to J_{H} gene segments rearranging first, followed by V_{H} to DJ_{H} gene rearrangements (Fig. 2). If a productive V_{H}DJ_{H} rearrangements has occured on one allele, µH chain can be expressed on the cell surface as a preB cell receptor pairing with surrogate L chain (encoded by the preB cell specific genes λ₅ and V_{preB}) (Karasuyama et al., Adv. Immunol., 63, 1-41, 1996). The expression of a preB cell receptor allows the differentiation of preB cells to a stage, at which V_{L} to J_{L} rearrangements are initiated, which usually occurs first on the κL chain alleles and later on the λL chain alleles (Melchers et al., *Ann. Rev. Immunol.*, 12, p. 209-225, 1994). If a productive IgL chain rearrangement has occurred, membrane bound IgM and/or IgD antibodies can be expressed by so-called immature B cells. Stimulation of peripheral, IgM/IgD positive, mature B cells with appropriate signals can lead to class switch recombination on the IgH chain alleles that changes the isotype of the expressed antibodies to any of the IgG, IgA, IgE subtypes (in mice: IgG1, IgG2a, IgG2b, IgG3, IgA, IgE, in humans: IgG1, IgG2, IgG3, IgG4, IgA1, IgA2, IgE) (Fig. 1).

As already outlined before, the murine preB cells suitable for carrying out the invention are characterized by the expression of B220 and c-kit surface markers, the expression of at least one of the lymphoid V(D)J recombination genes RAG-1 and RAG-2, and the ability to proliferate for longer periods of time in responsiveness of stromal cells and/or IL7 or IL3 (Rolink et al., *EMBO J*., 10, p. 327-336, 1991; Winkler et al., *Blood,* 85, p. 2045-2051, 1995). If preB cell lines and clones are established from wild-type mice, they usually carry DJ_{H} rearrangements on both IgH chain alleles (Alt et al., *EMBO J*., 3, p. 1209-1219, 1984), or in some cases non-productive V_{H}DJ_{H} rearrangements (Fig. 2). However, rearrangements of their IgH chain alleles are not required to establish stromal cell, IL7 or IL3 responsive cells from mice with a preB cell phenotype, as these cells can also be established from mutant mouse strains, that are deficient in V(D)J recombination (Grawunder et al., *International Immunology,* 7, p. 1915-25, 1995). In any circumstance, irrespective of the potential for Ig gene rearrangements preB cells retain the potential to differentiate into more mature B lineage cells *in vitro,* and eventually into isotype-switched plasma cells (Rolink et al., *Immunity*, 5, p. 319-330, 1996).

Long term proliferating murine preB cell lines and clones can be established from various lymphoid organs of mice, including fetal liver, fetal blood, fetal spleen, and adult bone marrow, but also other fetal or adult organs that harbour stromal cell, IL7 or IL3 responsive preB cells, especially early in life (< 4 weeks of age) (Rolink et al., *Blood,* 81, p. 2290-2300, 1993).

Mice used for the generation of the above mentioned preB cells can be wild-type mice, chimaeric mice, transplanted mice or mouse strains carrying mutations impeding V(D)J recombination on the endogenous murine Ig gene loci. The above mentioned mutations may be in *cis*, including deletions of, or within the IgH diversity (D) gene segments, the IgH J gene segments (Chen et al., *Int. Immunol*., 5, p. 647-656, 1993), the µH chain constant region, including the two exons for the µH transmenbrane anchor (Kitamura and Rajewsky, *Nature,* 356, p. 154-156, 1992), the Igκ and Igλ J gene segments, the κL chain constant region (C_{κ}) (Chen et al., *EMBO J*., 12, p. 821-830, 1993), the λL chain constant regions (C_{λ}1-4) and the various IgH and L chain enhancers, including the heavy chain intron enhancer (EµH), the κ intron enhancer (κiE), the 3' κ enhancer (3'κE), and the two λ enhancers (Eλ2-4 and Eλ3-1).

According to the invention, preB cells carrying any of the above mentioned cis-acting mutations can be used for the introduction of exogenous genetic elements comprising part or all of heterologous, especially human immunoglobulin gene loci in non-rearranged, i.e. germline configuration. Such genetically modified preB cells can be used for the *de novo* production of antibody specificities, because the novel specificities are only generated upon V(D)J recombination on the heterologous genetic immunoglobulin gene loci.

Alternatively, suitable preB cells can also be generated from mice incapable of rearranging endogenous immunoglobulin gene loci due to trans-acting mutations, like e.g. in the lymphoid specific recombination activating genes RAG-1 and RAG-2 (Mombaerts et al., *Cell,* 68, p. 869-877, 1992; Shinkai et al., *Cell*, 68, p. 855-867, 1992), or in the ubiquitously expressed DNA repair factors Ku70, Ku86, XRCC4, DNA ligase IV, Artemis or the catalytic subunit of the DNA dependent protein kinase (DNA-PKcs), which are involved in the non-homologous DNA end joining, and that are also required for V(D)J recombination. For the production of heterologous antibodies and binding proteins in preB cells carrying such trans-acting mutations, expression constructs are to be used that do not require the DNA rearrangements of V, (D) and J gene segments (see below).

In the case of *cis*-acting mutations in the mouse, these preferably need to minimally include (a) homozygous mutation(s) in the immunoglobulin heavy and the immunoglobulin κL chain gene loci. In the case of trans-acting mutations, one of the above mentioned homozygous mutations in a given preB cell line is sufficient to preclude the expression of endogenous immunoglobulins being a fundamental requirement of the method according to the invention.

Precursor B lymphocytes carrying *cis-* or *trans*-acting genetic modifications interfering with the expression of endogenous immunoglobulins can be isolated from mice carrying either naturally (e.g. *scid* mice) or artificially generated mutations. Alternatively, preB lymphocytes from wildtype mice can be screened for mutations on their immunoglobulin alleles precluding the expression of endogenous antibodies, e.g. terminally out-of-frame DJ_{H} rearrangements on both heavy chain alleles. Mutations ablating the expression of endogenous murine antibodies, can be introduced into hematopoietic stem cells or into early progenitor cells, from which suitable long-term proliferating preB cells can be generated either upon differentiation *in vitro* or *in vivo*.

Alternatively, the genetic effects referred to hereinbefore can also be directly introduced into at least one allele of the precursor lymphocytes to be subjected to genetic modification. The inactivation of one allele can be sufficiant for carrying out the present invention, since mutated clones can be isolated, which already carry certain mutations on the other allele (heterozygous mutations), such that a homozygously mutated gene locus is established within the precursor lymphocyte cells to be used according to the invention.

Although it is to be understood that the invention can be realized with wild-type precursor lymphocytes, it is preferred in the method according to the invention, that said vertebrate precursor lymphocytes are deprived of their potential to express endogenous antibodies and/or antigen receptors or functional fragment(s) thereof, which is achieved by isolating/selecting vertebrate precursor lymphocytes being deficient in expressing endogenous immunoglobulins or fragments thereof, and/or by introducing into said vertebrate precursor lymphocytes at least one vector construct designed to functionally inactivate at least one allele of at least one genetic element, which is selected from the group consisting of:
(a) the coding regions of the immunoglobulin heavy chain gene locus, including all or parts of the V, D, and J gene segments, and any of the coding regions for the constant region exons for µ, δ, γ, ε, and α heavy chains, with or without their membrane spanning exons;
(b) the coding regions of the immunoglobulin κ and/or λ light chain gene loci, including any of the V and J gene segment coding regions, as well as any of the constant region exons;
(c) the coding regions of the T cell receptor α, β, γ, and δ gene loci, including all or parts of the V, D and J gene segments, and any of the coding regions for the α, β, γ, and δ constant region exons;
(d) the *cis*-acting immunoglobulin heavy chain gene locus enhancer elements, including the heavy chain intron enhancer and the 3' α enhancer;
(e) the *cis*-acting immunoglobulin light chain gene locus enhancer elements, including the κ light chain intron enhancer (κiE), the 3'κ enhancer, and the λ2-4 and λ3-1 enhancers;
(f) the *cis*-acting T cell receptor gene loci enhancer elements, including the TCR α, β, γ, and δ enhancers;
(g) the trans-acting recombination activating genes, RAG-1 and RAG-2, including their promoter and enhancer elements, as well as their coding regions; and
(h) the trans-acting DNA repair genes essential for V(D)J recombination, including Ku70, Ku86, the catalytic subunit of DNA-dependent protein kinase (DNA-PKcs), DNA ligase IV, XRCC4 and Artemis, including their promoter and enhancer elements, as well as the coding regions of said genes.

Preferably, the above vector constructs include gene targeting vectors comprising regions of DNA sequence homology to said at least one genetic element, preferably flanking a positive selection marker enabling selection of positive transfectants. For example, suitable targeting vectors contain two regions of high sequence homology (>99%), or sequence identity to the targeted gene locus, preferably flanking a positive selection marker (such as an antibiotic resistance marker), such that a double crossover event in each of the sequence homology regions results in targeted integration of the positive selection marker and thus in the targeted disruption of an endogenous gene (Fig. 4a-c). Positive selection markers employed in these targeting vectors may include expression cassettes conferring resistance to antibiotics like puromycin, hygromycin B, neomycin (G418), histidinol, mycophenolic acid, zeocin and the like.

It is preferred, that said gene targeting vectors additionally comprise a pair of DNA recognition sequences for site-specific DNA recombination enzymes, flanking the positive selection marker, enabling deletion of said positive selection marker upon transfection and transient expression of nucleic acid sequences encoding at least one of the cognate recombinase enzymes. For example, the positive selection marker may be flanked by loxP or FLP sequences recognized by the cre-recombinase or FLP recombinase enzymes, respectively (Kühn and Schwenk, *Curr*. *Opin. Immunol*., 9, p. 183-188, 1997), allowing the site-specific removal of the selection marker expression cassette from the genome of gene targeted preB cells by transient transfection of cre-or flp-recombinase expression vectors (Fig. 6a, b). This procedure allows the repeated use of targeting vectors using the same positive selection marker.

In a prefered embodiment, said gene targeting plasmid vectors additionally comprise a negative selection marker enabling selection against transfectants in which said gene targeting vectors are randomly integrated into the genome by non-homologous recombination. For example, suitable negative selection markers selecting against random integration of targeting constructs, may include expression cassettes for the herpes simplex virus thymidine kinase gene (HSV-TK), for the diphtheria toxin gene (DT) (McCarrick et al., *Transgenic Res.,* 2, p. 183-190, 1993), or for its attenuated version DTtox176 (Maxwell et al., *Mol. Cell. Biol.*, 7, p. 1576-1579, 1987). These negative selection markers are positioned in the targeting constructs, such that a targeted integration of the positive/negative targeting construct results in the removal of the negative selection marker (Fig. 6a,b). Thus, only integration of the targeting vector by homologous recombination will allow survival of the cells, and - as a consequence - selection for targeting and disruption of part of endogenous gene loci by homologous recombination.

It is to be understood, that the use of vertebrate precursor lymphocytes deprived of their potential to express endogenous antibodies and/or antigen receptors or functional fragment (s) thereof is preferred. However, vertebrate precursor lymphocytes with the potential to express antibodies and/or antigen receptors or functional fragment(s) thereof can likewise be used according to the invention. Regardless of the actual genetic background of the precursor lymphocytes, these cells have to be subjected to genetic modification in order to allow production of heterologous antibodies or binding proteins. It is to be understood, that said modification will preferably be performed after appropriate target cells or clones have been identified by selection and/or generation as described before. However, it is also possible to genetically modify the cells before they are rendered incapable of expressing endogenous immunoglobulins or parts thereof. Furthermore, it may even appear appropriate to simultaneously effect both steps.

Accordingly, it is preferred in a further embodiment that the at least one exogenous genetic element encoding a binding protein or (a) functional fragment(s) thereof, used for effecting the desired genetic modification, is carried on a genetic construct selected from the group consisting of:
(a) recombinant retroviral DNA constructs comprising promoter, enhancer and coding nucleic acid sequences operably linked to ensure expression of at least one binding protein or functional fragment thereof, being either wild-type or having (a) designed mutation(s) in the primary amino acid sequence(s) or being artificial;
(b) recombinant plasmid-based DNA constructs comprising promoter, enhancer and coding nucleic acid sequences operably linked to ensure expression of at least one binding protein or functional fragment thereof, being either wild-type or having (a) designed mutation(s) in the primary amino acid sequence(s) or being artificial;
(c) recombinant plasmid-based mini-immunoglobulin or T cell receptor gene loci with unrearranged V, D and J gene segments operably linked to allow V(D)J recombination and subsequent expression of at least one heterologous antibody or T cell receptor, or functional fragment thereof, being either wild-type or having (a) designed mutation(s) in the primary amino acid sequence(s);
(d) bacterial, yeast or vertebrate artificial chromosomes comprising parts or all of immunoglobulin or T cell receptor gene loci in germline configuration operably linked to allow V(D)J recombination and subsequent expression of at least one heterologous antibody or T cell receptor, or functional fragment thereof, being either wild-type or having (a) designed mutation(s) in the primary amino acid sequence(s);
(e) bacterial, yeast or vertebrate artificial chromosomes comprising parts or all of at least one heterologous immunoglobulin or T cell receptor gene locus in modified arrangement designed to allow V(D)J recombination and subsequent expression of at least one heterologous antibody or T cell receptor, or functional fragment thereof, being either wild-type or having (a) designed mutation(s) in the primary amino acid sequence(s);
(f) trans-chromosome elements which are fragments of heterologous chromosomes harbouring parts or all of immunoglobulin or T cell receptor gene loci in germline configuration allowing V(D)J recombination and subsequent expression of at least one heterologous antibody or T cell receptor, being wild-type with respect to the primary amino acid sequence(s);
wherein the at least one exogenous genetic element most preferably encodes a native or modified human antibody, a human binding protein, a human antigen receptor, or (a) functional fragment(s) thereof.

Furthermore, it is preferred that the at least one exogenous genetic element encodes a heterologous or artificial receptor capable of undergoing affinity maturation of the binding region.

For carrying out the step of genetic modification, several different procedures can be employed according to the invention which are examplified in the following.

A first approach is based on the stable introduction of recombinant plasmid DNA constructs encoding parts of heterologous antigen receptor gene loci in non-rearranged, germline configuration, similar to what has been achieved earlier in the context of transgenic mice. These heterologous antigen receptor gene loci may include V, D and J gene segments of the immunoglobulin H, κL and λL chain gene loci, as well as gene segments from any of T cell receptor (TCR) α, β, γ, or δ gene loci.

A second approach relates to the stable integration of megabase-sized heterologous antigen receptor gene loci, including all or parts of the immunoglobulin and/or TCR gene loci in non-rearranged, germline configuration either as trans-chromosome fragments, or cloned into bacterial artificial chromosomes (BACs), yeast artificial chromosomes (YACs), or vertebrate artificial chromosomes (VACs), which has also been performed in the context of transgenic mice (Green et al., *Nat. Genet*., 7, p. 13-21, 1994; Tomizuka et al., *Proc. Natl. Acad. Sci. U S A*, 97, p. 722-727, 2000).

The genetic elements of both approaches have to be stably integrated into preB cells carrying *cis*-acting mutations in their endogenous murine Ig gene loci, that only interfere with the rearrangement of the endogenous Ig gene loci, but that still allow the generation of diverse antibody repertoires from non-rearranged and/or germline heterologous antigen receptor gene loci. The construction of DNA constructs encoding the human Ig gene loci relies on the knowledge of the organization of all human Ig gene loci and their almost completely published DNA sequence as a result of the human genome project. For example, the human IgH chain gene locus is located on chromosome 14q32.33 within a stretch of 1.25 Mbp. It contains 123-129 (depending on allelic variation) V_{H} gene segments (41-47 of these functional), 27 D, and 6 J_{H} gene segments upstream of the constant region gene cluster. The human IgκL chain gene locus is located on chromosome 2p12, comprises 1.82 Mbp, and contains 40 or 76 (depending on allele) Vκ gene segments (of which 34 or 64, respectively, functional), and 5 Jκ gene segments upstream of a single Cκ region. The human IgλL chain locus is located on chromosome 22q11.2, spans 1.05Mbp, and contains 70 or 71 Vλ (31 or 32 functional) gene segments upstream of 7 to 11 tandemly repeated Jλ-Cλ gene segments.

The DNA constructs carrying part(s) or all of the germline heterologous immunoglobulin or T cell receptor gene loci being present on trans-chromosome fragments, or that may be cloned into BAC, YAC, or VAC vector constructs, can be stably transferred into long-term proliferating target preB cells using standard gene transfer procedures, including e.g. electroporation, calcium phosphate or DEAE-dextran transfection, liposome mediated transfer, sphero- or protoplast fusion, ballistic transfer, microinjection, or specific protein-adduct formation, or a combination thereof. Stable transfectants can be selected by virtue of appropriate (drug) selection markers included in the YAC, BAC and VAC vector backbone, comprising genes conferring resistance to e.g. puromycin, hygromycin B, neomycin (G418), histidinol, mycophenolic acid, zeocin and the like.

A third, conceptionally different approach, can be used for introducing heterologous genetic elements encoding heterologous, preferably human antibodies and binding proteins. According to this alternative method, heterologous genetic elements are stably transferred into murine preB cells, using retroviral transduction, that has been described in the context of gene therapy for inherited diseases using pluripotent hematopoietic stem cells as target cells (cf. An Dong Sung et al., J. Virol. 75(8), 3547-3555 (2001)). Retroviral transfer vectors can only accommodate 7-10 kb of foreign DNA, which significantly facilitates the cloning of expression vectors for heterologous, preferably human antibodies and binding proteins. Therefore, all properties of heterologous antibodies and binding proteins can easily and rapidly be modified by standard molecular biology techniques using retroviral transfer vectors. In contrast, introducing modifications in the context of a transgenic mouse system for heterologous antibody expression is a very tedious and time consuming process, and therefore practically impossible. Using transgenic mice for changing functions of transgene encoded heterologous antibodies would require the generation of several novel transgenic mouse strain, including its breeding onto an immunodeficient genetic background.

Recombinant retroviral transfer vectors encoding heterologous antibodies or binding proteins can either be produced as clones expressing monoclonal binding specificities, or can be generated in the form of libraries encoding polyclonal binding specificities. Because retroviral vectors are not large enough to incorporate significant regions of germline antigen receptor gene loci, the heterologous coding regions need to contain already V(D)J rearranged gene segments encoding variable domains in combination with coding regions for the constant regions of heterologous antibodies and binding proteins. The expression of clones or libraries of retroviral expression vectors for heterologous antibodies or binding proteins therefore does not require a functional cellular machinery for V(D)J recombination, and can therefore be used in conjunction with preB lymphocytes carrying *cis-* and/or trans-acting mutations that ablate endogenous immunoglobulin gene expression, as already outlined hereinabove.

Retroviral expression vectors for conferring expression of heterologous antibodies or binding proteins to murine preB cells are first transfected into retroviral packaging cell lines, of which a wide variety is commercially available having the capacity to produce recombinant retroviruses with either ecotropic, amphotropic, or pantropic spectrum of infectability. Such packaging cell lines are stably transfected with expression constructs for the retroviral *gag, pol* and *env* genes. Transfection of retroviral packaging cells with recombinant retroviral plasmid vectors encoding heterologous antibody or binding proteins will then result in the formation of recombinant retroviruses able to infect preB cells and to stably transduce the heterologous genetic information into preB cell lines and clones (Fig. 8). The retroviral expression vectors used in these methods are constructed from a commercially available empty retroviral transfer vector containing the minimally required retroviral elements, including 5'-LTR a Psi packaging signal and a 3'-LTR (Fig. 11a).

The final retroviral vectors for heterologous antibody or binding protein expression may contain any of the following genetic elements, operably linked in a way to ensure expression of heterologous antibodies and binding proteins in B lineage cells, selected from:
(a) promoter elements, including constitutive promoters, like e.g. CMV (cytomegalovirus), SV40 (simian virus 40), β-actin or PGK (phosphoglycerate kinase) promoters, or B lineage specific promoters, like e.g. the CD19 promoter, or IgH and IgL chain gene promoters;
(b) variable (V) region exons from any antigen receptor gene locus, including immuno-globulin and TCR gene loci;
(c) enhancer elements allowing high level and B lineage specific expression of heterologous antibodies or binding proteins, like e.g. EµH or κiE intron enhancer elements;
(d) constant region exons, including exons encoding the secreted and membrane bound forms of antibodies and binding proteins (Fig. 7a,b); and
(e) any of the 3' immunoglobulin gene enhancers of the heavy and light chain gene loci (i.e. the 3'α, the 3'κ or λ3-1 and λ2-4 enhancers), which are required for somatic hypermutation of the variable region coding exons of the heterologous antibodies or binding proteins (Fig. 7a,b).

Furthermore, expression cassettes for appropriate (drug) selection markers, like e.g. puromycin, hygromycin B, neomycin (G418), histidinol, mycophenolic acid, zeocin, or for suitable reporter genes, like EGFP (enhanced green fluorescent protein) and its mutants YFP (yellow fluorescent protein), CFP (Cyan fluorescent protein), and BFP (blue fluorescent protein), or RFP (red fluorescent protein), may be part of the retroviral vectors allowing selection, or isolation of stably transduced cells, respectively (Fig 7b). The arrangement and the presence of all these coding regions and control elements needs to be carefully designed in order to allow differential expression of membrane or secreted forms of heterologous Ig glycoproteins, as well as antigen driven affinity maturation of the heterologous antibodies and binding proteins in germinal centers *in vivo.* If desired, constant region domain exons can be exchanged between isotypes, or designed mutations can be introduced affecting the effector functions of the expressed heterologous antibodies (Fig. 7a,b). These non-limiting examples are mentioned in oder to illustrate that this system allows the rapid modification of simply any region of the retrovirally encoded heterologous antibodies or binding proteins.

The exon encoding the variable domains within these recombinant retroviral vectors may be monoclonal for the expression of one given specificity, e.g. of an existing antibody whose antigen binding specificity is intended to be modified by affinity maturation after transplantation of stably transduced preB cells into murine hosts and subsequent immunization (Fig. 9). In addition, the exon encoding variable domains may be derived from a library of diverse V(D)J rearrangements. These V region libraries can be isolated from heterologous, preferably human B lymphocytes by PCR using degenerate primer pairs amplifying a multitude of different V_{H} gene families and J_{H} gene segments (Fig. 9). The B lymphocytes may be derived from individuals that either have or have not been immunized (primed versus naïve repertoire), or from patients suffering from autoimmune disease (autoimmune repertoire). In addition, completely synthetic libraries of V region domains can be constructed using the *in vitro* assembly of V domain specific gene fragments that display random nucleotide sequences in regions corresponding to the complementarity determining regions (Fig. 9).

Combinations of retroviral constructs can simultaneously or sequentially be transduced into preB cells allowing the expression and sectretion of complete heterologous immunoglobulins and dimeric binding proteins upon differentiation of the preB cells *in vitro* or *in vivo* (Fig. 8).

Vertebrate preB cells that have been genetically modified by any of the above mentioned methods, such that they have gained the potential to express heterologous antibodies or binding proteins, need to be differentiated into mature B lineage cells and eventually plasma cells, such that the heterologous antibodies or binding proteins can be expressed and eventually secreted. This can either be achieved by differentiation in tissue culture *in vitro,* or upon transplantation of the genetically modified preB cells into appropriate vertebrate hosts *in vivo,* under the proviso that the *in vivo* production in humans is excluded.

Accordingly, it is preferred to effect the differentiation of vertebrate precursor B lymphocytes *in vitro* by:
(a) arresting proliferation of said vertebrate precursor lymphocytes and inducing differentiation into mature lymphocyte lineage cells by cultivating the same in the absence of any precursor lymphocyte growth factor; and
(b) inducing terminal lymphocyte differentiation by further cultivating said cells in the presence of at least one of the following components selected from:
   (i) soluble T cell related stimulating factors, comprising interleukin-2, interleukin-4, interleukin-5, interleukin-6, interleukin-10, interleukin-13, TGF-β, and IFN-γ;
   (ii) factors activating co-stimulatory receptors of B cells, comprising agonistic antibodies or active, recombinant ligands specific forCD40, B7-1 (CD80), B7-2 (CD86), complement receptors 1 (CD35) and 2(CD21), LFA-1 (CD11a), LFA-3 (CD58), CD19, CD20, CD30, CD32, CD37, CD38, CD70, CD71, Igα (CD79α), Igβ (CD79β), TAPA-1(CD81), Fas (CD95), TNF-receptor1 (p55, CD120a), TNF-receptor2(p75, CD120b), Ox-40 (CD134), and lymphotoxin-b receptor; and
   (iii) B cell mitogenic factors, T cell independent antigens of type 1, and other polyclonal activators, including lipoplysaccharide (LPS), lipoproteins from gram negative bacteria, polyanions, poly-dldC, pokeweed mitogen (PWM), and anti-immunoglobulin reagents; and combinations thereof.

The *in vitro* differentiation in tissue culture can be achieved by a two step process, including (a) the removal of preB cell growth factors, like e.g. IL7, or IL3, from the tissue culture medium, which will arrest proliferation of preB cells and at the same time will induce differentiation into cells of a mature B cell phenotype, and including (b) stimulation of the differentiated B cells by said T cell related and/or mitogenic, T cell independent stimuli, such that terminal plasma cell differentiation will be induced. The initial differentiation of preB cells into cells with a B cell phenotype by removing preB cell growth factors is accompanied by the induction of apoptosis. It is thus a preferred embodiment to use preB cells overexpressing anti-apoptotic genes, like e.g. bcl-2 or bcl-x_{L}. These anti-apoptotic genes can be introduced into preB cells either by using preB cells that have been isolated from bcl-2, or bcl-x_{L} transgenic animals, or by transfecting or transducing preB cells with overexpression vectors for any of the anti-apoptotic genes.

As an alternative to the *in vitro* differentiation of genetically modified precursor lymphocytes, these cells can also be differentiated *in vivo* upon transplantation into a suitable vertebrate non-human host, resulting in migration of these cells to secondary lymphoid organs and differentiation into more mature lymphoid lineage cells. Preferably, said lymphocytes are co-transplanted into said host with naïve or antigen primed T helper lymphocytes, wherein the term 'co-transplanted' is to be understood not to be limited to the simultaneous transplantation at exactly the same time. According to another preferred embodiment, the differentiation *in vivo* is followed by immunization of said host with at least one desired immunogenic compound or composition. In contrast to the method described herein, the transplantation of genetically unmodified human CD34+ hematopoietic stem cells into SCID mice (WO 01/87058) does neither allow the generation of high affinity antibodies (because of the lack of compatible human T cells) nor any flexibility in the type and properties of the antibodies produced by these B cells.

Furthermore, it is preferred that said vertebrate host is a compatible host being deficient with respect to the generation of endogenous B cells, T cells, and/or NK (natural killer) cells, or a combination thereof.

As already set forth hereinbefore with respect to the selection of appropriate sources for vertebrate precursor lymphocytes, it is preferred that the vertebrate host is selected from jawed vertebrates comprising cartilaginous fish, bony fish, amphibians, reptilia, birds, and mammals including pigs, sheep, cattle, horses and rodents including mice, rats, rabbits and guinea pigs, with mice being the preferred host species.

The appropriate hosts serving as recipients for the transplantation of genetically modified vertebrate precursor lymphocytes may be wild-type with respect to lymphocyte development, or they may preferably harbour any of various *cis* acting mutations inhibiting endogenous murine Ig gene rearrangements. These may include mutations in enhancer elements of Ig gene loci, like the aforementioned EµH and κiE enhancer elements, deletions within Ig gene coding regions, like the D_{H}, the J_{H} or J_{L} gene segments and the IgC regions including their membrane spanning exons. Furthermore, suitable hosts can be used carrying *trans* acting mutations selectively impeding B (and T) lymphocyte development, e.g. mutations in lineage or lymphocyte specific transcription factors or the RAG-1 and RAG-2 genes required for the initiation of V(D)J recombination (Mombaerts et al., s.a.; Shinkai et al., s.a.).

Furthermore, trans-acting mutations leading to immunodeficient hosts comprise mutations in the ubiquitously expressed DNA repair genes also required for V(D)J recombination like, Ku70, Ku86, the catalytic subunit of the DNA-dependent protein kinase (DNA-PKcs), XRCC4, DNA ligase IV and Artemis. Vertebrate hosts with any of the aforementioned mutations lacking either B lymphocyte populations alone, or both B and T lymphocytes, can then be transplanted with the genetically modified preB cells, and in the case of B- and T cell deficient mice, may be co-transplanted with histocompatible T helper cell populations. The T helper cell populations preferably comprise naive T helper cell, antibody primed effector T cell populations or memory T cells, or any combination thereof.Co-transplantation of T helper cell populations may occur simultaneaously, before or after the actual timepoint of the transplantation of genetically modified preB cells.

Murine or vertebrate hosts whose peripheral lymphocyte populations have been partially or fully reconstituted by the transplantation of modified preB cells and the optional co-transplantation of T helper cell populations can then be used for the immunization with any desired antigen, in order to trigger an immune response to that antigen and to stimulate such lymphocytes expressing the appropriate heterologous receptor specificty, which lead to the proliferative expansion of B cell clones, the affinity maturation of the binding domains of the encoded heterologous antibodies or binding proteins, and the terminal differentiation into plasma cells secreting the heterologous antibodies or binding proteins.

As will be apparent from the foregoing, a further aspect of the present invention is to provide a method for the production of any binding protein or functional fragment(s) thereof with the ability to selectively bind to an antigen or ligand, including any heterologous antibody, any antigen receptor composed of variable domains and constant regions comprising T cell receptors and membrane bound immunoglobulins, any artificial binding protein displaying either wild-type immune effector functions or modified or artificial effector functions not derivable from germline encoded heterologous immunoglobulins or antigen receptors, and any functional fragment(s) thereof, in a manner known *per se*, involving the steps of:
(a) genetically modifying vertebrate precursor lymphocytes, which
   (i) are derived from primary lymphoid organs, and
   (ii) have the potential to differentiate into mature lymphoid lineage cells, by introducing at least one exogenous genetic element encoding at least one binding protein or functional fragment thereof; or, alternatively, using genetically modified vertebrate precursor lymphocytes, which

   (i) are derived from primary lymphoid organs,
   (ii) have the potential to differentiate into mature lymphoid lineage cells, and,
   (iii) carry at least one exogenous genetic element encoding at least one binding protein or functional fragment thereof;
(b) effecting differentiation of said genetically modified precursor lymphocytes into mature lymphoid lineage cells either *in vitro* or *in vivo,* thereby generating genetically modified and differentiated vertebrate lymphocytes capable of producing said binding protein or functional fragment thereof; or, alternatively, using said genetically modified and differentiated vertebrate lymphocytes capable of producing said binding protein or functional fragment thereof;
(c) effecting expression of the binding protein or functional fragment(s) thereof; under the proviso, that the *in vivo* production in humans is excluded.

In this context, it is preferred that any of the above methods involving the generation of the genetically modified and differentiated vertebrate lymphocytes is followed by the steps of:
(a) isolating from said differentiated lymphocytes the at least one exogenous genetic element, and
(b) placing said genetic element(s) in a context enabling production of said at least one binding protein or functional fragment(s) thereof.

In order to achieve large scale production of the desired immunoglobulin or immunoglobulin-like protein or functional fragment thereof, the genetic information which codes for the polypeptides of interest can be isolated from these cells and transferred into different expression systems, where the genetic information can be maintained, amplified and/or used for the production of the desired protein or part thereof.

In more detail, the open reading frames representing the coding regions for the heterologous antibodies or binding proteins can be isolated by standard molecular biology methods, including PCR amplification with specific primer pairs, and transferred into the context of different expression systems, allowing the continuous production of the heterologous antibodies or binding proteins. These expression systems comprise *in vitro* transcription/translation systems, prokaryotic expression systems, like e.g. E. coli, or eukaryotic expression systems, like expression in yeast cells, insect cells using baculovirus infection, or mammalian cells.

The above-mentioned binding protein or functional fragment(s) thereof can either display one unique specificity and can therefore be monoclonal, or can be encoded by more than one unique specificity and can therefore be polyclonal.

According to a preferred embodiment, said binding protein or functional fragment(s) thereof completely or partially shares structural and/or functional features of a human antibody, antigen receptor, or binding protein, as can be assembled on the basis of the human genetic repertoire or parts thereof.

Furthermore, it is preferred that the binding protein or functional fragment(s) thereof to be produced is selected from the group consisting of:
(a) antibodies being either membrane bound or secreted, and consisting of both heterologous heavy and light chain polypeptides in the stochiometric composition found in natural antibodies and consisting of any of the known heavy (ρ, δ, γ, α, ε) and/or light (κ and λ) chain isotypes;
(b) antibodies with combinations of heavy and light chain polypeptides being completely human with respect to the primary amino acid sequence;
(c) hybrid antibodies containing heterologous heavy or light chain polypeptides from different vertebrate species;
(d) secreted Fab, scFv and F(ab')2 antibody fragments being either completely or partially heterologous;
(e) fragments of antibodies covalently coupled via linker peptides, resulting in bispecific or multispecific antibody fragments;
(f) T cell receptors of the α, β, γ, and δ isotype, antigen receptors, and other binding proteins with structural resemblance to proteins of the immunoglobulin superfamily;
and functional fragments thereof.

According to another aspect, the present inventon provides genetically modified vertebrate precursor lymphocytes, which
(a) are derived from primary lymphoid organs and have the potential to differentiate into mature lymphoid lineage cells,
(b) carry at least one exogenous genetic element encoding at least one heterologous binding protein or functional fragment thereof with the ability to selectively bind to an antigen or ligand, including any heterologous antibody, any heterologous antigen receptor composed of variable domains and constant regions comprising T cell receptors and membrane bound immunoglobulins, any heterologous artificial binding protein displaying either wild-type immune effector functions or modified or artificial effector functions not derivable from germline encoded heterologous immunoglobulins or antigen receptors, and any functional fragment(s) thereof, and
(c) carry at least one selection marker being operably linked to said at least one exogenous genetic element.

According to still another aspect, the present inventon provides mature lymphoid lineage cells, which
(a) carry at least one exogenous genetic element encoding at least one heterologous binding protein or functional fragment thereof with the ability to selectively bind to an antigen or ligand, including any heterologous antibody, any heterologous antigen receptor composed of variable domains and constant regions comprising T cell receptors and membrane bound immunoglobulins, any heterologous artificial binding protein displaying either wild-type immune effector functions or modified or artificial effector functions not derivable from germline encoded heterologous immunoglobulins or antigen receptors, and any functional fragment(s) thereof, and
(b) carry at least one selection marker being operably linked to said at least one exogenous genetic element.

According to a further aspect, the present inventon provides immortalized cells derived from mature lymphoid lineage cells, which
(a) carry at least one exogenous genetic element encoding at least one heterologous binding protein or functional fragment thereof with the ability to selectively bind to an antigen or ligand, including any heterologous antibody, any heterologous antigen receptor composed of variable domains and constant regions comprising T cell receptors and membrane bound immunoglobulins, any heterologous artificial binding protein displaying either wild-type immune effector functions or modified or artificial effector functions not derivable from germline encoded heterologous immunoglobulins or antigen receptors, and any functional fragment(s) thereof,
(b) carry at least one selection marker being operably linked to said at least one exogenous genetic element, and
(c) produce said at least one heterologous binding protein or functional fragment thereof.

As will be appreciated, fully human or humanized antibodies or functional fragment(s) thereof can be used in the diagnosis, prevention and therapy of human disease by virtue of their ability to bind with their variable region domains to specific antigens, and, furthermore, by their ability to mediate immune effector functions via their constant region domains. Some possible uses of human monoclonal antibodies, which can be produced according to the invention, for the diagnosis, prevention and therapy of human disease are detailed below. For diagnostic purposes, human monoclonal antibodies can be used for the identification and visualization of certain pathologic conditions by the introduction of labeled antibodies into the vascular system of individuals, where specific disease related antigenic structures (e.g. expressed on certain pathologic cells) can be detected. Applications in the prevention of human disease include antibodies with the ability to block interactions of certain cell surface receptor-ligand systems in pathologic conditions (antagonistic antibodies), or conversely, to mimick the effect of ligand binding to cell surface receptors (agonistic antibodies) in case of the pathologic absence of a ligand. Furthermore, human monoclonal antibodies can be used for blocking the functions of toxic substances, or of pathogens, e.g. during poisoning, inflammation, and infections. In this context, human antibodies are of particular use, because they mediate the tagging of these substances and antigens for the removal by specialized cells of the innate immune system. Human monoclonal antibodies can also be used for targeting immune functions to cancerous cells by virtue of specific binding to altered self structures expressed on the surface of malignant cells. In case of autoimmune conditions, human monoclonal antibodies may further be useful for neutralizing or counteracting the pathologic effects of autoimmune factors (autoantibodies, allergic antibodies). Human monoclonal antibodies are so useful for the diagnosis, prevention and therapy of human disease, because they can be introduced into the vascular system of individuals, without causing any adverse effects, as antibodies are normal constituents of blood plasma and fluid components of humans (e.g. mucus, saliva, lymph fuid etc.).

### Deposition of biological material

Plasmids carrying genetic elements used in accordance to the present invention have been deposited under the Budapest Treaty with the Deutsche Sammlung von Mikroorganismen und Zellkulturen (DSMZ) in Braunschweig, Germany, on December 17, 2001, under the following Accession Nos.:

| Plasmid | Accession No. |
|---|---|
| pBS-DT4 | DSM 14703 |
| pPGK-hygro | DSM 14704 |
| pGL2neo(m)+ | DSM 14705 |
| pBS-DT4-tox176 | DSM 14706 |

The following experimental procedures describe detailed methods for the generation of human monoclonal antibodies using murine preB cells that are genetically modified in order to confer the potential for human antibody production. Selected examples of methods are descibed as follows allowing the genetic modifications of murine preB cells, such that endogenous antibodies can no longer be expressed, but that human antibodies can be produced from stably delivered heterologous retroviral expression constructs. Furthermore, experimental details are presented relating to the transplantation of these genetically modified murine preB cells into appropriate murine hosts, as well as methods relating to the immunization of these transplanted mice, the isolation of antibody secreting cells, and their establishment as immortal monoclonal human antibody secreting hybridoma cell lines. Although the provided methods are complete and sufficient for the production of heterologous, especially human monoclonal antibodies using murine preB cells, that are genetically modified, the methods should not be viewed by way of limitation, but rather by way of illustration.

### Examples

### Example 1: Establishment of long-term proliferating, murine precursor B cells with the potential to differentiate into mature B lineage cells

Stromal cell and IL7 responsive murine precursor B cells are found in fetal liver of mouse fetuses at day 15-18 *post-coitum,* in the bone marrow of adult mice, as well as in the spleen of newborn animals (< 3 weeks of age) (Rolink et al., Blood, 81, 2290-2300, 1993). In order to isolate these preB cells, mice are sacrificed by CO₂ suffocation and the respective organs are removed under sterile conditions. Total bone marrow cells are obtained by removing femur and tibia, opening of the bones at both ends with sterile scissors and flushing the marrow with 2-5 ml phosphate buffered saline (PBS) using a 5 ml disposable syringe and a 25 or 26 gauge needle. Isolated spleen or fetal liver also prepared under sterile conditions is placed onto a sterile 200 mesh steel grid in a petri dish with 5-10 ml of PBS. The organs are cut into pieces and gently squashed through the metal grid with the plunger of a 5 ml plastic syringe. Cell suspensions are washed once by centrifugation (200 g, 10 min, 4°C) and resuspended in 10-20 ml PBS. All cell preparations are carried out with ice-cold solutions, and cell suspensions are kept on ice until transfer into tissue culture.

Establishment of individual preB cell clones is performed by limiting dilution in 96-well plates coated with semi-confluent, 3000rad γ-irradiated ST-2 (Ogawa et al., EMBO J., 7, 1337-1343, 1988) or PA-6 (Kodama et al., J. Cell. Physiol., 118, 233-240, 1984) murine stromal cells grown in special stromal cell medium (see below). For limiting dilution plating, total organ cell populations can be used or, alternatively, B220⁺ or CD19⁺ surface marker positive B lineage cells enriched by fluorescent or magnetic bead activated cell sorting (FACS or MACS) using commercial antibodies specific for these markers.

The long-term culture of murine preB cells is performed in a special serum-free medium, containing 100 units of recombinant interleukin-7 (IL-7) (see below). Individual preB cell colonies develop on stromnal cell feeders under limiting dilution conditions within 6-7 days of culture at 37°C, in a humidified incubator under a 10% CO₂ atmosphere. Single preB cell colonies are initially transferred into 24-well plates, and then sequentially expanded into 25cm² and eventually 75cm² tissue culture flasks, always pre-coated with 3000rad γ-irradiated stromal cells. After the initial expansion, preB cell densities need to be kept between 1x10⁵ and 2x10⁶ preB cells/ml tissue culture medium, which requires subculturing of the cells every two to three days.

Special serum-free tissue culture medium (Iscove and Melchers, J. Exp. Med., 147, 923-933, 1978) required for the establishment of long-term proliferating, stromal cell, IL-7 dependent murine preB cells (formulation for 1 liter of medium):
- 11 g DMEM-powder (without bicarbonate)
- 10 ml 1 M HEPES, pH 7.3
- 33.3 ml 7.5% NaHCO₃ solution
- 8 ml amino acid stock solution containing:
   - L-alanine 600 mg
   - L-asparagine 520 mg
   - L-aspartate 720 mg
   - L-glutamate 1800 mg
   - L-proline 960 mg
   - Na-pyruvate 2640 mg
      + 1.6 ml BiotinNitamin B12 stock solution containing
   - Vitamin B12 5.0 mg
   - D-biotin 5.0 mg
      dissolved in 20ml + 10 µl 1 M HCl
   all components dissolved in 240 ml ultrapure H₂O
- 4 ml cysteine stock solution containing
   - L-cysteine 1.4 g
      dissolved in 150 ml H₂O, 50 ml 1M HCl
- 5 ml 10% BSA (Bovine serum albumine) solution
- 0.15 ml human transferrin stock solution composed of:
   - 10 ml of solution a.) and 80 µl solution b.)
      a.) 1g human transferrin dissolved in 10 ml DMEM
         (1.3g/100ml H₂O) + 100µl 1 M HEPES, pH 7.3
      b.) 440mg FeCl₃x6 H₂O + 185 ml H₂O + 200 µl 1M

### HCI

- 5 ml soybean-lipid stock solution
   - 200 mg soybean lipids mixed with 45 ml DMEM
      (1.3g/100ml H₂O) + 5 ml 10% BSA. Sonication 3x 15 min in ice-water.
- 20 ml kanamycin (5000 µg/ml)
- 30 µl 2-mercaptoethanol (1.43 M)
- 10⁵ U recombinant mouse interleukin-7 (IL7)

All components are dissolved in a final volume of 1000ml triple-distilled, ultrapure H₂O, filtered sterile and kept at 4°C until use.

ST-2 or PA-6 stromal cell feeder cells need to be grown in a special, low serum containing medium as follows (formulation for 1 liter):
- 17.7 g IMDM-powder (without bicarbonate)
- 3 g NaHCO₃
- 10 ml 100x non-essential amino acids (GIBCO-BRL)
- 10 ml 100x Penicillin/Streptomycin (GIBCO-BRL)
- 1 ml 5 mg/ml porcine insulin-solution
- 1 ml 50 mM 2-mercaptoethanol
- 3 ml 10% primatone (ultrafiltrated to exclude proteins >10 kD) (source: Quest International, Naarden , NL)
- 20 ml fetal calf serum

All components are dissolved in a final volume of 1000ml triple-distilled, ultrapure H₂O, filtered sterile and kept at 4°C until use.

### Example 2: Stepwise differentiation of murine precursor B cells into antibody secreting plasma cells in vitro

Long-term proliferating, stromal cell and IL7 dependent murine preB cells retain their potential to differentiate into more mature B lineage cells (Rolink et al., EMBO J., 10, 327-336, 1991), which can be achieved *in vitro.* This differentiation can be performed in two steps by first inducing differentiation to cells with an immature B cell phenotype and, second, by inducing differentiation to cells with a phenotype of antibody secreting plasma cells.

The first differentiation stage is reached by removing IL7 from the preB cells in the continued presence of stromal cells. For this preB cells are harvested from proliferating cultures, washed three times with serum free preB cell culture medium lacking IL7 and plated at a density of 1-2x10⁶ cells/ml onto fresh, 3000rad γ-irradiated stromal cells in the absence of IL7. Within a period of three days the preB cell cease to proliferate and differentiate into cells with a phenotype of immature B cells. The phenotypic changes include e.g. the loss of c-kit, λ₅ and V_{preB} expression and the gain of CD25 and CD40 expression. In preB cells from wild-type mice, this differentiation is further accompanied by sequential V_{H} to DJ_{H} rearrangements on the IgH chain gene loci followed by V_{L} to J_{L} rearrangements on the IgL chain gene loci, which may result in the expression of surface bound IgM antibody on the differentiated B lineage cells (Rolink et al., EMBO J., 10, 327-336, 1991). However, it has to be noted that the ordered rearrangement of IgH and L gene segments is not a prerequisite for the phenotypic differentiation of these preB cells *in vitro* (Grawunder et al., Int. Immunol., 7, 1915-1925, 1995).

The second differentiation stage can be achieved by stimulating the IL7 deprived cells with either an agonistic anti-CD40 antibody or LPS in combination with cytokines, like e.g. IL4, IL5, IL10 or TGF-β. This treatment will induce proliferation, class switch recombination to various Ig gene isotypes (depending on the cytokine combination employed) and eventually differentiation to antibody secreting plasma cells. In case of e.g. anti-CD40/IL4 stimulation, cells are washed three times in preB cell tissue culture medium, plated onto fresh, subconfluent 3000rad γ-irradiated stromal cells in the presence of 100U/ml IL4 and 5µg/ml anti-CD40 monoclonal antibody for 4-6 days. Large, proliferating plasma cell stage cells can be identified and separated by fluorescent activated cell sorting (FACS), based on their large size which results in distinctly increased forward scatter values.

The *in vitro* differentiation of wild-type preB cells is usually accompanied by loss of substantial amounts of cells due to growth factor withdrawal induced apotosis. This problem can be circumvented by overexpression of anti-apoptotic genes, like bcl-2 or bcl-x_{L} in these cells (Rolink et at., J. Exp. Med., 178, 1263-1270, 1993). This can be achieved by isolating preB cells from mice containing a B lineage specifically expressed bcl-2 transgene (Strasser et al., Curr. Top. Microbiol. Immunol., 166, 175-181, 1990), or by transducing a bcl-2 encoding recombinant retroviral vector into the long-term proliferating, stromal cell and IL7 dependent murine preB cells (see below).

### Example 3: Cloning of a retroviral expression construct for murine Bcl2 overexpression

A prerequisite for the stable transduction of genes into long-term proliferating, stromal cell and IL7 dependent murine preB cells is the construction of a recombinant retroviral transfer vector containing an expression cassette for a gene of interest. Retroviral transfer vectors consist of retroviral 5' and 3' long terminal repeat (LTR) sequences flanking a retroviral packaging signal, Psi(+), a multiple cloning site (MCS) into which heterologous sequences can be inserted, and bacterial plasmid control elements required for replication and maintenance of the vector in *E.coli*. An example for an empty retroviral transfer vector is the commercially available plasmid pLIB (Clontech) (Fig. 11 a).

In order to construct a retroviral expression vector containing the anti-apoptotic gene bcl-2, a strategy for coexpression of the murine bcl-2 cDNA and the hygromycinB drug selection marker is applied by linking expression of bcl-2 and hygromycinB using an internal ribosomal entry sequence (IRES), allowing simultaneous expression of two construct, which can be selected with the antibiotic drug hygromycin B will simultaneously select cells for the expression the anti-apoptotic gene bcl-2. The generation of the retroviral vector for coupled expression of the bcl-2 and hygromycinB cDNAs proceeds in two stages, first the assembly of the promoter-bcl-2-IRES-hygromycinB expression cassette and, second, the cloning of this cassette into the MCS of the retroviral transfer vector pLIB (Fig. 11 a+b).

### (a) Construction of the promoter-bcl-2-IRES-hygromycinB expression cassette

Both the murine bcl-2 cDNA and the open reading frame (ORF) for the hygomycinB drug resistance marker are cloned into the commercially available CMV-promoter/IRES vector pIRES (Clontech). For this, the hygromycinB ORF is amplified from plasmid pPGK-hygro (SEQ ID NO 1) by polymerase chain reaction (PCR) using primer pair SEQ ID NO. 5 (P001) 5'-cgTCTAGAccatgaaaaagcctgaactcaccgcgacgtctg-3', and SEQ ID NO. 6 (P002) 5'-catGCGGCCGCtattcctttgccctcggacgagtgctgggg-3' containing additional restriction enzyme recognition sites (indicated in uppercase letters) for restriction endonucleases Xbal and Notl (2-4 random nucleotides 5' of the restriction enzyme recognition sequence are also added, because most restriction enzymes do not efficiently digest DNA, if the recognition sequence is located directly at the end of PCR amplified DNA fragments. These flanking nucleotides are indicated in lowercase letters, in order to highlight the restriction enzyme recognition sites. The sequences downstream, or 3' of the highlighted restriction sites correspond to DNA sequences of the fragment to be amplified. This way of presentation of primer sequences is maintained throughout the description of experimental examples. A PCR with primers P001 and P002 will amplify a DNA fragment of 1017 basepairs (bp) containing unique recognition sites for restriction endonucleases Xbal and Notl at the ends of the fragment. The PCR fragment is then double digested with Xbal and Notl restriction enzymes and is then directionally cloned into XbaI/NotI double digested pIRES plasmid, in order to generate the plasmid construct pIRES-hygroB (Fig.11a). The ORF of the murine bcl-2α cDNA is isolated and amplified from mouse spleen mRNA by reverse transcriptase coupled polymerase chain reaction (RT-PCR) using the following primers designed based on the published NCBI-Genbank sequences M16506 and L31532 (primer P003 binding to position 1821-53 of M16506, and primer P004 binding to position 506-535 of L31532): 
SEQ ID NO. 7 (P003) 5'-attGCTAGCatggcgcaagccggagaacagggtatgataac-3'
and
SEQ ID NO. 8 (P004) 5'-cgcACGCGTcacttgtggcccaggtatgcacccagagtg-3
 containing restriction enzyme recognition sites (in uppercase letters) for Nhel and Mlul. A NheI/NotI double digested PCR product of 699 bp size is then directionally cloned into Nhel/Mlul double digested vector pIRES-hygroB in order to generate pBcl2-IRES-hygroB.

### (b) Construction of a retroviral Bcl-2 expressing transfer vector

Due to the lack of compatible restriction enzyme sites in the multiple cloning site of the empty retroviral transfer vector pLIB and the dual bcl-2/hygromycinB expression vector pBcl2-IRES-hygroB, the entire expression cassette for Bcl-2-IRES-hygromicinB including the constitutive CMV promoter and polyadenylation site is cloned into pLIB using PCR with appropriated restriction enzyme recognition sites. For this the CMV-bcl2-IRES-hygromycinB expression cassette is PCR amplified with pBcl2-IRES-hygroB as the PCR template using primers: 
SEQ ID NO. 9 (P005) 5'-atatGTCGACtcaatattggccattagccatattattcattg-3'
and
SEQ ID NO. 10 (P006) 5'-ccggATCGATccttatcggattttaccac-3'
 containing restriction enzyme recognition sites for Sall and Clal (in uppercase letters). A Sall/Clal double digested PCR product of 3714 bp is then directionally cloned into the Sal/Clal double digested pLIB vector thereby generating retroviral transfer vector pLIB-bcl2-IRES-hygroB allowing coupled bcl-2 and hygromycinB expression.

### Example 4: Retroviral transduction of long-term proliferating, stromal cell and IL7 dependent murine preB cells

One of the ways to stably transfer genetic elements into murine preB cells is to use retroviral transduction (Fig. 8). For this procedure a recombinant DNA construct containing retroviral sequences necessary for packaging of a retroviral genome (5'LTR, packaging signal Psi(+), 3'LTR) as well as heterologous sequences or genes of interest, are transiently or stably transfected into a packaging cell line constitutively expressing genes required for the production of retroviral particles.

For the transduction of stromal cell and IL7 dependent murine preB cells, the ecotropic retroviral packaging cell line GPE (Markowitz et al., *J. Virol*., 62, p. 1120-1124, 1988) can be used, which routinely results in stable transduction of preB cells at efficiencies of 50-80%. This ecotropic packaging cell line in conjunction with (recombinant) retroviral transfer vectors will only produce replication deficient (recombinant) retroviruses able to transduce murine cells. For transient transfection of the ecotropic GPE packaging cell line, standard calcium phosphate transfection is used as detailed below:

### (a) Transient transfection of the ecotropic retroviral packaging cell line GPE:

GPE cells are grown in IMDM-based low-serum medium also used for ST-2 and PA-6 stromal cells and described in example 1. One day prior to transfection, adherent GPE packaging cells are harvested by trypsinization and seeded at 50% confluency into fresh IMDM-based low-serum medium and cultivated at 37°C in 10% CO₂. The following day, 20µg retroviral DNA is transfected per T75 (75cm²) tissue culture flasks of GPE cells using standard calcium phosphate precipitation. 24 hours post transfection, the transfected GPE cells can be used for the transduction of recombinant retroviruses into preB cells.

### (b) Retroviral transduction of preB cells using transfected GPE cell culture supernatant or coculture with transfected GPE cells:

Two methods can be used interchangably for the transduction of recombinant retroviruses produced by transfected GPE cells: The first method relies on the infection of preB cells with conditioned cell culture supernatant. For this, cell culture medium from transfected GPE cells is replaced 24 hours after transfection, and is harvested after another 24 hours of cell culture. The recombinant retrovirus containing cell culture supernatant is then added to cultures of proliferating preB cells at ratios of 1:8 (vol) to 1:1 (vol) in the presence of 8µg/ml polybrene and incubation is continued for 3-6 hours at 37°C. Cells are then harvested, the polybrene/retrovirus containing medium is removed , and cells are plated at a density of 1-2x10⁵ onto fresh, 3000rad γ-irradiated stromal cells in preB cell medium containing 100U rlL7. Retrovirally transduced preB cells are then harvested 24 hours later.

The second method relies on the coculture of preB cells with transfected, recombinant retrovirus producing GPE cells. For this, log-phase preB cells are harvested and resuspended in fresh preB cell culture medium containing 100U rIL7 and 8 µg/ml polybrene at a density of 2-4x10⁵ cells and are plated onto transfected GPE cells 24 hours post transfection with retroviral constructs. PreB cells are co-cultured for 6 hours at 37°C, they are then harvested and plated onto fresh, 3000rad γ-irradiated stromal cells in preB cell medium containing 100U rIL7 for recovery. Retrovirally transduced preB cells are then harvested and can be utilized 24 hours later.

### Example 5: Inactivation of endogenous immunoglobulin heavy and light chain gene loci in long-term proliferating, murine precursor B cells

The first step for the generation of heterologous, preferably human antibodies from murine precursor B cells, is the inactivation of the endogenous, murine IgH and IgL, which can, among other methods, e.g. be accomplished by gene targeting within the immunoglobulin gene locus in preB cells. In theory, it would be required to inactivate all three murine immunoglobulin gene loci, i.e. the IgH, IgκL and IgλL chain gene loci. However, the murine λL chain gene locus comprises only three functional V-J-C clusters with one gene segment each and only very limited diversity is comprised in λL chains. Furthermore, less than 5% of murine B cells express λL chain gene loci. Therefore, in practical terms, it is sufficient to inactivate the endogenous, murine IgH and KL chain gene loci in order to assure that upon transfer of heterologous IgH and IgkL chain genes or gene loci, the vast majority of B cells (>95%) produced from preB cells will express heterologous antibodies.

### (a) Isolation of preB cells with naturally occuring irreversibly non-functional IgH alleles.

Generally, the most controlled way for inactivation of the endogenous IgH and κL chain gene loci is to use gene targeting (see below). However, in case of the IgH chain gene locus, another method can be applied:

Long-term proliferating, stromal cell IL7 dependent murine preB cells carry DJ_{H} rearrangements on both of their havy chain alleles. Due to imprecision in the D to J_{H} joining process, these DJ_{H} rearrangements may occur such that D gene segments can be read in all three possible reading frames relative to the fixed reading frame of the J_{H} gene segment. These reading frames have arbitrarily been designated reading frames I, II and III (Kaartinen and Mäkelä, *Immunol. Today,* 6, p. 324-330, 1985). Most D gene segments contain stop codons, if a DJ_{H} junction occurs in reading frame III (Kaartinen and Mäkelä, s.a.), such that a functional H chain can never be expressed from such a heavy chain allele.

Therefore, statistically roughly 1/9 (11.1%) of all stromal cell, IL7 dependent preB cells carry DJ_{H} rearrangements in reading frame III with stop coding on both of their IgH chain alleles. These preB cells can be isolated by subcloning under limiting dilution conditions and screening for the desired non-functional rearrangements. To ensure the isolation of stable clones, rearrangements need to be screened for two non-functional DJ_{H}4 gene rearrangements, which cannot be reverted by secondary rearrangements because the last J_{H} gene segment has been used up. Such clones can be used for further targeted inactivation of the κL chain alleles.

### (b) Generation of targeting constructs for the murine IgH and IgκL chain loci

Two examples for methods resulting in gene targeted inactivation of both the endogenous IgH and IgκL chain gene loci are presented as examples for many *cis*-acting mutations that would similarily lead to preB cells without the potential to express endogenous IgH and IgκL chain proteins (Fig. 5a,b). For both targeting strategies, first, empty positive-negative gene targeting vectors need to be constructed containing unique cloning sites allowing the insertion of DNA sequences homologous to the target gene locus, a positive drug selection marker, like neomycin or puromycin, flanked by loxP sites and a negative selection marker, like diphtheria toxin, or herpes simplex virus thymidine kinase (HSV-tk), which will select against random integration of the gene targeting vector into the genome of cells. The presence of a loxP flanked (floxed) positive selection marker will allow the sequential gene targeting of both alleles in the same cell clone using the same antibiotic, because , after successful targeting of the first allele, the antibiotic resistance marker can be deleted from cells upon transient expression of cre recombinase deleting any nucleotide sequence located in between the cognate loxP recognition sites. The empty positive-negative targeting vectors are constructed as follows (Fig. 6a,b):

### Step 1: Extending the multiple cloning site of pBluescript SK(+)

Two complementary synthetic DNA oligomers: 
SEQ ID NO. 11 (P007)
5'-TCGAccggtacctaggcgcgccatcgatatcgctagctcgagctcagatctGTAC-3' and
SEQ ID NO. 12 (P008)
5'-agatctgagctcgagctagcgatatcgatggcgcgcctaggtaccgg-3'
 are annealed by mixing 100pmol of each oligomer in 50µl of 50mM Tris/HCl, 100mM NaCI, pH8.0. The mixture is then denatured for 2 minutes at 95°C and slowly cooled from 65°C to room temperature within 20min. This will generate the following double stranded DNA linker with Xhol and Kpnl compatible overhangs (indicated in uppercase letters):

5'-TCGAccggtacctaggcgcgccatcgatatcgctagctcgagctcagatctGTAC-3'
| | | | | | | | | | | | | | | | | | | | | | | | | | | | | | | | | | | | | | | | | | | | | | |
3'-ggccatggatccgcgcggtagctatagcgatcgagctcgagtctaga-5'

The annealed synthetic DNA linker is then ligated into Xhol-Kpnl linearized commercially available pBluescriptSK(+) (Stratagene) resulting in plasmid pBSL containing an extended multiple cloning site (MCS+, see Fig. 6a) required for the following cloning steps.

### Step 2: Insertion of expression cassettes for wild-type or attenuated diphtheria toxin α as a negative selection marker against random integration of targeting constructs

As a negative selection marker that allows selection against random chromosomal integration of gene targeting constructs, an expression cassette for diphtheria toxin α (McCarrick et al., s.a.), or its attenuated version, diphtheria toxin α (DT-αtox176) (Maxwell et al., s.a.), under control of a constitutive β-actin promoter can be used .Although other negative selection markers, like the herpes simlex virus thymidine kinase gene (HSV-tk), is commonly used in gene targeting experiments with mouse embryonic stem cells, diphtheria toxin α and its attenuated mutant tox176 have been proven to be effective for gene targeting in somatic cells (Grawunder et al., *Mol. Cell,* 2, p. 477-484, 1998). The diphtheria toxin expression cassettes are isolated as 2.1kb Bglll-Xbal fragments from plasmids pBS-DT4 (SEQ ID NO. 2), or pBS-DT4tox176 (SEQ ID NO. 3; see Fig. 6a) and are ligated into Bglll-Nhel linearized pBSL resulting in plasmids pBSL-DT4 and pBSLDT4tox176, respectively (see Fig. 6a).

### Step 3: Insertion of IoxP flanked positive drug selection markers into pBSL-DT4 and pBSLDT4tox176 for the generation of empty gene targeting vectors

The next step for the generation of positive/negative gene targeting vectors is the insertion of a positive drug selection marker into the pBSL-DT4 or pBSL-DT4-tox176 vector, upstream of the diphtheria expression cassette. As representative examples the insertion of loxP site flanked neomycin, puromycin and hygromycin B expression cassettes upstream of the diphtheria toxin α expression cassette in pBSL-DT4 are presented. However, it should be noted that identical cloning strategies can be applied to the pBSL-DT4tox176 vector, containing the attenuated version of diphtheria toxin α. Furthermore, other positive drug selection markers conferring resistance e.g. to histidinol, mycophenolic acid, bleomycin, or zeocin may be used.

In each case, the positive antibiotic selection marker cassette will be flanked by loxP sites, that can be recognized by the cre recombinase enzyme, and which can be used to delete the drug selection marker located between the loxP sites upon transient transfection of a cre recombinase expression vector, for sequential use of the same selection marker.

### i) Insertion of a floxed neomycin resistance cassette:

A loxP site flanked neomycin resistance cassette can be isolated as a 1497bp Bsp120I-Xbal digested DNA fragment from plasmid pGL2neo(m)+ (SEQ ID NO. 4) and can be ligated into Notl-Xbal linearized pBSL-DT4 to generate the empty targeting vector pBSL-flneo-DT4.

### ii) Cloning of floxed puromycin and hygromycinB cassettes (Fig. 6b):

Because floxed antibiotic selection markers other than neomycin are rarely found, an essential step for the insertion of loxP flanked puromycin or hygromycinB markers into pBSL-DT4 is the preparatory step of inserting two loxP sites. This can be achieved by inserting into pBSL-DT4 annealed synthetic DNA oligomers. The synthetic loxP sites are contained in the following two complementary DNA oligos:

SEQ ID NO. 13 (P009)
5'-CTAGATAACTTCGTATAGCATACATTATACGAAGTTATACGCGTATAACTTCGTATAGCATA
CATTATACGAAGTTAT -3' and

SEQ ID NO. 14 (P010)
5'-CTAGATAACTTCGTATAATGTATGCTATACGAAGTTATACGCGTATAACTTCGTA
TAATGTATGCTATACGAAGTTAT -3'

These two DNA oligomers are annealed as described in example 5(b), step 1, resulting in a double stranded DNA fragment containing two loxP sites separated by a unique Mlul restriction site. In addition, the annealed synthetic DNA oligomers contain two 5'-CTAG single-stranded overhangs, which can directly be ligated into compatible restriction site overhangs (generated e.g. by Spel, Nhel, Xbal, or Avrll restriction enzymes).

The annealed double loxP site containing DNA fragment is then ligated into Spel linearized pBSL-DT4 resulting in vector pBSL-DT4-2loxP (Fig. 6b).

Expression cassettes for puromycin or hygromycinB resistance can be PCR amplified using plasmids pPur (Clontech) or pPGK-hygro (SEQ ID NO. 1) as PCR templates, respectively. The primers used for PCR amplification of the 1366 bp puromycin expression cassette from pPur (containing the puromycin ORF under control of the simian virus40 early promoter) are:

SEQ ID NO. 15 (P011) 5'-tcgACGCGTggaatgtgtgtcagttagggtgtggaaag-3'
and
SEQ ID NO. 16 (P012) 5'-tcgACGCGTttggacaaaccacaactagaatgcagtg -3'

Both primers contain recognition sites for the restriction endonuclease Mlul (indicated in uppercase letters), such that the resulting PCR product can be cloned using this restriction enzyme.

After Mlul digestion of the isolated PCR product the puromycin marker fragment is ligated into Mlul linearized pBSL-DT4-2loxP generating the empty targeting vector pBSL-flpur-DT4 (Fig. 6b).

The primers used for PCR amplification of the 2007 bp hygromycin B expression cassette from pPGK-hygro (SEQ ID NO. 1; containing the hygromycinB ORF under control of the phospho glycerat kinase promoter) are:

SEQ ID NO. 17 (P013)     5'-tcgACGCGTgaattctaccgggtaggggaggcgcttttc-3'
and
SEQ ID NO. 18 (P014)     5'-tcgACGCGTggaattagaacttggcaaacaatactgag -3'

Both primers contain recognition sites for the restriction endonuclease MluI (indicated in uppercase letters), such that the resulting PCR product can be digested and ligated using this restriction enzyme.

After Mlul digestion of the isolated PCR product the hygromycinB marker fragment is ligated into Mlul linearized pBSL-DT4-2loxP generating the empty targeting vector pBSL-flhyg-DT4 (Fig. 6b).

### Step 4: Cloning of final targeting vectors for murine IgH and κL chain gene loci, as well as for the RAG1 gene

As representative examples for cloning of targeting vectors for endogenous gene loci, the cloning strategies for gene targeting vectors useful for the *cis*-acting targeted mutation of the endogenous IgH and IgκL chain gene loci, and for the *trans*-acting mutation of the RAG1 gene in murine preB cells are described.

### i) Construction of IgH chain targeting vectors (Fig. 5a)

Inactivation of the endogenous murine IgH chain gene locus in preB cells can be achieved by generating many different *cis*-acting targeted deletions on both IgH chain alleles. One of IgH inactivating deletion is for instance the deletion of all D_{H} and J_{H} gene segments, which is described here as one of the possibilities to render the endogenous IgH alleles incapable of producing endogenous immunoglobulins. Targeting vector construction is presented here using a "floxed" neomycin targeting construct as an example (see Fig. 5a). For the cloning of gene targeting vectors containing other positive antibiotic selection marker, like e.g. puromycin or hygromycinB , similar strategies may be applied.

Stromal cell, IL7 dependent murine preB cells usually carry DJ_{H} rearrangements on both alleles (see above). This fact needs to be taken into account for the design of targeting vectors. Regardless of the type of DJ_{H} rearrangement, the DNA sequences intervening the variable gene segments and the D gene segment cluster, as well as the intervening sequences between the J_{H} gene cluster and the Cµ coding region are still present on both IgH alleles in all preB cells. Targeting vectors for DJ_{H} rearranged alleles therefore have to contain homology regions upstream of the most 5' located D element (D_{FL16.1}) and downstream of the most 3' located J_{H} gene segment (J_{H}4).

The genomic organization of one possible D_{FL16.1}-J_{H4} rearranged allele with surrounding genomic DNA sequences is depicted in Fig. 5a. As a general strategy for gene targeting, a short region of DNA sequence homology (1-1.5 kb) located upstream of the region or gene to be deleted is cloned into a unique restriction enzyme recognition site upstream of a "floxed" positive selection marker (here Notl), and a longer region of DNA sequence homology (>2.5kb) located downstream of the region or gene to be deleted is cloned into a unique restriction enzyme recognition site located between the "floxed" positive selection marker and the diphtheria toxin α expression cassette (Fig. 5a) (here Ascl). Expression of the negative selection marker (diphtheria toxin α) is therefore only prevented, if homologous recombination occurs in the long region of DNA sequence homology of the targeting vector and the endogenous gene locus, such that the DT-α expression cassette will be lost upon targeted integration of the targeting vector into the chromosomal DNA. If homologous recombination further occurs in the short homology arm, the region located between the double crossover will be replaced by the positive drug selection marker. Thus using antibiotic selection on preB cells transfected with the targeting construct results in strong selection for the targeted integration of the targeting vector by homologous recombination resulting in the deletion of the endogenous gene locus.

A short region of homology upstream of the most 5' located D element (D_{FL16.1}) can be PCR amplified from mouse genomic DNA using the following primers designed based on the published NCBI-Genbank sequence AF018146:

SEQ ID NO. 19 (P015)     5'-aatGCGGCCGCgaacctcctgtgttgcaagcacaaatggg -3',
and
SEQ ID NO. 20 (P016)     5'-aatGCGGCCGaggcagcacggttgagtttcagttgtcatc-3'

Nucleotides of the appended Notl restriction enzyme recognition sites introduced for cloning purposes are indicated in uppercase letters. The primers will amplify a 1465bp genomic PCR fragment that can be digested with Notl restriction enzyme and ligated into Notl linearized pBSL-flneo-DT4 generating pBSL-5'J_{H}-flneo-DT4 (Fig. 5a). The long region of sequence homology downstream of the J_{H} gene segment cluster, also comprising the EµH heavy chain intron enhancer, can be PCR amplified from mouse genomic DNA using the following primers designed based on published NCBI-Genbank sequence file J00440 (nucleotides of the appended Ascl restriction enzyme recognition site are indicated in uppercase letters):

SEQ ID NO. 21(P017) 5'-ttGGCGCGCCgtaagaatggcctctccaggtctttatttt -3'
and
SEQ ID NO. 22 (P018) 5'-ttGGCGCGCCagctcagctcagctcaccccagctcagctc-3'

The primers will PCR amplify a 3215bp genomic PCR fragment from mouse genomic DNA that can be digested with Ascl restriction enzyme and ligated into Ascl linearized pBSL-5'J_{H}-flneo-DT4 generating the final J_{H} targeting vector pBSL-5'J_{H}-flneo-3'J_{H}-DT4 that can be used to delete all remaining D_{H} and J_{H} gene segments in DJ_{H} rearranged preB cells (Fig. 5a).

### ii) Construction of κL chain targeting vectors (Fig. 5b)

Like in the case of the IgH chain locus, the κL chain gene loci may be inactivated by several *cis*-acting mutations, including e.g. deletions of all of the J_{κ} gene segments, the κL chain intron enhancer (κiE), the constant κL chain region (C_{κ}), or the 3'κ enhancer (3'κE). As an example, targeting constructs and methods are described here that can be used for the targeted deletion of all of the _{κ} gene segments. Furthermore, construction of targeting constructs described herein is presented only for targeting constructs containing a "floxed" neomycin resistance marker. Cloning of targeting constructs containing "floxed" puromycin or hygromycinB expression cassettes are performed in analogous fashion. A short region of homology upstream of the J_{κ} gene segment cluster is PCR amplified from mouse genomic DNA using the following primers designed according to published NCBI-Genbank entry V00777 (nucleotides of the appended Notl restriction enzyme recognition site used for cloning purposes are printed in uppercase letters):

SEQ ID NO. 23 (P019) 5'-tatGCGGCCGcttatctttctcctttattaacggttgctg-3'
and
SEQ ID NO. 24 (P020) 5'-tatGCGGCCGCacagtggtagtactccactgtctggctg -3'

Primer P019 binds to position 1-30 and primer P020 to position 711-738 of NCBI-Genbank entry V00777. The resulting 738 bp PCR fragment is digested with Notl restriction enzyme and is ligated into Notl linearized empty targeting vector pBSL-flneo-DT4 generating pBSL-5'J_{κ}-flneo-DT4 (Fig. 5b). The long region of DNA sequence homology downstream of the Jₖ gene segment cluster comprising the κiE and the constant kL chain region (Ck) is PCR amplified from mouse genomic DNA using the following primers designed based on published NCBI-Genbank entry V00777 (nucleotides of the appended Ascl restriction enzyme recognition site are printed in uppercase letters):

SEQ ID NO. 25 (P021) 5'-ttGGCGCGCCtgtgtaagacacaggttttcatgttaggag -3'
and
SEQ ID NO. 26 (P022) 5'-ttGGCGCGCCtgcttcgccaagtttactgggtaggttg -3'

Primer P021 will bind to position 2129-2158, and primer P022 to position 5456-5483 of NCBI-Genbank sequence V00777. The resulting 3379 bp PCR fragment is digested with AscI restriction enzyme and is ligated into AscI linearized vector pBSL-5'J_{κ}-flneo-DT4 generating pBSL-5'J_{κ}-flneo-3'J_{κ}-DT4 (Fig. 5b).

### iii) Generation of a RAG locus targeting vector (Fig. 5c):

As an alternative to inactivating the potential of preB cell to express endogenous immunoglobulins by introducing *cis*-acting mutations within the IgH and IgκL chain gene loci, preB cells can also be rendered incapable for rearrangement of their endogenous Ig genes, by introducing trans-acting mutations. This can for instance be achieved by targeting one of the two recombination activating (RAG) genes that are essential for Ig gene rearrangements. Murine preB cells with deletions in either the RAG-1, or the RAG-2 gene will be unable to rearrange immunoglobulin gene segments and are therefore unable to express endogenous immunoglobulins.

As an example, the construction of a targeting vector is described allowing the complete deletion of the ORF of the RAG-1 gene. A short arm of DNA sequence homology of about 1kb of genomic sequence upstream of the RAG-1 coding region is amplified by the following primer pair designed based on published NCBI-Genbank sequence AC084753:

SEQ ID NO. 27 (P023) 5'-tataGCGGCCGcttctgctcctcttctttagtactggattc-3'
and
SEQ ID NO. 28 (P024) 5'-tataGCGGCCGCgttggctaagctacctgggaacaatggggg-3'

Notl cloning sites appended to the 5' end of each primer are highlighted in uppercase letters and are used for the cloning of a 1095bp PCR fragment containing the genomic reagion upstream of the RAG-1 open reading frame (Fig. 5c). The PCR product is digested with Notl and ligated into the unique Notl site of a positive-negative targeting vector, e.g. pBSL-flneo-DT4. A plasmid clone containing the short arm of homology upstream of the RAG-1 coding region in correct orientation is isolated and designated pBSL-5'R1-flneo-DT4.

For the cloning of the long arm of a targeting construct for the RAG-1 gene, a genomic region of approximately 3kb directly downstream of the RAG-1 coding region (Fig. 5c) is amplified by the following primer pair, designed based on Genbank sequence AC084753:

SEQ ID NO. 29 (P025) 5'-ttGGCGCGCCataggatctccacatagaagttggtatttgcc-3'
and
SEQ ID NO. 30 (P026) 5'-ttGGCGCGCCgtgggcacacatatgtcatcccagttaccc-3'

Recognition sequences for the Ascl resrtiction enzyme are appended to the 5' end of each primer and are used for the cloning of the 2954 bp long PCR fragment containing the long arm of DNA sequence homogy downstream of the RAG-1 open reading frame. After AscI digestion, this PCR product is cloned into the unique AscI restriction site of the vector pBSL-5'R1-flneo-DT4. A plasmid clone containing the long arm of homology downstream of the RAG-1 coding region in the correct orientation is isolated and designated as pBSL-5'R1-flneo-3'R1-DT4 (Fig. 5c) and can be directly used for the targeted deletion of the endogenous RAG-1 gene in preB cells.

### Step 5: Targeting of endogenous gene loci in long-term proliferating, stromal cell, IL7 dependent murine preB cells

For the sequential targeted deletion of both alleles of an endogenous gene locus, two different strategies can be applied. One strategy is to utilize targeting vectors with two different positive selection markers for the targeting of the two different alleles. Alternatively,another strategy is to use the same targeting construct twice for the sequential targeting of both endogenous alleles. However, in case of the latter strategy targeting of the first allele has to be followed by the transient expression of an expression vector for cre-recombinase into preB cells, which will lead to the permanent deletion of the loxP site flanked drug selection marker, such that the same antibiotic drug selection may be used for the second targeting of the other allele.

As an example, the experimental procedure for the sequential targeting of both alleles of an endogenous gene according to the first strategy, i.e. using targeting vectors with two different antibiotic resistance markers (puromycin and hygromycinB) is described.

For targeting of a gene locus on the first allele, 20 µg of linearized targeting vector with a puromycin resistance marker is transfected into 10⁷ preB cells resuspended in PBS by electroporation at 350V with 960µF capacitance using a BioRad electroporator and a 0.4cm electroporator cuvette. The cells are then plated in IL7 growth medium at a density of 5x10⁴ cells/well into 96well plate cultures coated with a subconfluent layer of 3000 rad irradiated puromycin resistant ST-2 stromal cells. 30 hours post transfection puromycin is added at a final concentration of 2µg/ml. Puromycin resistant colonies are isolated 7-10 days post transfection and expanded on fresh, irradiated and puromycin resistant ST-2 stromal cells under continued selection with 2µg/ml puromycin. Individual puromycin resistant preB cell clones are analyzed for the targeted integration of the targeting vector into one allele by standard PCR and genomic southern blot hybridization analysis. PreB cell clones with a targeted integration of the targeting vector on one allele are again transfected, this time with 20µg of linearized targeting vector containing a hygromycinB resistance marker under the same conditions as described above. Transfected preB cells are then plated in IL7 growth medium at a density of 5x10⁴ cells/well into 96well plate cultures coated with a subconfluent layer of 3000 rad irradiated puromycin and hygromycinB double resistant ST-2 stromal cells. Double resistant preB cell clones are selected 30 hours post transfection by addition of 2µg/ml puromycin and 400µg/ml hygromycinB. Puromycin and hygromycin B double resistant colonies are isolated 7-10 days post transfection and expanded on fresh, irradiated and puromycin/hygromycinB double resistant ST-2 stromal cells under continued selection with 2µg/ml puromycin and 400µg/ml hygromycinB. Individual puromycin/hygromycinB double resistant preB cell clones are analyzed for the targeted integration of the targeting vector into the second allele again by standard genomic PCR and/or southern blot analysis.

Puromycin/hygromycinB double resistant preB cell clones with two targeted alleles may then transiently be transfected with a constitutive cre-recombinase expression vector in order to repeat gene targeting experiments on other gene loci and alleles. This can be done by transiently transfecting preB cells with 20µg of supercoiled cre-recombinase expression vector by electroporation as described above. The transiently transfected cells are then plated under limiting dilution conditions in IL-7 growth medium into several 96 well plates onto 3000 rad irradiated, subconfluent ST-2 stromal cells. After 7 days of culture a set of 48 clones is replica plated onto normal ST-2 stromal cells for continued culture, and onto either puromycin resistant or hygromycinB single resistant ST-2 cells und the respective selection. Previously puromycin/hygromycinB double resistant preB cell clones that became sensitive two both antibiotic drugs have likely undergone cre-recombinase mediated deletion of both loxP flanked resistance markers. Once the cre-recombinase mediated deletion of the two resistance markers has been verified by southern blot analyses, the clones can be subjected to the additional gene targeting procedures for other endogenous gene loci and alleles.

### Example 6: Construction of retroviral expression constructs for human immuno-globulin polypeptides

In order to re-program murine stromal cell, IL7 dependent preB cells for the production of human immunoglobulins, novel retroviral expression vectors encoding human antibody polypeptides can be used. Methods for the generation of recombinant retroviral vectors are described that allow the regulated expression of human IgH and L chain proteins, depending on the B cell differentiation stage, first as membrane-bound and later as secreted immunoglobulins. It should be noted that any type of heterologous binding protein can be cloned into these retroviral vectors allowing the expression of any type of heterologous and human binding protein. The description of the cloning of retroviral transfer vectors for the expression of human IgH and IgL chains described herein, should therefore only be viewed by way of illistration and not by way of exclusion. Recombinant retroviral vectors can accommodate up 7-10kb of heterologous DNA sequences, which requires the design of separate constructs for IgH and L chain glycoproteins including all the required cell-type and differentiation stage specific promoter, enhancer and control elements, as well as endogenous Ig gene splice signals. These control elements are necessary to ensure proper expression, membrane deposition and secretion of immunoglobulins, their affinity maturation, in the various B cell differentiation stages, as well as high level secretion of heterologous antibodies in plasma cells. Individual retroviral constructs for IgH and L chains can be co-transduced into preB cells upon which the constructs stably integrate into the genome of the infected preB cells. If murine preB cells are used which are deprived of the potential express endogenous immunoglobulins (see description above), then only heterologous or human antibodies or binding proteins will be expressed in B lineage cells upon differentiation of the retrovirally transduced preB cells.

### (a) Generation of retroviral constructs for the regulated expression of IgH chains

Because the retroviral constructs for the various IgH and L chain proteins will eventually be transduced into murine cells, the promoter and enhancer elements controlling Ig expression will be from murine origin in order to ascertain optimal interactions with B lineage specific murine transcription factors.

The starting vector from which the retroviral expression constructs are generated is the empty retroviral cloning vector pLIB (Clontech) (Fig. 12a). The generation of a recombinant retroviral expression vector for human immunoglobulin heavy chains is a multistep cloning process, which involves the sequential cloning of promoter and enhancer elements, as well as the coding regions for the various variable and constant regions of human antibodies into the empty retroviral cloning vector pLIB. In a first step, a murine V_{H} promoter is PCR cloned into the unique Clal restriction site of the multiple cloning site of pLIB. This is done by PCR amplification of a mouse V_{H} promoter element from mouse genomic DNA using the following primers designed based on the published NCBI-Genbank database accession number X71119.

SEQ ID NO. 31 (P027) 5'-ccATCGATtgactggatgcttgttaattctaataag-3', and
SEQ ID NO. 32 (P028) 5'-ccATCGATGGATCCtgtgtgccagtaactgtagagagaac -3'

These primers will PCR amplifiy a 394bp V_{H} promoter fragment which will contain restriction enzyme recognition sites for Clal (ATCGAT) at their 5' and 3' ends, because they are incorporated at the 5' ends of primers P027 and P028. In addition to Clal sites in both primers, the reverse primer P028 contains an additional BamHI restriction site (GGATCC, underlined) introducing another unique restriction site into the PCR product (see Fig. 12a), which will be required for further cloning steps. After Clal restriction enzyme digestion of the PCR fragment, the amplified V_{H} promoter PCR fragment including the novel BamHI site is ligated into Clal linearized pLIB vector. Ligation products containing the V_{H} promoter in the desired orientation (opposite to the transcriptional orientation of the 5'LTR promoter) are identified by diagnostic restriction enzyme digestions and DNA sequencing and are designated pAB-1 (Fig. 12a).

The second step is the cloning of the murine intron heavy chain enhancer (EµH) with its flanking matrix attachment regions (MARs) into retroviral vector pAB-1. The EµH enhancer element with its flanking (MARs) is known to be required for the efficient expression of immunoglobulin proteins in B lineage cells. The cloning of the EµH enhancer element is performed by amplifying a 983bp PCR fragment from mouse genomic DNA using the following primers designed according to published NCBI-Genbank sequence J00440:

SEQ ID NO. 33 (P029)
5'-cgGGATCCAAGCTTagagaggtctggtggagcctgcaaaagtcc-3'
and
SEQ ID NO. 34 (P030)
5'-gatcGCGGCCGCtctagataattgcattcatttaaaaaaaaatatttc-3'

Restriction sites (BamHI/HindIII for P029 and NotI for P030) appended to the 5' end each primer are highlighted in uppercase letters. The PCR product can then be digested with BamHI and Notl restriction enzymes and is directionally ligated into BamHI/NotI linearized retroviral vector pAB1, resulting in vector pAB-2. The sense primer P029 contains the sequence for an additional HindIII site (underlined), which will give rise to a unique restriction enzyme recognition site in pAB-2, required for later cloning steps.

Next, a DNA fragment containing the murine 3'α enhancer is added to the construct pAB-2. The 3'α enhancer is known to be required for optimal and high level expression and secretion of antibodies at the differentiation stage of plasma cells. The 3'α enhancer is known to contain four DNAsel hypersensitivity sites (HS1, HS2, HS3, and HS4) that comprises various binding sites for late B cell/plasma cell specific transcription factors. In reporter gene assays, it was shown that HS1 and 2, which are located within a 0.6kb genomic DNA fragment, confer at least 80% of the 3'α enhancer activity with little contribution of HS3 and 4 that are highly homologous in DNA sequence.

All four 3'α enhancer components, HS1, 2, 3 and 4, constitute a so-called locus control region (LCR), which is able to regulate the transcriptional activity of a given gene locus independently of flanking DNA sequences. A basic retroviral expression vector for immunoglobulin heavy chains should therefore at least contain the HS1,2 regions of the 3'α enhancer which can be PCR amplified from mouse genomic DNA using primers designed according to the published NCBI-Genbank sequence entry X96607 (recognition sequences for additional restriction enzyme sites required for the cloning of the PCR fragment or later cloning steps are highlighted in uppercase letters):

SEQ ID NO. 35 (P031)
5'-gcatGTCGACACGCGTggggctcagatatcagtaccagaaacaagg-3', and
SEQ ID NO. 36 (P032)
5'-cgatGTCGACCTCGAGttggagtcacaggcctgtctccatgtgg-3'

In addition to 5' terminal Sall sites (GTCGAC) in each of the primers, the sense primer P031 contains an additional Mlul site (underlined) and the reverse primer P028 contains an additional Xhol site (underlined). Primers P031 and P032 will amplify a 644bp PCR fragment that can be digested with Sall restriction enzyme and ligated into Sall linearized vector pAB-2. A clone with the insert in the desired orientation (see Fig. 12b) is determined by diagnostic restriction enzyme digestion and DNA sequencing, and is designated pAB-3 (Fig. 12b).

Optionally, the further 3'α enhancer components HS3 and 4 can be included in the retroviral vector constructs for IgH expression. For this a 466 bp fragment comprising the 3'α enhancer HS4 region can be PCR amplified from mouse genomic DNA as a template using the following primers designed according to published NCBI-Genbank sequence entry S74166:

SEQ ID NO. 37 (P033) 5'-gcaCTCGAGgggtagatgcagcctgtgttccgtttactg -3' , and
SEQ ID NO. 38 (P034) 5'-gctCTCGAGCATGCctgagcccaccaggaagtcctctgtg -3'

Restriction enzyme sites appended to the 5' termini of each primer are highlighted in uppercase letters. In addition to Xhol restriction sites (CTCGAG) present in both primers, the reverse primer P034 contains an additional Sphl restriction site (GCATGC, underlined) containing an additional unique restriction site required for later cloning purposes. PCR amplification with primer pair P033 and P034 on genomic mouse DNA will result in a 466 bp PCR fragment that can be digested with the restriction enzyme Xhol and which is then ligated into Xhol linearized vector pAB-3. A clone with the HS4 insert in the desired orientation is determined by diagnostic restriction enzyme digestion and DNA sequencing, and is designated as vector construct pAB-4 (Fig. 12b). The last 3'α enhancer fragment, HS3, can be PCR amplified using mouse genomic DNA as a template with the following primers designed according to published NCBI-Genbank sequence entry X96607 (recognition sequences for the restriction enzyme Sphl are again highlighted in uppercase letters):

SEQ ID NO. 39 (P035) 5'- ttaaGCATGCaaccacatgcgatctaagggatattgggg -3'
and
SEQ ID NO. 40 (P036) 5'- ttaaGCATGCgatcattgagctccggctctaacaactggg -3'

The PCR amplified HS3 3'α enhancer region is digested with Sphl restriction enzyme and ligated into Sphl linearized vector pAB-4 in order to generate pAB-5. Any of the vectors pAB-3, 4, or 5 can be used for inserting the coding regions for heterologous immunoglobulin heavy chains (Fig. 12b).

Each of the human immunoglobulin isotypes can be expressed as a membrane bound surface immunoglobulin or as a secreted antibody, which is regulated by alternative splicing events either maintaining or deleting the membrane spanning exons from the mRNA transcript. Although each immunoglobulin isotype has its own coding region for the membrane spanning exons, the membrane regions of all human IgG, IgA and IgE antibodies are virtually identical for the individual subtypes. Therefore, the same IgG membrane exons can be employed for membrane anchoring IgG1, IgG2, IgG3 and IgG4 antibodies, a different set of exons are used for both IgA1 and IgA2, and each different exons are required for each IgE and IgM antibodies.

Therefore, the next step in the cloning procedure towards the generation of recombinant retroviral Ig expression constructs is the insertion of the various membrane spanning regions for human IgG, IgA, IgM and IgE antibodies into any of the vectors pAB-3, 4, or 5. As a representative example, the further cloning procedures are described for the basic retroviral construct pAB-3 containing the V_{H}promoter, the EµH enhancer and the minimal 3'αE enhancer (HS1,2) elements (Fig. 12c).

The µH chain membrane spanning region is encoded by two exons, µM1 and µM2, located 1.85 kb downstream of the C_{µ}H constant region exons C_{µ}H₁-C_{µ}H₄. The µM1 and µM2 exons including the endogenous polyadenylation site are contained within a genomic fragment of 773bp that can be amplified from mouse genomic DNA by PCR using the following primers designed based on published NCBI-Genbank contig sequence NT_010168:

SEQ ID NO. 41 (P037) 5'-catACGCGTcgctgcatcaggctttcagggcccagccc -3'
and
SEQ ID NO. 42 (P038) 5'-catACGCGTccctcaccagaaagcagtttcatggataaaatg-3'

The Mlul restriction sites appended to the 5' end of each primer are highlighted in uppercase letters. The resulting PCR product can be digested with Mlul restriction enzyme and the resulting 773bp fragment is ligated into Mlul linearized vector pAB-3. A clone containing the µM1 and µM2 exons in correct orientation and DNA sequence as verified restriction enzyme mapping and by DNA sequencing is designated construct pAB-3.1 (Fig. 12c).

Membrane deposition of IgG antibodies can e.g. be accomplished by the membrane spanning exons of the IgG₁ gene. The IgG₁ membrane spanning region is encoded by two exons located 1.25kb downstream of the C_{γ1}H exons spanning a region of 1.9 kb. In order to shorten the intron between γ1M1 and γ1M2, a single overlap extension (SOE) PCR approach is used, by which the two exons are fused to a smaller PCR fragment. Generally, the fusion of two PCR products can be achieved, if the reverse primer of the upstream PCR product and the sense primer of the downstream PCR product exhibit roughly 30-40 bp of complementarity. These regions of perfect complementarity can be appended as 15-20 nucleotides to the 5' ends of the reverse primer and forward primers used for the amplification of the upstream and the downstream PCR products, respectively. However, in case of the fusion of the two γ1M1 and γ1M2 exons, by coincidence, two completely identical sequences of 28bp are present 78bp downstream of γ1M1 and 256bp upstream of γ1M2 exon. These sequences can be used to generate a shorter PCR fragment by SOE-PCR containing both the γ1M1 and γ1M2 exons for the membrane spanning region of γH chains. Therefore, two PCRs are performed in parallel on human genomic DNA using the following primers designed based on the published NCBI-Genbank sequence AL122127. A PCR fragment of 315 basepairs containing the γ1 membrane spanning exon 1 (γ1M1) is amplified from human genomic DNA using primers: 
SEQ ID NO. 43 (P039) 5'-catACGCGTacagagggaatcacccccagaggcccaagccc -3'
and
SEQ ID NO. 44 (P040) 5'-gacagcgtcagggacaggtggggacagc -3',
 with primer P039 binding to position 9710-9741, and primer P040 binding to position 9435-9462 of NCBI-Genbank sequence entry AL122127.

A 555 basepair PCR fragment containing the membrane spanning exon 2 (γ1M2) can be amplified from human genomic DNA using primers: 
SEQ ID NO. 45 (P041) 5'-gctgtccccacctgtccctgacgctgtc -3', and
SEQ ID NO. 46 (P042)
5'-catACGCGTttatttggaagggggcgtgtcaggtgtgtcagggtc -3'
 with primer P041 binding to position 8137-8164, and primer P042 binding to position 7624-7654 of NCBI-Genbank sequence entry AL122127. The two PCR products can then be fused to a 822 bp PCR fusion product by mixing 1/100 of the γ1M1 exon PCR product with 1/100 of the y1M2 exon PCR product and repeating a PCR with sense and reverse primers P039 and P042. In addition to Mlul restriction enzyme recognition sites present in both primers P039 and P042 (highlighted in uppercase letters), which are required for the cloning of the SOE fusion PCR product, primer P042 additionally contains the complementary sequence of an artificial polyadenylation signal (underlined) allowing correct transcriptional termination of the immunoglobulin transcript in the final retroviral immunoglobulin expression vector. The 832bp γ1M1γ1M2-polyA signal PCR fragment is then digested with Mlul restriction enzyme and is ligated into vector pAB-3. A clone with the correct orientation of the insert verified by restriction enzyme mapping and DNA sequencing is designated clone pAB-3.2 (Fig. 12c).

Cloning of the α1 membrane spanning coding region can be achieved by PCR amplifying from human genomic DNA a fragment containing the single exon for α1M including its endogenous polyA site. The following primers can used for this PCR amplification, designed according to the published NCBI-Genbank sequence entry M60193:

SEQ ID NO. 47 (P043) 5'-atcACGCGTctcaggccttagatggggacccagaccc-3'
and
SEQ ID NO. 48 (P044) 5'-attACGCGTgacccccgtctcctcattcagagtctgtg-3'

Both primers contained Mlul cloning sites appended to the 5' end of each primer (highlighted in uppercase letters) and will amplify a PCR product of 768 basepairs that can be digested with Mlul restriction enzyme that is then ligated into Mlul linearized pAB-3. A clone with correct orientation of the insert verified by restriction enzyme mapping and DNA sequencing is designated clone pAB-3.3.

For the expression of membrane bound IgE antibodies the membrane spanning region for ε₁H chains, which is encoded by two exons, ε₁M1 and ε₁M2 is PCR cloned. For this a 858 bp PCR product is amplified from human genomic DNA using the following primers designed according to published NCBI-Genbank sequence entry X63693:

SEQ ID NO. 49 (P045) 5'-catACGCGtcgggacctgggtgcccaccctcagggctgg-3'
and
SEQ ID NO. 50 (P046)
5'-aatACGCGTttatttgtgccctgggctgggtgccgggccctccttgg-3'

Both primers contain Mlul cloning sites appended to the 5' end of each primer (highlighted in uppercase letters) resulting in a 858 bp PCR product that can be digested with Mlul restriction enzyme. In addition, the reverse primer P046 is designed to contain the complementary sequence of an artificial polyA signal (underlined) ensuring proper termination of mRNA transcription in the final expression construct. The Mlul digested PCR fragment is ligated into Mlul linearized pAB-3. A clone with correct orientation of the insert verified by restriction enzyme mapping and DNA sequencing is designated clone pAB-3.4.

Following cloning of the various membrane spanning exons for IgG, IgA, IgM and IgE isotypes, the exons for the various human constant regions have to be inserted into retroviral constructs pAB-3.1 to 3.4. For this, DNA fragments encoding the constant region exons are cloned into the unique Notl restriction enzyme recognition site of each of the pAB-3.1 to 4 constructs.

Cloning of retroviral expression vectors for all immunoglobulin H chain isotypes, γ 1, γ 2, γ 3 and γ 4 is depicted in Fig. 12d. The following primers can be used for the PCR amplification of the four different IgG heavy chain subtypes using human genomic DNA as a PCR template:

For γ1 (primers designed according to published NCBI-Genbank sequence AL122127):

SEQ ID NO. 51 (P047) 5'-gcatGCGGCCGCcctgggcccagctctgtcccacacc-3', and
SEQ ID NO. 52 (P048) 5'-gcatGCGGCCGCtgggtgctttatttccatgctgggtg-3'

For y2 (primers designed according to published NCBI-Genbank sequence J00230):

SEQ ID NO. 53 (P049) 5′-tataGCGGCCGCagggagtgggctaaggtgaggcaggtgg-3′
and
SEQ ID NO. 54 (P050) 5′-attaGCGGCCGCctcagactcggcctgacccacggaaagaac-
3′

For y3 (primers designed according to published NCBI-Genbank sequence AL122127):

SEQ ID NO. 55 (P051) 5'-gcatGCGGCCGCccagctctgtcccacaccgcagtcacatgg-3'
and
SEQ ID NO. 56 (P052) 5'-gctaGCGGCCGCtcgcaggggcccagggcagcgctgggtgctt-
3'

For y4 (primers designed according to published NCBI-Genbank sequence K01316):

SEQ ID NO. 57 (P053) 5′-attaGCGGCCGCggatagacaagaaccgagggcctctg-3′
and
SEQ ID NO. 58 (P054) 5′-taatGCGGCCGCccagggcagtggtgggtgctttatttcc-3′

In each case, the PCR products of 1788bp for γ₁H, of 1959bp for γ₂ H, of 2374bp for γ₃H, and of 1820bp for γ₄H can be digested with Notl restriction enzyme, upon which the digested fragment is ligated into Notl linearized pAB-3.2 (Fig. 12d). Constructs containing the inserts in correct orientation and without any mutations as determined by restriction enzyme mapping and DNA sequencing are designated pAB-3.2-G1, pAB-3.2-G2, pAB-3.2-G3, and pAB-3.2-G4, respectively.

For the cloning of the constant region exons encoding µH, α1H, α2H, and εH chains, identical method are employed (Fig. 12e). The following primers are used for the PCR amplification of the different IgH isotypes using human genomic DNA as a template:

For µH (primers designed according to published NCBI-Genbank sequence AB019441):

SEQ ID NO. 59 (P055) 5'-attaGCGGCCGCaccccagtaggccagagcatcgtgcac-3'
and
SEQ ID NO. 60 (P056) 5'-taatGCGGCCGCacccctgatagccatgacagtctggg-3'

For α₁H and α₂H (primers designed according to published NCBI-Genbank sequences J00220, and J00221):

SEQ ID NO. 61 (P057) 5'-attaGCGGCCGCtgtcaccaggcctctgtgctgggttcc–3'
and
SEQ ID NO. 62 (P058) 5'-tattGCGGCCGCcggatggaagcgtggggctgcttgggg –3'

For εH (primers designed according to published NCBI-Genbank sequence L00022):

SEQ ID NO. 63 (P059) 5'-taatGCGGCCGCcacggggtccccagctcccccatccagg-3'
and
SEQ ID NO. 64 (P060) 5'-atatGCGGCCGCtcccaagaatgggtgtactggggctc -3'

In each case, the PCR products of 2229bp for µH, of 1667bp for α₁H and α₂H, and of 1908bp for εH can be digested with Notl restriction enzyme and can be ligated into Notl linearized pAB-3.1, pAB-3.3, and pAB-3.4, respectively (Fig. 12e). Constructs containing the inserts in correct orientation and without any mutations as determined by restriction enzyme mapping and DNA sequencing are designated pAB-3.1-M, pAB-3.3-A1, pAB-3.3-A2, and pAB-3.4-E, respectively.

The different retroviral H chain expression constructs controlled by natural IgH chain promoter and enhancer elements still require the insertion of a variable domain coding region, which will be presented after the description of the methods for cloning of the retroviral IgL chain expression vectors, because similar strategies for expressing unique or diverse specificities will be presented for both the complete retroviral IgH and IgL chain expression constructs.

### (b) Generation of retroviral constructs for the regulated expression of IgκL chains

Similarly to the constructs designed for IgH chain expression, retroviral constructs designed for the differentiation stage specific expression of IgκL chains require the presence of defined κL chain locus specific promoter and enhancer elements. These include a Vκ promoter, the κ light chain intron enhancer (κiE), and the κ3' enhancer (κ3'E) elements. The sequential cloning of these elements can be accomplished as follows (Fig. 13 a,b).

A mouse Vκ promoter is PCR amplified as a 290bp PCR fragment from mouse genomic DNA using the following primers (designed according to the published NCBI-Genbank sequence entry X52768):

SEQ ID NO. 65 (P061) 5'-ccATCGATcctggtctacagtgtgaggtactggac -3', and
SEQ ID NO. 66 (P062)
5'-ccATCGATGGATCCtctgagagctggaagagaggatgctttattagg-3'

Restriction sites appended to the 5' end each primer are highlighted by uppercase letters. In addition to Clal sites present at the 5' ends of both primers, the reverse primer P062 contains an additional BamHI restriction site (GGATCC, underlined) introducing another unique restriction site into the PCR product which will be used for later cloning purposes. After Clal restriction enzyme digestion of the PCR fragment, the amplified V_{κ} promoter is ligated into Clal linearized pLIB vector. Ligation products containing the V_{κ} promoter in the desired orientation (opposite to the transcriptional orientation of the 5'LTR promoter) are identified by diagnostic restriction enzyme mapping and DNA sequencing giving rise to construct pAB-6 (Fig. 13a).

The κiE and the coding region for the κL chain constant domain are located in close proximity in both the human and the mouse κL chain gene locus and the enhancer appear to function across species. Therefore, the optional cloning procedures for two different retroviral expression vectors encoding κL chains is described, one utilizing the human, and the other utilizing the murine κ intron enhancer (κiE). It should be noted, that both constructs can be used interchangeably. Cloning of the construct containing the human κiE can be achieved by amplifying a PCR fragment from genomic human DNA containing both the human κiE and the human Cκ exon including a 5' located MAR using the following primers (designed based on published NCBI-Genbank sequence entry AF017732):

SEQ ID NO. 67 (P063)
5'-cgGGATCCTGACGCGTaccagggagaagactgatttattagagatttc -3', and
SEQ ID NO. 68 (P064)
5'-gatGCGGCCGCaaagattcactttatttattcattctcc-3'

Additional restriction sites appended to the 5' end of each primer are again highlighted in uppercase letters (BamHI in primer P063 and NotI in P064). Primer P063 contains the sequence for an additional Mlul restriction site used in later cloning steps. The PCR primers amplify a 2359 bp fragment that can be digested with BamHI and Notl, and which can then be directionally cloned into BamHI-Notl linerized pAB-6 generating retroviral construct pAB-7 (Fig. 13a).

For the construct utilizing the murine κiE a PCR product containing the murine κiE is fused to a PCR product containg the human Cκ region by the SOE-PCR approach described previously. For this, first PCR amplification of the murine κiE and the human Cκ region is performed in parallel, with the κiE reverse primer and the Cκ region forward primer exhibiting a 36bp region of complementarity. In the second PCR, which is used to fuse these two PCR products, 1/100 of each PCR reaction is mixed and amplified with the κiE forward (P065) and the Cκ region reverse primer (P064) resulting in the fusion of the two PCR products at the region of 36bp terminal complementarity.

The following primers can be used for the amplification of the murine κiE (designed based on the published NCBI-Genbank sequence entry V00777) using mouse genomic DNA as a template:

SEQ ID NO. 69 (P065) 5'-cgGGATCCAAGCTTgaaaatgtttaactcagctactataatccc-
3'
SEQ ID NO. 70 (P066) 5'-gttttgttggagctcccctttgaagatattctcaggcttccttc-
3'

The following primers can be used for the amplification of the human Cκ region (designed based on the published NCBI Genbank sequence entry AF017732) using human genomic DNA as a template:

SEQ ID NO. 71 (P067)
5'-aatatcttcaaaggggagctccaacaaaacaatttagaactttattaag-3'
SEQ ID NO. 68 (P064)
5'-gatGCGGCCGCaaagattcactttatttattcattctcc-3'

The regions of terminal complementarity in primers P066 und P067 are underlined, which mediate the fusion of the two PCR products in a second PCR carried out with primers P065 and P064 and a mixture of the two first PCR products as a template. The resulting murine κiE-human Cκ fusion PCR product of 1524bp can be digested with BamHI and Notl and is then directionally cloned into BamHI-Notl linearized vector pAB-6 generating vector pAB-8 (Fig. 13a).

Next the murine κ3'E is cloned downstream of the human Cκ regions in both pAB-7 and pAB-8 generating constructs pAB-7.1 and pAB-8.1, respectively. The primers used for the PCR amplification for the κ3'E from mouse genomic DNA are as follows (designed based on published NCBI-Genbank sequence entry X15878):

SEQ ID NO. 72 (P068) 5'-acgcGTCGACtagaacgtgtctgggccccatgaaacatc –3', and
SEQ ID NO. 73 (P069) 5'-cgatGTCGACagctcaaaccagcttaggctacacagagaaac-3'

Both primers contain Sall restriction sites (highlighted in uppercase letters), such that the resulting 827bp PCR product can be cloned into the compatible Sall restriction sites of pAB-7 and pAB-8. Vector clones carrying the insert in the correct orientation and with the correct DNA sequence, as determined by restriction enzyme mapping and DNA sequencing, are designated pAB-7.1 and pAB-8.1 (Fig. 13a).

In a next cloning step, a puromycin resistance marker under control of a constitutive SV40 promoter is cloned into the retroviral κL chain expression constructs. The inclusion of an expression cassette for puromycin resistance is preferred, because both IgH and IgL chain retroviral constructs need to be stably co-transduced into murine preB cells in order to allow expression of complete heterologous antibodies. In order to increase the efficiency of retroviral cotransduction, retroviral IgL chain constructs are transduced first, and stably transduced cells are selected for the integration of the IgL chain constructs using puromycin selection. A secondary transduction of IgH chain retroviral vectors will then give rise to cells with the potential for antibody production in each cell that is transduced with an IgH chain retroviral vector.

The puromycin expression cassette can be cloned from the puromycin resistance vector pPur (Clontech) by SOE-PCR deleting some unwanted internal restriction enzyme sites and adding suitable Clal cloning sites to the ends of the amplified PCR fragment.

The primers used for the SOE fusion PCR are:

SEQ ID NO. 74 (P070)
5'-ccATCGATctgtggaatgtgtgtcagttagggtgtgg-3'
SEQ ID NO. 75 (P071)
5'-tcaggggatggtggcggccctaggcctccaaaaagcctcctcactacttc-3'
SEQ ID NO. 76 (P072)
5'-ctttttggaggcctagggccgccaccatcccctgacccacgcccctgacc-3'
SEQ ID NO. 77 (P073)
5'-ccATCGATccagacatgataagatacattgatgag-3'

First, two PCR amplifications are performed in parallel with primer pairs P070/P071 and P072/P073 using the same pPur template for the PCRs. Reverse primer P071 and forward primer P072 are designed to contain a region of 36bp perfect complementarity (underlined), which will allow the fusion of the two PCR products by a second PCR using aliquots of the previous PCR fragments in an additional PCR amplification using primers P070 and P073 alone. The latter primers contain Clal restriction sites (uppercase letters) allowing the cloning of the resulting 1351bp puromycin expression cassette into compatible Clal sites of pAB-7.1 and pAB-8.1. This generates retroviral constructs pAB-7.1 Pur and pAB-8.1 Pur, respectively (Fig. 13b), which are the constructs into which single VJ_{κ} regions or VJ_{κ} region libraries encoding unique or diverse variable domains of κL chains, respectively, can be inserted. The procedure for cloning of the variable region exons will be presented further below together with the cloning of H chain and λL chain variable regions, after also the cloning of the retroviral expression vectors for heterologous, human λL chains have been described.

### (c) Generation of retroviral constructs for the regulated expression of IgλL chains

In some cases it is desirable to produce heterologous antibodies containing λL chains instead of κL chains. Although it might be sufficient to replace the human Cκ coding region in the retroviral constructs pAB-7.1Pur and pAB-8.1Pur with any of the human Cλ coding regions, a perfect endogenous λL chain expression pattern will only be achieved by controlling the expression of λL chains by λL chain gene locus specific promoter and enhancer elements. This is particularly true in the murine system, where κL chain gene rearrangement and expression precedes the rearrangement and expression of λL chains during B cell differentiation.

The murine λL chain gene locus specific enhancer elements are the λ2-4 enhancer and the λ3-1 enhancer, which are located close to the Cλ2, Cλ4, and Cλ1, Cλ3 constant region exons and regulate the expression of λ2, λ4, and λ1, λ3 light chain isotypes, respectively.

As in the case of κL chain retroviral expression constructs (see above), retroviral λL chain expression vectors are designed to contain a puromycin resistance gene expression cassette. Therefore, in the first cloning step, a SV40 promoter controlled puromycin expression cassette is fused to a Vλ promoter by SOE-PCR using mouse genomic DNA and pAB-7.1Pur as templates for PCR amplification, respectively. The primers for the two parallel SOE-PCR reactions are as follows (with the Vλ promoter specific primers P076 and P077 designed based on the published NCBI-Genbank database sequence entry J00591):

SEQ ID NO. 78 (P074)
5'-ccATCGATctgtggaatgtgtgtcagttagggtgtgg-3'
SEQ ID NO. 79 (P075)
5'-gatgtcacgtgagatcccagacatgataagatacattgatgagtttg-3'
SEQ ID NO. 80 (P076)
5'-tcttatcatgtctgggatctcacgtgacatcttataataaacctg-3'
SEQ ID NO. 81 (P077)
5'-ccATCGATACGCGTaattcacaaaccaagtctattattttcaata-3'

As for the SOE fusion PCRs described earlier, first two PCR amplifications are performed in parallel, i.e. the puromycin expression cassette with primers P074 and P075 on pAB-7.1Pur as a template, and the Vλ promoter with primers P076 and P077 on mouse genomic DNA as a template. Each 1/100 volume aliquots of the first parallel PCRs are then mixed and amplified again with primers P074 and P077. The puromycin expression cassette is thereby fused to the 5'end of the Vλ promoter, because of the 36bp designed perfect complementarity in reverse primer P075 and sense primer P076 (underlined) that is used for the first round parallel PCRs.

Both primers P074 and P077 contain Clal restriction sites that can be used to clone the PCR fusion product into Clal linearized pLIB. An additional Mlul restriction site (underlined) is also incorporated into reverse primer P077 giving rise to an additional unique Mlul site 3' of the Vλ promoter required for further cloning steps. A vector clone with the puromycin-Vλ promoter fusion PCR fragment inserted in pLIB in the correct orientation and with the correct DNA sequence, as verified by restriction enzyme mapping and DNA sequencing, is designated vector pAB-9 (Fig. 14a).

Next, the two murine λ2-4E and λ3-1E enhancer elements are PCR amplified from mouse genomic DNA, again followed by fusing both sequences by SOE-PCR. The following primers can be used (based on the published NCBI-Genbank sequences entries X54550 and X54608, respectively):

SEQ ID NO. 82 (P078)
5'-catGCGGCCGCgagtatccctgtgcagtggggatactcag-3'
SEQ ID NO. 83 (P079)
5'-tctcaggagagaCTCGAGactcctttgtgctctgatagcacacatgac-3'
SEQ ID NO. 84 (P080)
5'-gcacaaaggagtCTCGAGtctctcctgagatggttcataggcctgcc-3'
SEQ ID NO. 85 (P081)
5'-ggGAATTCtctagacataaggaacaaagtcagtgtgcc-3'

Like in the previous approach, first, two PCR fragnments are amplified in parallel with primer pair P078/P079 and P080/P081 using mouse genomic DNA as a template, and in a second PCR round aliquots of these PCR products are used as a template and fused by PCR amplification with primers P078 and P081 due to the 36bp complementarity of primers P079 and P080 (underlined).

The resulting 1513bp PCR fusion product can be digested with Notl and EcoRI restriction enzymes (the respective recognition sites being incorporated into primers P078 and P081; highlighted in uppercase letters) and is directionally cloned into Notl/EcoRI linearized pAB-9. A vector clone with the correct DNA sequence, as verified by restriction enzyme mapping and DNA sequencing, is designated vector pAB-10 (Fig. 14a). The complementary regions in primers P079 and P080 are designed to contain an additional unique Xhol site (highlighted in uppercase letters), in order to allow the separate removal of each of the enhancer elements, if desired.

In a next step, any of the human Cλ regions is cloned into the retroviral construct pAB-10 using the following primers (designed based on published NCBI-Genbank sequences D87023 and D87017):

SEQ ID NO. 86 (P082)      5'-ccACGCGTaaaagcttgtctaggtggagcccactccttgcc -3'

SEQ ID NO. 87 (P083)      5'-catGCGGCCGccctgtaccccacactgagaaccccgggg -3'

These primers are able to amplify any of the functional human Cλ regions as 1094bp PCR fragments if human genomic DNA is used as a template. Primers P082 and P083 contain restriction sites for Clal and Notl, respectively, allowing Clal-Notl double digestion of the PCR fragments and their directional ligation into pAB-10 in order to generate (depending on the amplified Cλ region) either pAB-11-λ1, pAB-11-λ2, pAB-11-λ3, or pAB-11-λ7 (Fig. 14b).

For the generation of a complete IgλL chain retroviral expression vector a VλJλ rearranged variable region exon has to be cloned into the unique Mlul and HindIII sites of the various pAB-11 constructs, which is described in the following section, together with the generation of complete IgH and IgκL chain retroviral expression vectors.

### Example 7: Cloning of V_{H}DJ_{H} or V_{L}J_{L} variable region exons into retroviral IgH and L chain expression constructs

The retroviral expression constructs for the different human IgH and IgL chains described so far contain all the coding regions and control elements for human antibody expression, with the exception of the coding regions for the variable domains. Diverse repertoires (libraries) of exons encoding variable region domains can e.g. be cloned from genomic DNA of human peripheral blood lymphocytes containing B lymphocytes with diverse V_{H}DJ_{H} and V_{L}J_{L} DNA rearrangements. These coding regions for the variable domains need to contain a common leader exon, located 5' of each V_{H}DJ_{H} and V_{L}J_{L} rearranged exon (Fig. 15), which is required for the proper transport of IgH and IgL chains through the endoplasmatic reticulum, the trans golgi network and eventually to the cell surface. Degenerate primers binding to the majority of leader sequences of the human IgH, IgκL and IgλL variable region leader sequences have been described and are used in combination with degenerate primers amplifying the various human J_{H} and J_{L} gene segments for the cloning of the V_{H}DJ_{H} or V_{L}J_{L} variable coding regions. Recognition sites for restriction enzymes used for the directional cloning of the PCR products (see Fig. 15) are appended to the 5' end of the degenerate primers and are highlighted in uppercase letters. Positions in the degenerate primers, which may contain more than only one nucleotide are indicated in parentheses.

Forward primers for the PCR amplificationof human Ig heavy chain variable domains including leader sequences from human genomic DNA of peripheral B lymphocytes are as follows:

SEQ ID NO. 88 (P084) 5'-gcGGATCCatggactggacctggagg(ag)tc(ct)tct(gt)c-3'

SEQ ID NO. 89 (P085) 5'-gcGGATCCatggag(ct)ttgggctga(cg)ctgg(cg)ttc(ct)t-3'

SEQ ID NO. 90 (P086)
5'-gcGGATCCatgga(ac)(ac)(at)act(gt)tg(gt)(at)(cgt)c(at)(ct)(cg)ct(ct)ctg -3'

A forward primer for the amplification of human Igκ light chain variable domains including leader sequence from human genomic DNA of peripheral B lymphocytes is as follows:

SEQ ID NO 91 (P087)
5'-gcGGATCCatggacatg(ag)(ag)(ag)(agt)(ct)cc(act)(acg)g(ct)(gt)ca(cg)ctt -3'

A forward primer for the amplification of human Igλ light chain variable domains including leader sequence from human genomic DNA of peripheral B lymphocytes is as follows:

SEQ ID NO 92 (P088)
5'-gcACGCGTatg(ag)cctg(cg)(at)c(ct)cctctc(ct)t(ct)ct(cg)(at)(ct)-3'

Note, that instead of a BamHI restriction enzyme recognition site appended to the 5' end of primers P084-P087 for cloning purposes, a recognition site for the Bglll restriction enzyme can be used instead (compare to Fig. 15), because Bglll generates the same compatible overhangs, as BamHI. In case of cloning of VλJλ regions the forward primer P088 contains a Mlul site, because the retroviral construct accepting the fragment already contains internal BamHI restriction sites. Alternatively, a primer similarly to P088 may be used containing an Ascl site producing the same CGCG overhang as Mlul restriction enzyme.

Reverse primers for the amplification of all six human IgH J chain gene segments: are as follows. HindIII sites, highlighted in uppercase letters, are appended to the 5' ends of the primers including some flanking nucleotides for cloning purposes.

A reverse primer for the PCR amplification of variabe region exons containing J_{H}1, J_{H} 4, and J_{H}5 gene segments using genomic DNA from human B lymphocytes is as follows:

SEQ ID NO 93 (P089)	5'-tatAAGCTTacctgaggagacggtgaccagggtgccctgg-3'

A degenerate reverse primer for the amplification of variable region exons containing J_{H}2, J_{H} 3, and J_{H}6 gene segments using genomic DNA from human B lymphocytes is as follows:

SEQ ID NO 94 (P090)
5'-tatAAGCTTacctgaggagacggtgacca(tg)(tg)gt(gc)cc(ta)(tc)(gt)g -3'

A reverse degenerate primer for the amplification of variabe κ region exons containing human Jκ1-4 gene segments using genomic DNA from human B lymphocytes is as follows:

SEQ ID NO 95 (P091) 5'-tatAAGCTTacgtttgatctcca(cg)(ct)ttggtccct(tgc)ggcc -3'

A reverse primer for the amplification of variabe κ region exons containing the human Jκ5 gene segment using genomic DNA from human B lymphocytes is as follows:

SEQ ID NO 96 (P092) 5'-tatAAGCTTacgtttaatctccagtcgtgtcccttggcc -3'

A reverse degenerate primer for the PCR amplification of variabe region IgλL exons containing Jλ1-3 gene segments using genomic DNA from human B lymphocytes is as follows:

SEQ ID NO 97 (P093)        5'-tatAAGCTTacctaggacggtcagcttggtccc(ta)(cg)c(tg)cc-3'

A reverse primer for the PCR amplification of variabe region IgλL exons containing Jλ7 using genomic DNA from human B lymphocytes is as follows:

SEQ ID NO 98 (P094) 5'-tatAAGCTTaccgagggcggtcagctgggtgcctcctcc-3'

For the PCR amplification and cloning of variable domain exons contain rearranged V_{H}DJ_{H} and V_{L}J_{L} gene segments with the above described primers P084-P094, equimolar amounts of primers are used, if more than one forward or reverse primer is indicated for a particular variable region. In case of cloning of variable regions for H and κL chains, the PCR fragments are digested with BamHI and HindIII and are directionally cloned into BamHI/HindIII double digested vectors for IgH and IgκL chain expression. In case of cloning of variable regions for λL chains, the PCR fragments are digested with MluI and HindIII and are directionally cloned into MluI/HindIII double digested vectors for IgλL chain expression.

### Example 8: Sequential transduction of retroviral expression vectors for heterologous IgH and L chains into genetically modified murine preB cells

The retroviral transduction of stromal cell and IL7 dependent murine preB cells has been described in detail in Example 3. For the sequential transduction of retroviral expression constructs encoding IgH and IgL chains, identical protocols are used for the transfection of the ecotropic packaging cells and the conditions to infect recombinant retroviruses into preB cells. The only difference is that two retroviral expression constructs are sequentially transduced by the protocol outlined below.

Stromal cell and IL7 dependent preB cells are first infected with supernatant from GPE packaging cells that have transiently been transfected with the retroviral construct encoding heterologous IgL chains. As described above, the IgL chain expression vectors additionally contain a puromycin resistance marker. Therefore, stably transduced cells can be selected by the addition of 2µg/ml puromycin to the tissue culture medium 24 hours post transduction. Transduced cells are cultured and expanded for an additional period of 48hours in puromycin containing medium. Non-transduced preB cells will be eliminated due to the puromycin selection and a 100% IgL chain transduced population of preB cells will be obtained after 48 hours of culture.

At this point, cells are incubated with supernatant of the ecotropic packaging cell line GPE that have transiently been transfected with retroviral vectors encoding heterologous IgH chains. These cells are then kept in culture for an additional period of 24 hours upon which they cease to proliferate due to the expression of the hetrologous immunoglobulin heavy chains. The cells are then harvested and may be used for the transplantation of immunocompromised mice, where these cells can reconstitute B lineage compartments, and where they are able to participate within an immune response giving rise to heterologous antibody secreting cells.

### Example 9: Transplantation of long-term proliferating murine precursor B cells into mice for the production of heterologous antibodies

Stromal cell and IL7 dependent preB cells expressing heterologous IgH and L chains from stably transduced retroviral expression vectors are intravenously injected into either B cell deficient JHT mice, or together with CD4⁺ T-helper cells into CB17 SCID, RAG-2 or RAG-2 deficient mice, such that the transplanted cells can mount T cell dependent or independent immune responses upon antigenic stimulation of the transplanted mice.

For this, 1.5 to 3 month old mice are sublethally irradiated with 300-600 rad γ-irradiaton and 4-6 hour post irradiation the mice are intravenously injected with 5x10⁶ to 10⁷ preB cells resuspended in PBS. In case of additional transplantation of CD4⁺ T-helper cells, CD4⁺ cells are isolated from pooled cervical, axillary, mesenteric and inguinal lymph nodes of syngeneic mice by depletion of B cells and CD8⁺ T cells by use of sheep-anti-mouse Ig antibody coated Dynabeads (Milan Analytica AG, La Roche, Switzerland). 10⁶ of these routinely more than 90% pure CD4⁺ T-helper cells are coinjected with preB cells. Cell populations are allowed to reconstitute B and T cell compartments of the transplanted hosts for 6 weeks following transplantation.

### Example 10: Immunization of preB cell grafted mice

Immunizations of immunocompromised mice transplanted and reconstituted with HupreB cells and T helper cells is done essentially as described (Harlow and Lane, *Cold Spring Harbor Laboratory Press,* p. 150-173, 1988). In brief, 10-50µg of antigen is dissolved in 250µl PBS and vigorously mixed with 250µl complete Freund's adjuvant and the antigen-adjuvant mixture is injected intraperitonally. After two weeks, mice are boosted intraperitonally with 5-20µg of antigen, this time mixed with incomplete Freund's adjuvant. 10 days following the first boost, mice are analyzed for antigen specific heterologous antibodies in the serum. The best responders are boosted again four weeks afterwards with 5-20µg of antigen in incomplete Freund's adjuvant both intraperitoneally and intravenously. 3 days later, spleen cells are isolated from mice and used for the generation of hybridoma cells secreting heterologous antigen-specific monoclonal antibodies.

### Example 11: In vitro stimulation of HupreB cells with for the differentiation into antibody secreting plasma cells

Stromal cell and IL7 dependent HupreB cells can also be differentiated into antibody secreting cells entirely in tissue culture *in vitro.* For this, continuously proliferating HupreB cell cultures are first differentiated into surface immunoglobulin positive B cell by withdrawal of IL7 from the tissue culture medium and the continued culture on irradiated stromal cells for two to three days. The absence of growth factors in the medium results in the arrest of proliferation and the induction of differentiation which is accompanied by the induction of apoptosis, unless an anti-apoptotic gene, like bcl-2 is expressed in the differentiating cells.

The differentiated cells are then harvested, and replated on fresh irradiated stromal cells e.g. in the presence of 100 U/ml rIL4 and 20µg/ml of an agonistic anti-CD40 monoclonal antibody. Within 5-6 days of culture the cells differentiate into large proliferating cells of plasma cell phenotype, that are able to secrete large amounts of antibodies from the heterologous Ig gene loci and that can be used for generation of heterologous monoclonal antibody secreting hybridoma cell clones.

### Example 12: Fusion of myeloma cells with plasma cells derived from HupreB cells for the generation of hybridomas producing human monoclonal antibodies

For the immortalization of plasma cells derived from HupreB cells, either upon *in vitro* differentiation (Example 11), or after immunization of transplanted mice (Example 10), the plasma cells need to be fused with an immortal myeloma cell line. The myeloma cell lines that are used for the cell fusion lack the expression of the HGPRT gene, involved in the salvage pathway of nucleotide biosynthesis. While these cells grow indefinitively in regular tissue culture medium, they will die, if *de novo* nucleotide synthesis is blocked by addition of e.g. the drug aminopterine to the tissue culture medium. In contrast, cells expressing the HGPRT gene can survive a block of *de novo* nucleotide synthesis by aminopterine, especially, if additional thymidine and hypoxanthine are supplemented, because the HGPRT gene product can utilize these substances for the synthesis of pyrimidine and purine nucleotides, respectively. Thus, in HAT selection medium (containing hypoxanthine, aminopterine and thymidine), only those cells survive and proliferate, that resulted from a fusion of the mortal, but HGPRT⁺, antibody secreting plasma cell and the immortal, but HGPRT, and therefore aminopterine sensitive myeloma cell. These cells are called hybridoma cells, and, if subcloned, will secrete a monoclonal antibody of the specificty encoded by the plasma cell fusion partner.

For the generation of hybridoma cells, either plasma cells from the spleen of immunized mice transplanted with HupreB cells, or plasma cells generated *in vitro* by anti-CD40 and IL4 stimulation are used. In each case a homogeneous cell suspension is generated and washed twice with IMDM medium without any supplements, by repeated centrifugation (500g, 5min) and resuspension steps. 5x10⁶ myeloma cells (Sp2/0 Köhler and Milstein, *Eur. J. Immunol*., 6, p. 511-519, 1976; or X63Ag8.653 Kearney et al., *J. Immmunol.*, 123, p. 1548-1550, 1979) and 5x10⁷ spleen cells (or 5x10⁷ *in vitro* differentiated plasma cells) are combined, mixed and centrifuged again at 500g for 5 min. 1ml of a 50% polyethylene glycol (PEG) 1500 dilution in IMDM medium is slowly added to the cell pellet, while the cells are resuspended by tapping. After 2 minutes incubation, 10ml of DMEM medium without supplements are slowly added, and cells are eventually centrifuged at 500g for 5 min. After removal of the supernatant, the cell pellet is resupended in 1 liter of IMDM based stroma cell growth medium (described in example 1), containing 100 U/ml recombinant IL6 and 1x concentrated HAT supplement. The cells are distributed in 50 96-well tissue culture plates and are incubated at 37°C until individual hybridoma clones can be identified and isolated for further expansion and characterization in tissue culture.

## Claims

1. A method for the generation of vertebrate lymphocytes that can be used for the production of any binding protein or functional fragment(s) thereof with the ability to selectively bind to an antigen or ligand, including any heterologous antibody, any antigen receptor composed of variable domains and constant regions comprising T cell receptors and membrane bound immunoglobulins, any artificial binding protein displaying either wild-type immune effector functions or modified or artificial effector functions not derivable from germline encoded heterologous immunoglobulins or antigen receptors, and any functional fragment(s) thereof, comprising the steps of:
(a) genetically modifying vertebrate precursor lymphocytes, which
(i) are derived from primary lymphoid organs, and
(ii) have the potential to differentiate into mature lymphoid lineage cells, by introducing at least one exogenous genetic element encoding at least one binding protein or functional fragment thereof; and
(b) effecting differentiation of said genetically modified precursor lymphocytes into mature lymphoid lineage cells either *in vitro* or *in vivo,* thereby generating lymphocytes capable of producing said binding protein or functional fragment(s) thereof; under the proviso that the *in vivo* production in humans is excluded.

2. The method according to claim 1, further comprising the step of immortalizing the differentiated lymphocytes by:
(a) fusing the same to myeloma cells for the generation of hybridoma cells;
(b) infecting the same with transforming viruses; or
(c) transfecting the same with a vector construct ensuring expression of at least one transforming oncogene;
thereby generating vertebrate lymphocytes capable of permanently producing said binding protein or functional fragment(s) thereof.

3. The method according to claim 1 or 2, wherein the vertebrate precursor lymphocytes are able to express at least one component of the lymphoid V(D)J recombination machinery and originate from jawed vertebrates comprising cartilaginous fish, bony fish, amphibians, reptilia, birds, mammals including pigs, sheep, cattle, horses and rodents including mice, rats, rabbits and guinea pigs, with murine precursor (pre) B lymphocytes from mice being preferred.

4. The method according to any of claims 1 to 3, wherein said vertebrate precursor lymphocytes are deprived of their potential to express endogenous antibodies and/or antigen receptors or functional fragment(s) thereof, which is achieved by isolating/selecting vertebrate precursor lymphocytes being deficient in expressing endogenous immunoglobulins or fragments thereof, and/or by introducing into said vertebrate precursor lymphocytes at least one vector construct designed to functionally inactivate at least one allele of at least one genetic element, which is selected from the group consisting of:
(a) the coding regions of the immunoglobulin heavy chain gene locus, including all or parts of the V, D, and J gene segments, and any of the coding regions for the constant region exons for µ, δ, γ, ε, and α heavy chains, with or without their membrane spanning exons;
(b) the coding regions of the immunoglobulin κ and/or λ light chain gene loci, including any of the V and J gene segment coding regions, as well as any of the constant region exons;
(c) the coding regions of the T cell receptor α, β, y, and δ gene loci, including all or parts of the V, D and J gene segments, and any of the coding regions for the α, β, γ, and δ constant region exons;
(d) the *cis*-acting immunoglobulin heavy chain gene locus enhancer elements, including the heavy chain intron enhancer and the 3' α enhancer;
(e) the cis-acting immunoglobulin light chain gene locus enhancer elements, including the κ light chain intron enhancer (κiE), the 3'κ enhancer, and the λ2-4 and λ3-1 enhancers;
(f) the *cis*-acting T cell receptor gene loci enhancer elements, including the TCR α, β, γ, and δ enhancers;
(g) the trans-acting recombination activating genes, RAG-1 and RAG-2, including their promoter and enhancer elements, as well as their coding regions; and
(h) the trans-acting DNA repair genes essential for V(D)J recombination, including Ku70, Ku86, the catalytic subunit of DNA-dependent protein kinase (DNA-PKcs), DNA ligase IV, XRCC4 and Artemis, including their promoter and enhancer elements, as well as the coding regions of said genes.

5. The method according to claim 4, wherein said vector constructs include gene targeting vectors comprising regions of DNA sequence homology to said at least one genetic element enabling for homologous recombination, preferably flanking a positive selection marker enabling selection of positive transfectants.

6. The method according to claim 5, wherein said gene targeting vectors additionally comprise a pair of DNA recognition sequences for site-specific DNA recombination enzymes, flanking the positive selection marker, enabling deletion of said positive selection marker upon transfection and transient expression of nucleic acid sequences encoding at least one of the cognate recombinase enzymes.

7. The method according to claim 5 or 6, wherein said gene targeting plasmid vectors additionally comprise a negative selection marker enabling selection against transfectants in which said gene targeting vectors are randomly integrated into the genome by non-homologous recombination.

8. The method according to any of claims 1 to 7, wherein said at least one exogenous genetic element encoding a binding protein or functional fragment(s) thereof, is carried on a genetic construct selected from the group consisting of:
(a) recombinant retroviral DNA constructs comprising promoter, enhancer and coding nucleic acid sequences operably linked to allow expression of at least one binding protein or functional fragment thereof, being either wild-type or having (a) designed mutation(s) in the primary amino acid sequence(s) or being artificial;
(b) recombinant plasmid-based DNA constructs comprising promoter, enhancer and coding nucleic acid sequences operably linked to allow expression of at least one binding protein or functional fragment thereof, being either wild-type or having (a) designed mutation(s) in the primary amino acid sequence(s) or being artificial;
(c) recombinant plasmid-based mini-immunoglobulin or T cell receptor gene loci with unrearranged V, D and J gene segments operably linked to allow V(D)J recombination and subsequent expression of at least one heterologous antibody or T cell receptor, or functional fragment thereof, being either wild-type or having (a) designed mutation(s) in the primary amino acid sequence(s);
(d) bacterial, yeast or vertebrate artificial chromosomes comprising parts or all of immunoglobulin or T cell receptor gene loci in germline configuration operably linked to allow V(D)J recombination and subsequent expression of at least one heterologous antibody or T cell receptor, or functional fragment thereof, being either wild-type or having (a) designed mutation(s) in the primary amino acid sequence(s);
(e) bacterial, yeast or vertebrate artificial chromosomes comprising parts or all of at least one heterologous immunoglobulin or T cell receptor gene locus in modified arrangement designed to allow V(D)J recombination and subsequent expression of at least one heterologous antibody or T cell receptor, or functional fragment thereof, being either wild-type or having (a) designed mutation(s) in the primary amino acid sequence(s);
(f) trans-chromosome elements which are fragments of heterologous chromosomes harbouring parts or all of immunoglobulin or T cell receptor gene loci in germline configuration allowing V(D)J recombination and subsequent expression of at least one heterologous antibody or T cell receptor, being wild-type with respect to the primary amino acid sequence(s).

9. The method according to any of claims 1 to 8, wherein the at least one exogenous genetic element encodes a native or modified human antibody, a human binding protein, a human antigen receptor, or (a) functional fragment(s) thereof.

10. The method according to any of claims 1 to 8, wherein the at least one exogenous genetic element encodes a heterologous or artificial receptor capable of undergoing affinity maturation of the binding region.

11. The method according to any of the preceding claims, wherein the differentiation of vertebrate precursor lymphocytes is effected in vitro by:
(a) arresting proliferation of said vertebrate precursor lymphocytes and inducing differentiation into mature lymphocyte lineage cells by cultivating in the . absence of any precursor lymphocyte growth factor; and
(b) inducing terminal lymphocyte differentiation by further cultivating said cells in the presence of at least one of the following components selected from:
(i) soluble T cell related stimulating factors, comprising interleukin-2, interleukin-4, interleukin-5, interleukin-6, interleukin-10, interleukin-13, TGF-β, and IFN-γ;
(ii) factors activating co-stimulatory receptors of B cells, comprising agonistic antibodies or active, recombinant ligands specific for CD40, B7-1 (CD80), B7-2 (CD86), complement receptors 1 (CD35) and 2 (CD21), LFA-1 (CD11a), LFA-3 (CD58), CD19, CD20, CD30, CD32, CD37, CD38, CD70, CD71, Igα (CD79α), Igβ (CD79β), TAPA-1(CD81), Fas (CD95), TNF-receptor1 (p55, CD120a), TNF-receptor2(p75, CD120b), Ox-40 (CD134), and Iymphotoxin-β receptor; and
(iii) B cell mitogenic factors, T cell independent antigens of type 1, and other polyclonal activators, including lipoplysaccharide (LPS), lipoproteins from gram negative bacteria, polyanions, poly-dldC, pokeweed mitogen (PWM), and anti-immunoglobulin reagents;
and combinations thereof.

12. The method according to any of claims 1 to 10, wherein said differentiation *in vivo* is effected upon transplantation of said genetically modified precursor lymphocytes into a suitable vertebrate host.

13. The method according to claim 12, wherein said lymphocytes are co-transplanted into said host with naive or antigen primed T helper lymphocytes.

14. The method according to claim 12 or 13, wherein said differentiation *in vivo* is followed by immunization of said host with at least one desired immunogenic compound or composition.

15. The method according to any of claims 12 to 14, wherein said vertebrate host is a compatible host being deficient with respect to the generation of endogenous B cells, T cells, and/or NK (natural killer) cells, or a combination thereof.

16. The method according to any of claims 12 to 15, wherein the vertebrate host is selected from jawed vertebrates comprising cartilaginous fish, bony fish, amphibians, reptilia, birds, and mammals including pigs, sheep, cattle, horses and rodents including mice, rats, rabbits and guinea pigs, with mice being the preferred host species.

17. Method for the production of any binding protein or functional fragment(s) thereof with the ability to selectively bind to an antigen or ligand, including any heterologous antibody, any antigen receptor composed of variable domains and constant regions comprising T cell receptors and membrane bound immunoglobulins, any artificial binding protein displaying either wild-type immune effector functions or modified or artificial effector functions not derivable from germline encoded heterologous immunoglobulins or antigen receptors, and any functional fragment(s) thereof, in a manner known *per se,* involving the steps of:
(a) genetically modifying vertebrate precursor lymphocytes, which
(i) are derived from primary lymphoid organs, and
(ii) have the potential to differentiate into mature lymphoid lineage cells, by introducing at least one exogenous genetic element encoding at least one binding protein or functional fragment thereof; or, alternatively, using genetically modified vertebrate precursor lymphocytes, which
(i) are derived from primary lymphoid organs,
(ii) have the potential to differentiate into mature lymphoid lineage cells, and
(iii) carry at least one exogenous genetic element encoding at least one binding protein or functional fragment thereof;
(b) effecting differentiation of said genetically modified precursor lymphocytes into mature lymphoid lineage cells either *in vitro* or *in vivo,* thereby generating genetically modified and differentiated vertebrate lymphocytes capable of producing said binding protein or functional fragment thereof; or, alternatively,
using said genetically modified and differentiated vertebrate lymphocytes capable of producing said binding protein or functional fragment thereof;
(c) effecting expression of the binding protein or functional fragment(s) thereof;
under the proviso, that the *in vivo* production in humans is excluded.

18. The method according to claim 1 or 17, wherein step (b) is followed by the steps of:
(a) isolating from said differentiated lymphocytes the at least one exogenous genetic element, and
(b) placing said genetic element(s) in a context enabling production of said at least one binding protein or functional fragment(s) thereof.

19. The method according to claim 17 or 18, wherein said binding protein or functional fragment(s) thereof displays one unique specificity and is therefore monoclonal.

20. The method according to claim 17 or 18, wherein said binding protein or functional fragment(s) thereof displays more than one unique specificity and is therefore polyclonal.

21. The method according to any of claims 17 to 20, wherein said binding protein or functional fragment(s) thereof completely or partially shares structural and/or functional features of a human antibody, antigen receptor, or binding protein, as can be assembled on the basis of the human genetic repertoire or parts thereof.

22. The method according to any of claims 17 to 21, wherein the binding protein or functional fragment(s) thereof to be produced is selected from the group consisting of:
(a) antibodies being either membrane bound or secreted, and consisting of both heterologous heavy and light chain polypeptides in the stochiometric composition found in natural antibodies and consisting of any of the known heavy (µ, δ, γ, α, ε) and/or light (κ and λ) chain isotypes;
(b) antibodies with combinations of heavy and light chain polypeptides being completely human with respect to the primary amino acid sequence;
(c) hybrid antibodies containing heterologous heavy or light chain polypeptides from different vertebrate species;
(d) secreted Fab, scFv and F(ab')2 antibody fragments being either completely or partially heterologous;
(e) fragments of antibodies covalently coupled via linker peptides, resulting in bispecific or multispecific antibody fragments;
(f) T cell receptors of the α, ρ, γ, and δ isotype, antigen receptors, and other binding proteins with structural resemblance to proteins of the immunoglobulin superfamily;
and functional fragments thereof.

23. Genetically modified vertebrate precursor lymphocytes, which
(a) are derived from primary lymphoid organs and have the potential to differentiate into mature lymphoid lineage cells,
(b) carry at least one exogenous genetic element encoding at least one heterologous binding protein or functional fragment thereof with the ability to selectively bind to an antigen or ligand, including any heterologous antibody, any heterologous antigen receptor composed of variable domains and constant regions comprising T cell receptors and membrane bound immunoglobulins, any heterologous artificial binding protein displaying either wild-type immune effector functions or modified or artificial effector functions not derivable from germline encoded heterologous immunoglobulins or antigen receptors, and any functional fragment(s) thereof, and
(c) carry at least one selection marker being operably linked to said at least one exogenous genetic element.

24. Mature lymphoid lineage cells, which
(a) carry at least one exogenous genetic element encoding at least one heterologous binding protein or functional fragment thereof with the ability to selectively bind to an antigen or ligand, including any heterologous antibody, any heterologous antigen receptor composed of variable domains and constant regions comprising T cell receptors and membrane bound immunoglobulins, any heterologous artificial binding protein displaying either wild-type immune effector functions or modified or artificial effector functions not derivable from germline encoded heterologous immunoglobulins or antigen receptors, and any functional fragment(s) thereof, and
(b) carry at least one selection marker being operably linked to said at least one exogenous genetic element.

25. Immortalized cells derived from mature lymphoid lineage cells, which
(a) carry at least one exogenous genetic element encoding at least one heterologous binding protein or functional fragment thereof with the ability to selectively bind to an antigen or ligand, including any heterologous antibody, any heterologous antigen receptor composed of variable domains and constant regions comprising T cell receptors and membrane bound immunoglobulins, any heterologous artificial binding protein displaying either wild-type immune effector functions or modified or artificial effector functions not derivable from germline encoded heterologous immunoglobulins or antigen receptors, and any functional fragment(s) thereof,
(b) carry at least one selection marker being operably linked to said at least one exogenous genetic element, and
(c) produce said at least one heterologous binding protein or functional fragment thereof.

## Patentansprüche

1. Verfahren zur Herstellung von Vertebraten-Lymphozyten, die verwendet werden können zur Produktion irgendeines Bindeproteins oder eines oder mehrerer funktioneller Fragmente davon mit der Fähigkeit, selektiv an ein Antigen oder einen Liganden zu binden, einschließlich irgendeines heterologen Antikörpers, irgendeines aus variablen Domänen und konstanten Regionen zusammengesetzten Antigen-Rezeptors umfassend T-Zell-Rezeptoren und membrangebundene Immunglobuline, irgendeines artifiziellen Bindeproteins mit entweder Wildtyp-Immuneffektorfunktionen oder modifizierten oder artifiziellen Effektorfunktionen, welche nicht von Keimbahn-kodierten heterologen Immunglobulinen oder Antigenrezeptoren hergeleitet werden können, und irgendeines funktionellen Fragments davon, umfassend die folgende Schritte:
(a) genetische Modifizierung von Vertebraten-Vorläufer-Lymphozyten, die
(i) aus primären lymphoiden Organen stammen und
(ii) das Potential haben, sich in reife Zellen der lymphoiden Linie zu differenzieren,
durch Einbringen mindestens eines exogenen genetischen Elementes, welches mindestens ein Bindeprotein oder funktionelles Fragment davon kodiert; und
(b) Herbeiführung der Differenzierung der genetisch modifizierten Vorläufer-Lymphozyten in reife Zellen der lymphoiden Linie entweder *in vitro* oder *in vivo*, wodurch Lymphozyten generiert werden, die in der Lage sind, das Bindeprotein oder funktionelle Fragment(e) davon zu produzieren, unter der Maßgabe, dass die in vivo Produktion in Menschen ausgeschlossen ist.

2. Verfahren nach Anspruch 1, ferner umfassend den Schritt der Immortalisierung der differenzierten Lymphozyten durch:
(a) Fusion derselben mit Myelomzellen zur Erzeugung von Hybridomzellen;
(b) Infektion derselben mit transformierenden Viren; oder
(c) Transfektion derselben mit einem Vektorkonstrukt, welches die Expression von mindestens einem transformierenden Onkogen sicherstellt;
wodurch Vertebraten-Lymphozyten erzeugt werden, die in der Lage sind, das Bindeprotein oder funktionelle Fragment(e) davon permanent zu produzieren.

3. Verfahren nach Anspruch 1 oder 2, wobei die Vertebraten-Vorläufer-Lymphozyten in der Lage sind, mindestens eine Komponente der lymphoiden V(D)J-Rekombinationsmaschinerie zu exprimieren, und abstammen von kiefertragenden Vertebraten umfassend Knorpelfische, Knochenfische, Amphibien, Reptilien, Vögel, und Säugetiere einschließlich Schweine, Schafe, Rinder, Pferde, und Nagetiere einschließlich Mäuse, Ratten, Kaninchen und Meerschweinchen, wobei murine Vorläufer(prä)-B-Lymphozyten von Mäusen bevorzugt sind.

4. Verfahren nach einem der Ansprüche 1 bis 3, wobei die Vertebraten-Vorläufer-Lymphozyten ihr Potential zur Expression endogener Antiköper und/oder Antigen-Rezeptoren oder funktioneller Fragmente davon verloren haben, was erreicht wird durch Isolation/Selektion von Vertebraten-Vorläufer-Lymphozyten, die hinsichtlich der Expression endogener Immunglobuline oder funktioneller Fragmente davon defizient sind, und/oder durch Einbringen in die Vertebraten-Vorläufer-Lymphozyten von mindestens einem Vektorkonstrukt zur funktionellen Inaktivierung von mindestens einem Allel mindestens eines genetischen Elementes, welches ausgewählt wird aus der Gruppe bestehend aus:
(a) den kodierenden Regionen des Immunglobulin-Genlocus für die schwere Kette, einschließlich sämtlicher oder Teilen der V-, D- und J-Gensegmente, und irgendeiner der kodierenden Regionen für die Exons der konstanten Regionen für die schweren Ketten µ, δ, γ, ε, und α, mit oder ohne ihrer Membranüberspannenden Exons;
(b) den kodierenden Regionen der Immunglobulin-Genloci für die leichte Kette κ und/oder λ, einschließlich jedweder kodierender Regionen der V- und J-Gensegmente, als auch jedweder Exons für die konstanten Regionen;
(c) den kodierenden Regionen der T-Zell-Rezeptor Genloci α, β, γ, and δ, einschließlich sämtlicher oder Teilen der V-, D- und J-Gensegmente, und irgendeiner der kodierenden Regionen für die Exons α, β, γ, and δ der konstanten Regionen;
(d) den *cis*-agierenden Enhancer-Elementen des Immunglobulin-Genlocus für die schwere Kette, einschließlich des Intron-Enhancers und des 3' α Enhancers der schweren Kette;
(e) den *cis*-agierenden Enhancer-Elementen des Immunglobulin-Genlocus für die leichte Kette, einschließlich des κ-Intron-Enhancers (κiE), des 3'κ Enhancers und des λ2-4 und λ3-1 Enhancers;
(f) den *cis*-agierenden Enhancer-Elementen der T-Zell-Rezeptor Genloci, einschließlich der TCR-Enhancer α, β, γ, and δ;
(g) den *trans*-agierenden, die Rekombination aktivierenden Genen RAG-1 und RAG-2, einschließlich ihrer Promotor- und Enhancer-Elemente als auch ihrer kodierenden Regionen; und
(h) den *trans*-agierenden, für die V(D)J-Rekombination essentiellen DNA-Reparaturgenen, einschließlich Ku70, Ku86, der katalytischen Untereinheit der DNA-abhängigen Protein-Kinase (DNA-PKcs), DNA Ligase IV, XRCC4 und Artemis, einschließlich ihrer Promotor- und Enhancer-Elemente als auch der kodierenden Regionen dieser Gene.

5. Verfahren nach Anspruch 4, wobei die Vektorkonstrukte Gen-Targeting-Vektoren einschließen, welche DNA-Regionen mit Sequenzhomologie zu dem mindestens einen genetischen Element umfassen und die homologe Rekombination ermöglichen und vorzugsweise einen positiven Selektionsmarker flankieren, der die Selektion positiver Transfektanten ermöglicht.

6. Verfahren nach Anspruch 5, wobei die Gen-Targeting-Vektoren zusätzlich ein Paar von DNA-Erkennungssequenzen für ortsspezifische DNA-Rekombinationsenzyme umfassen, die den positiven Selektionsmarker flankieren, wodurch die Deletion des positiven Selektionsmarkers bei Transfektion und transienter Expression von Nukleinsäuresequenzen, die mindestens eines der spezifischen Rekombinaseenzyme kodieren, ermöglicht wird.

7. Verfahren nach Anspruch 5 oder 6, wobei die Plasmid-Vektoren für das Gen-Targeting zusätzlich einen negativen Selektionsmarker umfassen, der die Selektion gegen Transfektanten ermöglicht, in denen die Gen-Targeting-Vektoren zufällig durch nichthomologe Rekombination in das Genom integriert sind.

8. Verfahren nach einem der Ansprüche 1 bis 7, wobei das mindestens eine exogene genetische Element, das ein Bindeprotein oder funktionelles Fragment davon kodiert, auf einem genetischen Konstrukt liegt, welches ausgewählt ist aus der Gruppe bestehend aus:
(a) rekombinanten retroviralen DNA-Konstrukten umfassend Promotor-, Enhancerund kodierende Nukleinsäuresequenzen, die operationell gekoppelt sind, um die Expression zu ermöglichen von mindestens einem Bindeprotein oder funktionellen Fragment davon, welches entweder die Wildtyp-Form oder eine oder mehrere Mutation(en) in der bzw. den primären Aminosäuresequenz(en) aufweist oder artifiziell ist;
(b) rekombinanten, auf Plasmiden basierenden DNA-Konstrukten umfassend Promotor-, Enhancer- und kodierende Nukleinsäuresequenzen, die operationell gekoppelt sind, um die Expression zu ermöglichen von mindestens einem Bindeprotein oder funktionellen Fragment davon, welches entweder die Wildtyp-Form oder eine oder mehrere Mutation(en) in der bzw. den primären Aminosäuresequenz(en) aufweist oder artifiziell ist;
(c) rekombinanten, auf Plasmiden basierenden Mini-Immunglobulin- oder T-Zell-Rezeptor-Genloci mit nicht-umgelagerten V-, D- und J-Gensegmenten, die operationell gekoppelt sind, um eine V(D)J-Rekombination und die nachfolgende Expression zu ermöglichen von mindestens einem heterologen Antikörper oder T-Zell-Rezeptor oder funktionellen Fragment davon, welcher bzw. welches entweder die Wildtyp-Form oder eine oder mehrere Mutation(en) in der bzw. den primären Aminosäuresequenz(en) aufweist;
(d) artifiziellen Chromosomen von Bakterien, Hefen oder Vertebraten, die Teile oder sämtliche Genloci für Immunglobuline oder T-Zell-Rezeptoren in Keimbahnkonfiguration umfassen und operationell gekoppelt sind, um eine V(D)J-Rekombination und die nachfolgende Expression zu ermöglichen von mindestens einem heterologen Antikörper oder T-Zell-Rezeptor oder funktionellen Fragment davon, welcher bzw. welches entweder die Wildtyp-Form oder eine oder mehrere Mutation(en) in der bzw. den primären Aminosäuresequenz(en) aufweist;
(e) artifiziellen Chromosomen von Bakterien, Hefen oder Vertebraten, die Teile oder sämtliche Bereiche mindestens eines heterologen Genlocus für Immunglobuline oder T-Zell-Rezeptoren in derart modifizierter Anordnung umfassen, damit die V(D)J-Rekombination und die nachfolgende Expression ermöglicht wird von mindestens einem heterologen Antikörper oder T-Zell-Rezeptor oder funktionellen Fragment davon, welcher bzw. welches entweder die Wildtyp-Form oder eine oder mehrere Mutation(en) in der bzw. den primären Aminosäuresequenz(en) aufweist;
(f) transchromosomalen Elementen, welche Fragmente von heterologen Chromosomen sind und Teile oder sämtliche Genloci für Immunglobuline oder T-Zell-Rezeptoren in Keimbahnkonfiguration aufweisen und eine V(D)J-Rekombination und die nachfolgende Expression mindestens eines heterologen Antikörpers oder T-Zell-Rezeptors erlauben, welcher hinsichtlich der primären Aminosäuresequenz(en) die Wildtyp-Form aufweist.

9. Verfahren nach einem der Ansprüche 1 bis 8, wobei das mindestens eine exogene genetische Element einen nativen oder modifizierten humanen Antikörper, ein humanes Bindeprotein, einen humanen Antigen-Rezeptor, oder ein oder mehrere funktionelle Fragmente davon kodiert.

10. Verfahren nach einem der Ansprüche 1 bis 8, wobei das mindestens eine exogene genetische Element einen heterologen oder artifiziellen Rezeptor kodiert, dessen Binderegion einer Affinitätsreifung unterzogen werden kann.

11. Verfahren nach irgendeinem der vorhergehenden Ansprüche, bei dem die Differenzierung der Vertebraten-Vorläufer-Lymphozyten *in vitro* herbeigeführt wird durch:
(a) Anhalten der Proliferation der Vertebraten-Vorläufer-Lymphozyten und Induzieren der Differenzierung in reife Zellen der lymphoiden Linie durch Kultivieren derselben in Abwesenheit irgendeines Vorläufer-Lymphozyten-Wachstumsfaktors, und
(b) Induzieren der terminalen Lymphozyten-Differenzierung durch weiteres Kultivieren der Zellen in Gegenwart mindestens einer der folgenden Komponenten ausgewählt aus:
(i) löslichen, mit T-Zellen im Zusammenhang stehenden stimulierenden Faktoren, umfassend Interleukin-2, Interleukin-4, Interleukin-5, Interleukin-6, Interleukin-10, Interleukin-13, TGF-β, und IFN-γ;
(ii) Faktoren, die co-stimulatorische B-Zell-Rezeptoren aktivieren, umfassend agonistische Antikörper oder aktive rekombinante Liganden, spezifisch für CD40, B7-1 (CD80), B7-2 (CD86), Komplement-Rezeptorenl (CD35) und 2 (CD21), LFA-1 (CD11a), LFA-3 (CD58), CD19, CD20, CD30, CD32, CD37, CD38, CD70, CD71, Igα (CD79α), Igβ (CD79β), TAPA-1(CD81), Fas (CD95), TNF-Rezeptorl (p55, CD120a), TNF-Rezeptor2 (p75, CD120b), Ox-40 (CD134), und Lymphotoxin-β-Rezeptor; und
(iii) B-Zell mitogenen Faktoren, T-Zell-unabhängigen Antigenen des Typs 1, und anderen polyklonalen Aktivatoren, einschließlich Lipopolysacchariden (LPS), Lipoproteinen von Gram-negativen Bakterien, Polyanionen, Poly-dIdC, Pokeweed-Mitogen (PWM), und Anti-Immunglobulin-Reagenzien;
und Kombinationen davon.

12. Verfahren nach einem der Ansprüche 1 bis 10, wobei die Differenzierung *in vivo* durch die Transplantation der genetisch modifizierten Vorläufer-Lymphozyten in einen geeigneten Vertebraten-Rezipienten herbeigeführt wird.

13. Verfahren nach Anspruch 12, wobei die Lymphozyten in den Rezipienten zusammen mit naiven oder Antigen-vorbehandelten T-Helfer-Lymphozyten co-transplantiert werden.

14. Verfahren nach Anspruch 12 oder 13, wobei sich an die Differenzierung in vivo die Immunisierung des Rezipienten mit mindestens einer gewünschten immunogenen Verbindung oder Zusammensetzung anschließt.

15. Verfahren nach einem der Ansprüche 12 bis 14, wobei der Vertebraten-Rezipient ein kompatibler Rezipient ist und eine Defizienz aufweist im Hinblick auf die Erzeugung endogener B-Zellen, T-Zellen und/oder NK (Natürliche Killer) Zellen, oder einer Kombination davon.

16. Verfahren nach einem der Ansprüche 12 bis 15, wobei der Vertebraten-Rezipient ausgewählt wird aus kiefertragenden Vertebraten umfassend Knorpelfische, Knochenfische, Amphibien, Reptilien, Vögel, und Säugetiere einschließlich Schweine, Schafe, Rinder, Pferde, und Nagetiere einschließlich Mäuse, Ratten, Kaninchen und Meerschweinchen, wobei Mäuse die bevorzugte Rezipientenspezies ist.

17. Verfahren zur Herstellung irgendeines Bindeproteins oder eines oder mehrerer funktioneller Fragmente davon mit der Fähigkeit, selektiv an ein Antigen oder einen Liganden zu binden, einschließlich irgendeines heterologen Antikörpers, irgendeines aus variablen Domänen und konstanten Regionen zusammengesetzten Antigen-Rezeptors umfassend T-Zell-Rezeptoren und membrangebundene Immunglobuline, irgendeines artifiziellen Bindeproteins mit entweder Wildtyp-Immuneffektorfunktionen oder modifizierten oder artifiziellen Effektor-funktionen, welche nicht von Keimbahn-kodierten heterologen Immunglobulinen oder Antigen-Rezeptoren hergeleitet werden können, und irgendeines funktionellen Fragmentes davon, in einer *per se* bekannten Weise, beinhaltend die folgenden Schritte:
(a) genetische Modifizierung von Vertebraten-Vorläufer-Lymphozyten, die
(i) aus primären lymphoiden Organen stammen und
(ii) das Potential haben, sich in reife Zellen der lymphoiden Linie zu differenzieren,
durch Einbringen mindestens eines exogenen genetischen Elementes, welches mindestens ein Bindeprotein oder funktionelles Fragment davon kodiert; oder, alternativ, durch Verwendung genetisch modifizierter Vertebraten-Vorläufer-Lymphozyten, die
(i) aus primären lymphoiden Organen stammen und
(ii) das Potential haben, sich in reife Zellen der lymphoiden Linie zu differenzieren, und
(iii) mindestens ein exogenes genetisches Element aufweisen, welches mindestens ein Bindeprotein oder funktionelles Fragment davon kodiert;
(b) Herbeiführung der Differenzierung der genetisch modifizierten Vorläufer-Lymphozyten in reife Zellen der lymphoiden Linie entweder *in vitro* oder *in vivo,* wodurch genetisch modifizierte und differenzierte Vertebraten-Lymphozyten generiert werden, die in der Lage sind, das Bindeprotein oder funktionelle Fragment davon herzustellen, oder, alternativ,
Verwendung der genetisch modifizierten und differenzierten Vertebraten-Lymphozyten, die in der Lage sind, das Bindeprotein oder funktionelle Fragment davon herzustellen;
(c) Herbeiführung der Expression des Bindeproteins oder funktionellen Fragments davon; unter der Maßgabe, dass die *in vivo* Produktion in Menschen ausgeschlossen ist.

18. Verfahren nach Anspruch 1 oder 17, wobei Schritt (b) gefolgt wird von den Schritten:
(a) Isolierung des mindestens einen exogenen genetischen Elementes aus den differenzierten Lymphozyten, und
(b) Platzierung des bzw. der genetischen Elemente(s) in einen Kontext, der die Produktion des mindestens einen Bindeproteins oder funktionellen Fragments davon ermöglicht.

19. Verfahren nach Anspruch 17 oder 18, wobei das Bindeprotein oder funktionelle Fragment davon eine einzigartige Spezifität aufweist und daher monoklonal ist.

20. Verfahren nach Anspruch 17 oder 18, wobei das Bindeprotein oder funktionelle Fragment davon mehr als eine einzigartige Spezifität aufweist und daher polyklonal ist.

21. Verfahren nach einem der Ansprüche 17 bis 20, wobei das Bindeprotein oder funktionelle Fragment davon vollständig oder teilweise hinsichtlich der strukturellen und/oder funktionellen Eigenschaften übereinstimmt mit einem menschlichen Antikörper, Antigen-Rezeptor, oder Bindeprotein, wie er bzw. es auf der Basis des menschlichen genetischen Repertoires oder Teilen davon zusammengebaut werden kann.

22. Verfahren nach einem der Ansprüche 17 bis 21, wobei das herzustellende Bindeprotein oder funktionelle Fragment davon ausgewählt wird aus der Gruppe bestehend aus:
(a) membrangebundenen oder sezernierten Antikörpern, bestehend aus heterologen Polypeptiden der schweren und leichten Ketten in der bei natürlichen Antikörpern vorliegenden stöchiometrischen Zusammensetzung, und bestehend aus irgendeinem der für die schweren (µ, δ, γ, α, ε) und/oder leichten (κ und λ) Ketten bekannten Isotypen;
(b) Antikörpern mit Kombinationen von Polypeptiden der schweren und leichten Ketten, die im Hinblick auf die primäre Aminosäuresequenz vollständig human sind;
(c) Hybrid-Antikörpem, die heterologe Polypeptide der schweren oder leichten Ketten von verschiedenen Vertebraten-Spezies enthalten;
(d) sezernierten Fab-, scFv- und F(ab')2-Antikörperfragmenten, die entweder vollständig oder teilweise heterolog sind;
(e) Fragmenten von Antikörpern, die über Linkerpeptide kovalent verbunden sind, woraus bispezifische oder multispezifische Antikörperfragmente resultieren;
(f) T-Zell-Rezeptoren des α-, β-, γ- und δ-Isotyps, Antigen-Rezeptoren, und anderen Bindeproteinen mit struktureller Ähnlichkeit zu Proteinen der Immunglobulin-Superfamilie;
und funktionellen Fragmenten davon.

23. Genetisch modifizierte Vertebraten-Vorläufer-Lymphozyten, die
(a) aus primären lymphoiden Organen stammen und das Potential haben, sich in reife Zellen der lymphoiden Linie zu differenzieren,
(b) mindestens ein exogenes genetisches Element tragen, das mindestens ein heterologes Bindeprotein oder funktionelles Fragment davon mit der Fähigkeit kodiert, selektiv an ein Antigen oder einen Liganden zu binden, einschließlich irgendeines heterologen Antikörpers, irgendeines aus variablen Domänen und konstanten Regionen zusammengesetzten heterologen Antigen-Rezeptors umfassend T-Zell-Rezeptoren und membrangebundene Immunglobuline, irgendeines heterologen artifiziellen Bindeproteins mit entweder Wildtyp-Immuneffektorfunktionen oder modifizierten oder artifiziellen Effektorfunktionen, welche nicht von Keimbahn-kodierten heterologen Immunglobulinen oder Antigen-Rezeptoren hergeleitet werden können, und irgendeines beliebigen funktionellen Fragments davon, und
(c) mindestens einen Selektionsmarker tragen, der mit dem mindestens einen exogenen genetischen Element operationell verbunden ist.

24. Reife Zellen der lymphoiden Linie, die
(a) mindestens ein exogenes genetisches Element tragen, das mindestens ein heterologes Bindeprotein oder funktionelles Fragment davon mit der Fähigkeit kodiert, selektiv an ein Antigen oder einen Liganden zu binden, einschließlich irgendeines heterologen Antikörpers, irgendeines aus variablen Domänen und konstanten Regionen zusammengesetzten heterologen Antigen-Rezeptors umfassend T-Zell-Rezeptoren und membrangebundene Immunglobuline, irgendeines heterologen artifiziellen Bindeproteins mit entweder Wildtyp-Immuneffektorfunktionen oder modifizierten oder artifiziellen Effektorfunktionen, welche nicht von Keimbahn-kodierten heterologen Immunglobulinen oder Antigen-Rezeptoren hergeleitet werden können, und irgendeines beliebigen funktionellen Fragments davon, und
(b) mindestens einen Selektionsmarker tragen, der mit dem mindestens einen exogenen genetischen Element operationell verbunden ist.

25. Immortalisierte Zellen, die von reifen Zellen der lymphoiden Linie abstammen, und
(a) mindestens ein exogenes genetisches Element tragen, das mindestens ein heterologes Bindeprotein oder funktionelles Fragment davon mit der Fähigkeit kodiert, selektiv an ein Antigen oder einen Liganden zu binden, einschließlich irgendeines heterologen Antikörpers, irgendeines aus variablen Domänen und konstanten Regionen zusammengesetzten heterologen Antigen-Rezeptors umfassend T-Zell-Rezeptoren und membrangebundene Immunglobuline, irgendeines heterologen artifiziellen Bindeproteins mit entweder Wildtyp-Immuneffektorfunktionen oder modifizierten oder artifiziellen Effektorfunktionen, welche nicht von Keimbahn-kodierten heterologen Immunglobulinen oder Antigen-Rezeptoren hergeleitet werden können, und irgendeines beliebigen funktionellen Fragments davon, und
(b) mindestens einen Selektionsmarker tragen, der mit dem mindestens einen exogenen genetischen Element operationell verbunden ist, und
(c) das mindestens eine heterologe Bindeprotein oder funktionelle Fragment davon produzieren.

## Revendications

1. Procédé pour générer des lymphocytes de vertébrés, qui peuvent être utilisés pour produire une quelconque protéine de liaison ou un(des) fragment(s) fonctionnel(s) de celle-ci, ayant la faculté de se lier sélectivement à un antigène ou à un ligand, y compris : un quelconque anticorps hétérologue ; un quelconque récepteur d'antigène, composé de domaines variables et de régions constantes, qui comprennent des récepteurs de lymphocytes T et des immunoglobulines liées à la membrane ; une quelconque protéine de liaison artificielle présentant soit des fonctions effectrices immunologiques de type sauvage soit des fonctions effectrices modifiées ou artificielles, qui ne sont pas dérivables à partir des récepteurs d'antigènes ou d'immunoglobulines hétérologues codés par la lignée germinale ; et n'importe quel(s) fragment(s) fonctionnel(s) de ceux-ci, comprenant les étapes consistant à :
(a) modifier génétiquement des lymphocytes précurseurs de vertébrés, lesquels :
(i) sont dérivés d'organes lymphoïdes primaires et
(ii)ont le potentiel pour se différencier en cellules d'une lignée lymphoïde mature, en introduisant au moins un élément génétique exogène codant pour au moins une protéine de liaison ou pour un fragment fonctionnel de celle-ci ; et
(b) effectuer une différenciation desdits lymphocytes précurseurs, génétiquement modifiés, en cellules d'une lignée lymphoïde mature, soit *in vitro* soit *in vivo,* en générant de ce fait des lymphocytes capables de produire ladite protéine de liaison ou un(des) fragment(s) fonctionnel(s) de celle-ci ; avec la condition que la production in vivo soit exclue chez les êtres humains.

2. Procédé, selon la revendication 1, comprenant en outre l'étape consistant à immortaliser les lymphocytes différenciés:
(a) en les fusionnant avec des cellules de myélome pour générer des cellules d'hybridome ;
(b) en les infectant avec des virus transformants ; ou
(c) en les transfectant avec une construction-vecteur, assurant l'expression d'au moins un oncogène transformant ;
en générant de ce fait des lymphocytes de vertébrés capables de produire de manière permanente ladite protéine de liaison ou un(des) fragment(s) fonctionnel(s) de celle-ci.

3. Procédé selon les revendications 1 ou 2, dans lequel les lymphocytes précurseurs de vertébrés sont capables d'exprimer au moins un composant de la machinerie de recombinaison lymphoïde V(D)J et proviennent de vertébrés à mâchoire, comprenant : les poissons cartilagineux ; les poissons osseux ; les amphibiens ; les reptiles ; les oiseaux ; les mammifères, y compris les porcs, les moutons, le bétail, les chevaux et les rongeurs, y compris les souris, les rats, les lapins et les cochons d'Inde ; les (pré)lymphocytes B précurseurs murins, provenant des souris, étant préférés.

4. Procédé selon l'une quelconque des revendications 1 à 3, dans lequel lesdits lymphocytes précurseurs de vertébrés sont privés de leur potentiel pour exprimer des anticorps et/ou des récepteurs d'antigènes endogènes ou un(des) fragment(s) fonctionnel(s) de ceux-ci, ce qui est atteint en isolant/sélectionnant des lymphocytes précurseurs de vertébrés déficients pour l'expression d'immunoglobulines endogènes ou de fragments de celles-ci, et/ou en introduisant dans lesdits lymphocytes précurseurs de vertébrés au moins une construction-vecteur créée pour inactiver fonctionnellement au moins un allèle d'au moins un élément génétique, qui est choisi dans le groupe composé :
(a) des régions codantes du locus du gène de la chaîne lourde des immunoglobulines, y compris tout ou partie des segments géniques V, D et J, et de n'importe quelle région codant pour des exons de la région constante des chaînes lourdes µ, δ, γ, ε et α, avec ou sans leurs exons trans-membranaires ;
(b) des régions codantes des locus du gène des chaînes légères κ et/ou λ des immunoglobulines, y compris n'importe quelle région codante des segments géniques V et J, ainsi que n'importe quel exon de la région constante ;
(c) des régions codantes des locus des gènes α, β, γ et δ du récepteur des lymphocytes T, y compris tout ou partie des segments géniques V, D et J, et n'importe quelle région codant pour des exons de la région constante des α, β, γ et δ ;
(d) des éléments, agissant en cis, amplificateurs du locus du gène de la chaîne lourde des immunoglobulines, y compris l'amplificateur de l'intron de la chaîne lourde et l'amplificateur 3' α ;
(e) des éléments, agissant en cis, amplificateurs du locus du gène de la chaîne légère des immunoglobulines, y compris l'amplificateur de l'intron de la chaîne légère κ (κiE), l'amplificateur 3' κ et les amplificateurs λ2-4 et λ3-1 ;
(f) des éléments, agissant en cis, amplificateurs des locus du gène du récepteur des lymphocytes T (RLT), y compris les amplificateurs α, β, γ et δ du RLT ;
(g) des gènes RAG-1 et RAG-2 activant la recombinaison et agissant en *trans*, y compris leurs éléments promoteurs et amplificateurs ainsi que leurs régions codantes ; et
(h) des gènes de réparation de l'ADN, agissant en *trans*, essentiels pour une recombinaison V(D)J, y compris le Ku70, le Ku86, la sous-unité catalytique de la protéine kinase dépendant de l'ADN (DNA-PKcs), l'ADN ligase IV, les XRCC4 et Artemis, y compris leurs éléments promoteurs et amplificateurs, ainsi que les régions codantes desdits gènes.

5. Procédé selon la revendication 4, dans lequel lesdites constructions-vecteurs comprennent des vecteurs de ciblage génique, comprenant des régions d'homologie de séquence d'ADN au moins avec ledit élément génétique permettant d'obtenir une recombinaison homologue, de préférence flanquant un marqueur à sélection positive qui permet une sélection des transfectants positifs.

6. Procédé selon la revendication 5, dans lequel lesdits vecteurs de ciblage génique comprennent en plus une paire de séquences de reconnaissance de l'ADN d'enzymes de recombinaison d'ADN spécifiques d'un site, flanquant le marqueur à sélection positive, laquelle permet une délétion dudit marqueur à sélection positive lors de la transfection et de l'expression transitoire des séquences d'acide nucléique codant pour au moins l'une des enzymes apparentées aux recombinases.

7. Procédé selon les revendications 5 ou 6, dans lequel lesdits vecteurs plasmidiques de ciblage génique comprennent en plus un marqueur à sélection négative permettant d'obtenir une sélection vis-à-vis des transfectants, dans laquelle lesdits vecteurs de ciblage génique sont intégrés de manière aléatoire dans le génome au moyen d'une recombinaison non homologue.

8. Procédé selon l'une quelconque des revendications 1 à 7, dans lequel au moins ledit élément génétique exogène, codant pour une protéine de liaison ou pour un(des) fragment(s) fonctionnel(s) de celle-ci, est transporté sur une construction génétique choisie dans le groupe composé :
(a) de constructions d'ADN rétroviral recombinant comprenant un promoteur, un amplificateur et des séquences d'acide nucléique codantes, liés de manière fonctionnelle pour permettre l'expression d'au moins une protéine de liaison ou d'un fragment fonctionnel de celle-ci, étant de type sauvage, ayant une(des) mutation(s) créée(s) dans la(les) séquence(s) primaire(s) d'acides aminés ou étant artificielle ;
(b) de constructions d'ADN recombinant, basées sur un plasmide, comprenant un promoteur, un amplificateur et des séquences d'acide nucléique codantes, liés de manière fonctionnelle pour permettre l'expression d'au moins une protéine de liaison ou d'un fragment fonctionnel de celle-ci, étant de type sauvage, ayant une(des) mutation(s) créée(s) dans la(les) séquence(s) primaire(s) d'acides aminés ou étant artificielles ;
(c) de locus de gènes recombinants, basés sur des plasmides, de mini-immunoglobulines ou d'un récepteur de lymphocytes T, ayant des segments géniques V, D et J non réarrangés, liés de manière fonctionnelle pour permettre d'obtenir une recombinaison V(D)J et une expression consécutive d'au moins un anticorps ou un récepteur de lymphocytes T hétérologue ou d'un fragment fonctionnel de ceux-ci, étant de type sauvage ou ayant une(des) mutation(s) créée(s) dans la(les) séquence(s) primaire(s) d'acides aminés ;
(d) de chromosomes artificiels de bactéries, de levures ou de vertébrés comprenant tout ou partie des locus des gènes d'immunoglobulines ou des récepteurs de lymphocytes T, dans la configuration de la lignée germinale, liés de manière fonctionnelle pour permettre d'obtenir une recombinaison V(D)J et une expression consécutive d'au moins un anticorps ou un récepteur de lymphocytes T hétérologue, ou d'un fragment fonctionnel de ceux-ci, étant de type sauvage ou ayant une(des) mutation(s) créée(s) dans la(les) séquence(s) primaire(s) d'acides aminés ;
(e) de chromosomes artificiels de bactéries, de levures ou de vertébrés comprenant tout ou partie d'au moins un locus de gène d'immunoglobulines hétérologue ou des récepteurs de lymphocytes T, dans un arrangement modifié, conçu pour permettre d'obtenir une recombinaison V(D)J et une expression consécutive d'au moins un anticorps ou un récepteur de lymphocytes T hétérologue ou d'un fragment fonctionnel de ceux-ci, étant de type sauvage ou ayant une(des) mutation(s) créée(s) dans la(les) séquence(s) primaire(s) d'acides aminés ;
(f) d'éléments trans-chromosomiques, qui sont des fragments de chromosomes hétérologues renfermant tout ou partie des locus des gènes d'immunoglobulines ou des récepteurs de lymphocytes T, dans la configuration de la lignée germinale, permettant d'obtenir une recombinaison V(D)J et une expression consécutive d'au moins un anticorps ou un récepteur de lymphocytes T hétérologue, étant de type sauvage en ce qui concerne la(les) séquence(s) primaire(s) d'acides aminés.

9. Procédé selon l'une quelconque des revendications 1 à 8, dans lequel au moins un élément génétique exogène code pour : un anticorps humain naturel ou modifié ; une protéine de liaison humaine ; un récepteur d'antigénique humain ; ou un(des) fragment(s) fonctionnel(s) de ceux-ci.

10. Procédé selon l'une quelconque des revendications 1 à 8, dans lequel au moins un élément génétique exogène code pour un récepteur hétérologue ou artificiel capable de subir une maturation d'affinité de la région de liaison.

11. Procédé selon l'une quelconque des revendications précédentes, dans lequel la différenciation des lymphocytes précurseurs de vertébrés est effectuée *in vitro* :
(a) en arrêtant la prolifération desdits lymphocytes précurseurs de vertébrés et en induisant une différenciation en cellules d'une lignée de lymphocytes matures, au moyen d'une culture en l'absence d'un quelconque facteur de croissance des lymphocytes précurseurs ; et
(b) en induisant une différenciation lymphocytaire terminale, au moyen d'une culture supplémentaire desdites cellules en présence d'au moins l'un des composants suivants choisis parmi :
(i) des facteurs solubles de stimulation se rapportant aux lymphocytes T, comprenant : l'interleukine-2, l'interleukine-4, l'interleukine-5, l'interleukine-6, l'interleukine-10, l'interleukne-13, le TGF-β et l'IFN-γ ;
(ii) des facteurs activant des récepteurs de lymphocytes B co-stimulateurs, comprenant : des anticorps agonistes ou des ligands recombinants actifs spécifiques des CD40, des B7-1 (CD80), des B7-2 (CD86), des récepteurs 1 (CD35) et 2 (CD21) du complément, des LFA-1 (CD11a), des LFA-3 (CD58), des CD19, des CD20, des CD30, des CD32, des CD37, des CD38, des CD70, des CD71, des Igα (CD79α), des Igβ (CD79β), des TAPA-1 (CD81), des Fas (CD95), du récepteur 1 du TNF (p55, CD120a), du récepteur 2 du TNF (p75, CD120b), des Ox-40 (CD134) et du récepteur de la lymphotoxine β ; et
(iii)des facteurs mitogènes des lymphocytes B, des antigènes de type 1 indépendants des lymphocytes T et d'autres activateurs polyclonaux, y compris un lipopolysaccharide (LPS), des lipoprotéines de bactéries gram négatives, des polyanions, des poly-dIdC, un mitogène de la phytolaque (PWM) et des réactifs anti-immunoglobulines ;
et des combinaisons de ceux-ci.

12. Procédé selon l'une quelconque des revendications 1 à 10, dans lequel ladite différenciation *in vivo* est effectuée lors d'une transplantation desdits lymphocytes précurseurs génétiquement modifiés dans un hôte vertébré approprié.

13. Procédé selon la revendication 12, dans lequel lesdits lymphocytes sont transplantés conjointement dans ledit hôte avec des lymphocytes T auxiliaires naturels ou amorcés avec un antigène.

14. Procédé selon les revendications 12 ou 13, dans lequel ladite différenciation *in vivo* est suivie d'une immunisation dudit hôte avec au moins un composé ou une composition immunogène souhaité.

15. Procédé selon l'une quelconque des revendications 12 à 14, dans lequel ledit hôte vertébré est un hôte compatible, déficient en ce qui concerne la génération de lymphocytes B, de lymphocytes T et/ou de lymphocytes NK (tueurs naturels) endogènes ou d'une combinaison de ceux-ci.

16. Procédé selon l'une quelconque des revendications 12 à 15, dans lequel ledit hôte vertébré est choisi parmi les vertébrés à mâchoire, comprenant : les poissons cartilagineux ; les poissons osseux ; les amphibiens ; les reptiles ; les oiseaux ; et les mammifères, y compris les porcs, les moutons, le bétail, les chevaux et les rongeurs, y compris les souris, les rats, les lapins et les cochons d'Inde ; les souris étant l'espèce hôte préférée.

17. Procédé de production d'une quelconque protéine de liaison ou d'un(des) fragment(s) fonctionnel(s) de celle-ci ayant la faculté se lier sélectivement à un antigène ou à un ligand, y compris : un quelconque anticorps hétérologue ; un quelconque récepteur d'antigène, composé de domaines variables et de régions constantes, qui comprennent des récepteurs de lymphocytes T et des immunoglobulines liées à la membrane ; une quelconque protéine de liaison artificielle, présentant soit des fonctions effectrices immunologiques de type sauvage soit des fonctions effectrices modifiées ou artificielles, qui ne sont pas dérivables à partir de récepteurs d'antigènes ou d'immunoglobulines hétérologues codés par la lignée germinale ; et n'importe quel(s) fragment(s) fonctionnel(s) de ceux-ci, d'une manière connue en soi, impliquant les étapes consistant à :
(a) modifier génétiquement des lymphocytes précurseurs de vertébrés, lesquels :
(i) sont dérivés d'organes lymphoïdes primaires et
(ii) ont le potentiel pour se différencier en cellules d'une lignée lymphoïde mature,
en introduisant au moins un élément génétique exogène codant pour au moins une protéine de liaison ou pour un fragment fonctionnel de celle-ci ; ou, en alternative,
en utilisant des lymphocytes précurseurs de vertébrés génétiquement modifiés, lesquels :
(i) sont dérivés d'organes lymphoïdes primaires,
(ii) ont le potentiel pour se différencier en cellules d'une lignée lymphoïde mature
et
(iii)portent au moins un élément génétique exogène codant pour au moins une protéine de liaison ou pour un fragment fonctionnel de celle-ci ;
(b) effectuer une différenciation desdits lymphocytes précurseurs, génétiquement modifiés, en cellules d'une lignée lymphoïde mature, soit *in vitro* soit *in vivo,* en générant de ce fait des lymphocytes de vertébrés génétiquement modifiés et différenciés capables de produire ladite protéine de liaison ou un(des) fragment(s) fonctionnel(s) de celle-ci ;
ou, en alternative,
en utilisant lesdits lymphocytes de vertébrés génétiquement modifiés et différenciés capables de produire ladite protéine de liaison ou un fragment fonctionnel de celle-ci ;
(c) effectuer l'expression de la protéine de liaison ou d'un(des) fragment(s) fonctionnel(s) de celle-ci ;
avec la condition que la production *in vivo* soit exclue chez les êtres humains.

18. Procédé selon les revendications 1 ou 17, dans lequel l'étape (b) est suivie des étapes consistant à :
(a) isoler à partir desdits lymphocytes différenciés au moins l'élément génétique exogène et
(b) placer ledit(lesdits) élément(s) génétique(s) dans un contexte permettant d'obtenir une production d'au moins ladite protéine de liaison ou d'un(des) fragment(s) fonctionnel(s) de celle-ci.

19. Procédé selon les revendications 17 ou 18, dans lequel ladite protéine de liaison ou un(des) fragment(s) fonctionnel(s) de celle-ci présente une spécificité unique et est, par conséquent, monoclonale.

20. Procédé selon les revendications 17 ou 18, dans lequel ladite protéine de liaison ou un(des) fragment(s) fonctionnel(s) de celle-ci présente plus d'une spécificité unique et est, par conséquent, polyclonale.

21. Procédé selon l'une quelconque des revendications 17 à 20, dans lequel ladite protéine de liaison ou un (des) fragment(s) fonctionnel(s) de celle-ci partage complètement ou partiellement les caractéristiques structurales et/ou fonctionnelles d'un anticorps humain, d'un récepteur d'antigène ou d'une protéine de liaison, telle qu'elle puisse être assemblée sur la base du répertoire génétique humain ou de parties de celui-ci.

22. Procédé selon l'une quelconque des revendications 17 à 21, dans lequel ladite protéine de liaison devant être produite ou un(des) fragment(s) fonctionnel(s) de celle-ci est choisie dans le groupe composé :
(a) d'anticorps qui sont soit liés à la membrane soit sécrétés et qui sont composés de polypeptides hétérologues à chaînes lourdes et légères, avec la composition stoechiométrique que l'on trouve dans les anticorps naturels, et composés de l'un quelconque des isotypes connus à chaînes lourdes (µ, δ, γ, α, ε) et/ou légères (κ et λ) ;
(b) d'anticorps, avec des combinaisons de polypeptides à chaînes lourdes et légères, étant complètement humains en ce qui concerne la séquence primaire d'acides aminés ;
(c) d'anticorps hybrides contenant des polypeptides hétérologues à chaînes lourdes ou légères provenant de différentes espèces de vertébrés ;
(d) de fragments Fab, scFv et F(ab')2 d'anticorps sécrétés, étant complètement ou partiellement hétérologues ;
(e) de fragments d'anticorps couplés de manière covalente, au moyen de peptides lieurs, conduisant à des fragments d'anticorps bi-spécifiques ou multi-spécifiques ;
(f) de récepteurs de lymphocytes T des isotypes α, β, γ et δ, des récepteurs d'antigènes et d'autres protéines de liaison, ayant des ressemblances structurales avec des protéines de la superfamille des immunoglobulines ;
et des fragments fonctionnels de ceux-ci.

23. Lymphocytes précurseurs de vertébrés génétiquement modifiés, qui :
(a) sont dérivés d'organes lymphoïdes primaires et qui ont le potentiel pour se différencier en cellules d'une lignée lymphoïde mature,
(b) portent au moins un élément génétique exogène codant pour au moins une protéine de liaison hétérologue ou pour un fragment fonctionnel de celle-ci, ayant la faculté de se lier sélectivement à un antigène ou à un ligand, y compris : un quelconque anticorps hétérologue ; un quelconque récepteur d'antigène hétérologue, composé de domaines variables et de régions constantes, qui comprennent des récepteurs de lymphocytes T et des immunoglobulines liées à la membrane ; une quelconque protéine de liaison hétérologue artificielle présentant soit des fonctions effectrices immunologiques de type sauvage soit fonctions effectrices modifiées ou artificielles, qui ne sont pas dérivables à partir de récepteurs d'antigènes ou d'immunoglobulines hétérologues codés par la lignée germinale ; et n'importe quel(s) fragment(s) fonctionnel(s) de ceux-ci ; et
(c) portent au moins un marqueur de sélection étant lié de manière fonctionnelle au moins audit un élément génétique exogène.

24. Cellules d'une lignée lymphoïde mature, qui :
(a) portent au moins un élément génétique exogène codant pour au moins une protéine de liaison hétérologue ou pour un fragment fonctionnel de celle-ci, ayant la faculté de se lier sélectivement à un antigène ou à un ligand, y compris : un quelconque anticorps hétérologue ; un quelconque récepteur d'antigène hétérologue, composé de domaines variables et de régions constantes qui comprennent des récepteurs de lymphocytes T et des immunoglobulines liées à la membrane ; une quelconque protéine de liaison artificielle hétérologue présentant soit des fonctions effectrices immunologiques de type sauvage soit des fonctions effectrices modifiées ou artificielles, qui ne sont pas dérivables à partir de récepteurs d'antigènes ou d'immunoglobulines hétérologues codés par la lignée germinale ; et n'importe quel(s) fragment(s) fonctionnel(s) de ceux-ci ; et
(b) portent au moins un marqueur de sélection étant lié de manière fonctionnelle au moins audit un élément génétique exogène.

25. Cellules immortalisées dérivées de cellules d'une lignée lymphoïde mature, qui :
(a) portent au moins un élément génétique exogène codant pour au moins une protéine de liaison hétérologue ou pour un fragment fonctionnel de celle-ci, ayant la faculté de se lier sélectivement à un antigène ou à un ligand, y compris : un quelconque anticorps hétérologue ; un quelconque récepteur d'antigène hétérologue, composé de domaines variables et de régions constantes qui comprennent des récepteurs de lymphocytes T et des immunoglobulines liées à la membrane ; une quelconque protéine de liaison artificielle hétérologue présentant soit des fonctions effectrices immunologiques de type sauvage soit des fonctions effectrices modifiées ou artificielles, qui ne sont pas dérivables à partir de récepteurs d'antigènes ou d'immunoglobulines hétérologues codés par la lignée germinale ; et n'importe quel(s) fragment(s) fonctionnel(s) de ceux-ci ;
(b) portent au moins un marqueur de sélection étant lié de manière fonctionnelle au moins audit élément génétique exogène ; et
(c) produisent au moins ladite protéine de liaison hétérologue ou un fragment fonctionnel de celle-ci.
